(19) Europäisches Patentamt
European Patent Office
Office européen des brevets

(11) EP 4 613 736 A1

(12) EUROPEAN PATENT APPLICATION
published in accordance with Art. 153(4) EPC

(43) Date of publication:
**10.09.2025 Bulletin 2025/37**

(21) Application number: **23884610.9**

(22) Date of filing: **18.10.2023**

(51) International Patent Classification (IPC):
***C07C 229/32*** (2006.01) ***C07C 227/04*** (2006.01)
***A61K 31/195*** (2006.01) ***A61P 25/04*** (2006.01)
***A61P 25/08*** (2006.01) ***A61P 25/22*** (2006.01)

(52) Cooperative Patent Classification (CPC):
**A61K 31/195; A61P 25/04; A61P 25/08; A61P 25/22; C07C 227/04; C07C 229/28; C07C 229/32**

(86) International application number:
**PCT/CN2023/125229**

(87) International publication number:
**WO 2024/093678 (10.05.2024 Gazette 2024/19)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **03.11.2022 CN 202211372369**

(71) Applicants:
- **Zhongshan Institute for Drug Discovery, SIMM, Chinese Academy of Sciences Zhongshan, Guangdong 528451 (CN)**
- **Shanghai Institute of Materia Medica, Chinese Academy of Sciences Shanghai 201203 (CN)**

(72) Inventors:
- **ZHAO, Guilong Zhongshan, Guangdong 528451 (CN)**
- **GAO, Zhaobing Shanghai 201203 (CN)**
- **WU, Qingqing Zhongshan, Guangdong 528451 (CN)**
- **ZHENG, Yueming Shanghai 201203 (CN)**
- **SUN, Li Zhongshan, Guangdong 528451 (CN)**
- **TIAN, Fuyun Shanghai 201203 (CN)**
- **HE, Jinyan Zhongshan, Guangdong 528451 (CN)**
- **XU, Haiyan Shanghai 201203 (CN)**
- **CHEN, Yuting Zhongshan, Guangdong 528451 (CN)**
- **ZHAN, Li Shanghai 201203 (CN)**
- **JIN, Zhengsheng Zhongshan, Guangdong 528451 (CN)**
- **GU, Yueling Shanghai 201203 (CN)**
- **CHENG, Xinqiang Zhongshan, Guangdong 528451 (CN)**
- **ZHANG, Wenbo Shanghai 201203 (CN)**
- **ZHANG, Yuanwen Zhongshan, Guangdong 528451 (CN)**

(74) Representative: **Uexküll & Stolberg Partnerschaft von Patent- und Rechtsanwälten mbB Beselerstraße 4 22607 Hamburg (DE)**

# (54) GAMMA-AMINOBUTYRIC ACID DERIVATIVE COMPRISING POLYCYCLIC STRUCTURE, METHOD FOR PREPARING SAME, AND USE THEREOF

(57) The invention relates to the field of pharmaceuticals. Specifically, the present application relates to a voltage-gated calcium ion channel $\alpha 2\delta$ subunit ligand comprising a polycyclic $\gamma$-aminobutyric acid structure represented by general formula I, a method for preparing same, and use thereof in the treatment of chronic neuropathic pain, epilepsy, and anxiety.

EP 4 613 736 A1

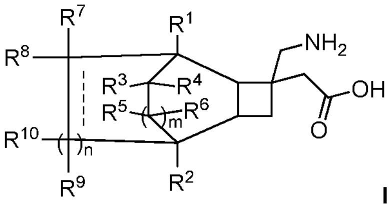

I

## Description

## TECHNICAL FIELD

The present application belongs to the field of medicine. Specifically, the present application relates to a series of voltage-gated calcium ion channel $\alpha2\delta$ subunit ligands comprising a polycyclic $\gamma$-aminobutyric acid structure, a preparation method thereof, a pharmaceutical composition comprising the same, and use thereof in medicine.

## BACKGROUND

Chronic neuropathic pain (CNP) is pain caused by nerve injuries arisen from various causes, such as long-term diabetes mellitus, certain viral infection, cancer, central nervous injury, use of certain chemotherapy drugs. Diabetic peripheral neuropathic pain (DPNP) and postherpetic neuralgia (PHN) are two most common types of the chronic neuropathic pain. The untreated or poorly treated chronic neuropathic pain will cause great pain to the body of a patient, have a great negative impact on the mood of the patient, and lead to mental and psychological problems, such as insomnia, anxiety, and depression, thereby significantly reducing the life quality of the patient and imposing a great burden on the family and the society.

Currently, there are three kinds of drugs mainly used for treating the chronic neuropathic pain: antidepressant drugs, anticonvulsant drugs (antiepileptic drugs) and analgesic drugs. The antidepressant drugs used for treating the chronic neuropathic pain can be roughly divided into tricyclic antidepressant drugs and other antidepressant drugs. The tricyclic antidepressant drugs include amitriptyline, maprotiline, clomipramine, doxepin, etc. The tricyclic antidepressant drugs have many side effects, such as an anticholinergic effect (dry mouth, constipation, blurred vision, hypersomnia, weight gain, etc.), central nervous system toxicity (inattention, epilepsy seizures, abnormal social behavior, hallucination, etc.), and cardiovascular system toxicity (hypotension, tachycardia, arrhythmia, etc.). Such drugs have many precautions for concomitant medication and relatively complicated drug interactions. The other antidepressant drugs are mostly selective 5-hydroxytryptamine and/or norepinephrine reuptake inhibitors, such as imipramine, paroxetine, fluoxetine, escitalopram, duloxetine, bupropion, venlafaxine, sertraline. The antidepressant drugs also have many precautions for concomitant medication and complicated drug interactions, which result in many challenges to clinical medication and patient compliance. The antiepileptic drugs used for treating the chronic neuropathic pain are mainly sodium ion channel drugs and calcium ion channel drugs, such as gabapentin, pregabalin, lamotrigine, topiramate, carbamazepine, oxcarbazepine, sodium valproate. The dosage of gabapentin is extremely high, and it needs to be used in a range of 1,800-3,600 mg per day to achieve a good effect, which has an absorption saturation phenomenon in the high dose range and takes on action slowly (takes on action after oral administration for two weeks). Sodium ion channel blockers, represented by lamotrigine and topiramate etc., exhibit many adverse reactions, such as rash, nausea and vomiting, dizziness and fatigue, and blurred vision, and have many precautions for concomitant medication and also relatively complicated drug interactions. The analgesic drugs used for treating the chronic neuropathic pain include opioids, tramadol, tapentadol, etc., wherein action mechanisms of the latter two include a considerable proportion of action mechanisms of the opioids. The opioid drugs have a certain, but not strong, effect on the neuropathic pain, and have many side effects and addiction. Studies have shown that when being used for treating the diabetic peripheral neuropathic pain at 60 mg/day and 120 mg/day, the duloxetine has clinical effective rates of only 49% and 52%, respectively (Goldstein, D. J.; et al. Pain, 2005, 116(1-2), 109-118.); when being used at daily doses of up to 1,800 mg/day, 2,400 mg/day and 3,600 mg/day, the gabapentin has clinical effective rates of 32%, 34% and 43% for the postherpetic neuralgia, respectively (Rice, A. S. C.; et al. Pain, 2001, 94(2), 215-224; Rowbotham, M.; et al. JAMA, 1998, 280(21), 1837-1842.); and when being used at 150-600 mg per day, the pregabalin has a clinical effective rate of 26%-50% for the postherpetic neuralgia (Dworkin, R. H.; et al. Neurology, 2003, 60(8), 1274-1283; Sabatowski, R.; et al. Pain, 2004, 109(1-2), 26-35.). These extremely low clinical effective rate data all reflect the dilemmas of therapeutic effects of currently marketed drugs that there are no specific drugs for the diseases currently, and there are no simple treatment solutions capable of preventing or reversing neuropathy or completely relieving the pain. A voltage-gated calcium ion channel $\alpha2\delta$ subunit is an important target for the therapeutic drugs of the diseases, and among the four drugs (pregabalin, duloxetine, fluoxetine, and talpentadol) used for the diabetic peripheral neuropathic pain approved by FDA of the United States, pregabalin acts on the target (Field, M.J.; et al. Proc. Natl. Acad. Sci. U.S.A. 2006, 103, 17537-17542). Voltage-gated calcium ion channel $\alpha2\delta$ subunit ligand drugs, such as gabapentin, pregabalin and mirogabalin, can also be used for resisting epilepsy (pregabalin, indications approved by FDA of the United States) and resisting anxiety (pregabalin, indications approved by EMA of the Europe) in addition to being used for treating the chronic neuropathic pain.

The present application discloses a $\gamma$-aminobutyric acid derivative comprising a polycyclic structure, and the compound has a strong inhibition effect on the human voltage-gated calcium ion channel $\alpha2\delta$ subunit when being combined with [$^3$H] gabapentin and can be used for preparing a drug for treatment of the chronic neuropathic pain, epilepsy and anxiety.

## SUMMARY OF THE INVENTION

One objective of the present application is to provide a voltage-gated calcium ion channel $\alpha 2\delta$ subunit ligand of general formula **I,** a chiral isomer thereof and a pharmacologically acceptable salt thereof.

Another objective of the present application is to provide a method of preparing the above voltage-gated calcium ion channel $\alpha 2\delta$ subunit ligand of general formula **I,** a chiral isomer thereof and a pharmaceutically acceptable salt thereof.

Yet another objective of the present application is to provide use of the above compound of general formula **I,** a chiral isomer thereof and a pharmaceutically acceptable salt thereof in the treatment of chronic neuropathic pain, epilepsy and anxiety.

Yet another objective of the present application is to provide a pharmaceutical composition, comprising the compound of general formula **I,** a chiral isomer thereof and a pharmaceutically acceptable salt thereof as an effective ingredient, and one or more of a pharmaceutically acceptable carrier, an excipient, a diluent, or a combination thereof.

Yet another objective of the present application is to provide use of the above pharmaceutical composition in the treatment of chronic neuropathic pain, epilepsy and anxiety.

Contents of the present application are described in details in conjunction with the objectives of the present application.

The compound of general formula **I** in the present application has the following structural formula:

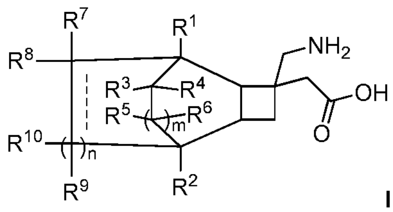

**I**

wherein

$R^1$ and $R^2$ are independently selected from H, halogen, and $C_1$-$C_6$ alkyl;
each of $R^3$, $R^4$, $R^5$ and $R^6$ is independently selected from H, halogen, $C_1$-$C_6$ alkyl, and $C_1$-$C_6$ alkoxy; or $R^3$ and $R^4$ together with a C atom to which they are connected form $C_3$-$C_6$ cycloalkyl, or $R^5$ and $R^6$ together with a C atom to which they are connected form $C_3$-$C_6$ cycloalkyl;
each of $R^7$, $R^8$, $R^9$ and $R^{10}$ is independently selected from H, halogen, and $C_1$-$C_6$ alkyl;
a chemical bond between atoms can be a single bond or a double bond; when the chemical bond represents the double bond, $R^7$ and $R^9$ are absent;
m and n are independently selected from 0, 1, 2, and 3;
alternatively, when n≥1, a C atom connected to $R^8$ and an adjacent C atom connected to $R^{10}$ are capable of forming $C_3$-$C_6$ cycloalkyl together with $R^8$ and $R^{10}$;
when n≥1, a solid line and a dashed line between the C atom connected to $R^8$ and the adjacent C atom connected to $R^{10}$ represent that a chemical bond between the C atom connected to $R^8$ and the C atom connected to $R^{10}$ can be a single bond or a double bond; and when the chemical bond represents the double bond, corresponding $R^7$ and $R^9$ are absent.

According to the present application, the following compound of general formula I, a chiral isomer thereof or a pharmaceutically acceptable salt thereof is preferred, wherein

$R^1$ and $R^2$ are independently selected from H and $C_1$-$C_3$ alkyl;
$R^3$ and $R^4$ are independently selected from H, halogen, and $C_1$-$C_3$ alkyl; or $R^3$ and $R^4$ together with the C atom to which they are connected form cyclopropyl;
$R^7$, $R^8$, $R^9$ and $R^{10}$ are independently selected from H and $C_1$-$C_6$ alkyl; or the C atom connected to $R^8$ and the C atom connected to $R^{10}$ form cyclopropyl together with $R^8$ and $R^{10}$;
the solid line and the dashed line between the C atom connected to $R^8$ and the C atom connected to $R^{10}$ represent that the chemical bond between the C atom connected to $R^8$ and the C atom connected to $R^{10}$ can be the single bond or the double bond; when the chemical bond represents the double bond, $R^7$ and $R^9$ are absent;
m=0; and n=1.

According to the present application, the following compound of general formula **I,** a chiral isomer thereof or a pharmaceutically acceptable salt thereof is more preferred, wherein

$R^1$ and $R^2$ are independently selected from H or methyl;

$R^3$ and $R^4$ are independently selected from H and methyl; or $R^3$ and $R^4$ together with the C atom to which they are connected form cyclopropyl;

$R^7$, $R^8$, $R^9$ and $R^{10}$ are independently selected from H or methyl; or the C atom connected to $R^8$ and the C atom connected to $R^{10}$ form cyclopropyl together with $R^8$ and $R^{10}$;

the solid line and the dashed line between the C atom connected to $R^8$ and the C atom connected to $R^{10}$ represent that the chemical bond between the C atom connected to $R^8$ and the C atom connected to $R^{10}$ can be the single bond or the double bond; when the chemical bond represents the double bond, $R^7$ and $R^9$ are absent;

m=0; and n=1.

According to the present application, the following compound of general formula **I,** a chiral isomer thereof or a pharmaceutically acceptable salt thereof is more preferred, wherein

$R^1$ and $R^2$ are independently selected from H or methyl;

$R^3$ and $R^4$ together with the C atom to which they are connected form cyclopropyl;

$R^7$, $R^8$, $R^9$ and $R^{10}$ are independently selected from H or methyl;

m=0; and n=1.

According to the present application, the following compound of general formula **I,** a chiral isomer thereof or a pharmaceutically acceptable salt thereof is more preferred, wherein

$R^1$ and $R^2$ are independently selected from H or methyl;

$R^3$ and $R^4$ are independently selected from H and methyl;

$R^7$ and $R^9$ are independently selected from H or methyl; the C atom connected to $R^8$ and the C atom connected to $R^{10}$ form cyclopropyl together with $R^8$ and $R^{10}$;

m=0; and n=1.

According to the present application, the following compound of general formula **I,** a chiral isomer thereof or a pharmaceutically acceptable salt thereof is more preferred, wherein

$R^1$ and $R^2$ are independently selected from H or methyl;

$R^3$ and $R^4$ are independently selected from H and methyl;

$R^7$ and $R^9$ are independently selected from H or methyl; the C atom connected to $R^8$ and the C atom connected to $R^{10}$ form cyclopropyl together with $R^8$ and $R^{10}$;

m=0; and n=1.

According to the present application, the following compounds are more preferred:

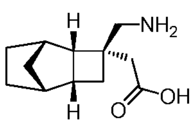 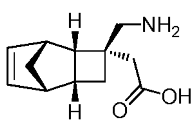 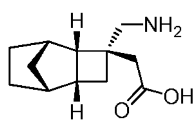 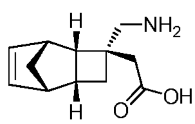 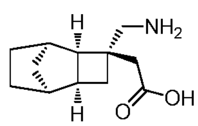

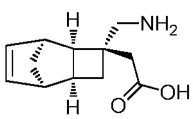 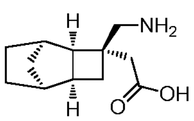 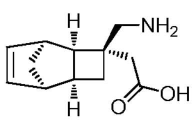 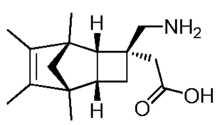 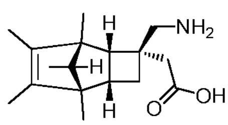

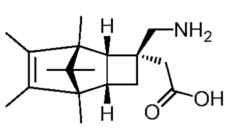 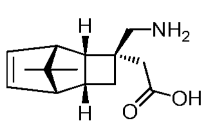 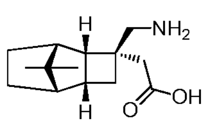 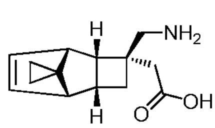 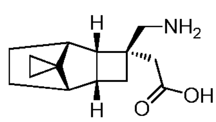

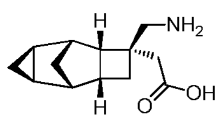

The compound of general formula **I** in the present application can be synthesized through the following methods.

In one typical case, ketone **K** and phosphonoacetate **W1** undergo a Wittig condensation reaction in the presence of a base to obtain $\alpha,\beta$-unsaturated acetate **L-1,** and the base is selected from an inorganic base and an organic base; wherein $R^{11}$ and $R^{12}$ are selected from $C_1$-$C_6$ alkyl; $R^1$-$R^{10}$, m and n have definitions as described above; and L-1 is a mixture of two cis/trans geometric isomers.

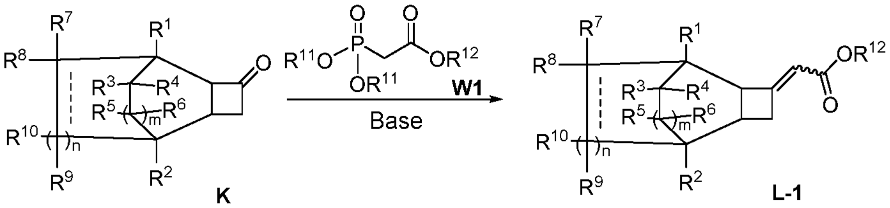

K
L-1

When the solid line and the dashed line between the C atom connected to $R^8$ and the C atom connected to $R^{10}$ in **L-1** represent that the chemical bond between the C atom connected to $R^8$ and the C atom connected to $R^{10}$ is the double bond, **L-1** is **L-1-1.** At this time, **L-1-1** can be converted into **L-1-2** using a Simmons-Smith reaction or a similar reaction. At this time, the C atom connected to $R^8$ and the C atom connected to $R^{10}$ in **L-1-2** form cyclopropyl together with $R^8$ and $R^{10}$. **L-1-2** is a specific case of **L-1.** The Simmons-Smith reaction or the similar reaction includes treating a substrate containing a C=C double bond with $CH_2I_2/Et_2Zn$, $CH_2I_2/Et_2Zn$/trifluoroacetic acid, or $CH_2I_2$/Cu-Zn to obtain a cyclopropyl product.

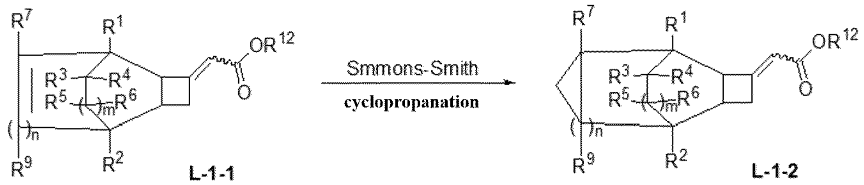

L-1-1
L-1-2

**L-1** and nitromethane undergo a Michael addition reaction in the presence of a base to obtain **M-1,** and a chiral center newly generated in **M-1** is affected by a chiral center derived from **K.** To distinguish configurations of chiral centers generated at corresponding positions by the following other methods, a configuration of the chiral center newly generated in **M-1** is marked as *R** herein. The base is selected from various inorganic bases and organic bases.

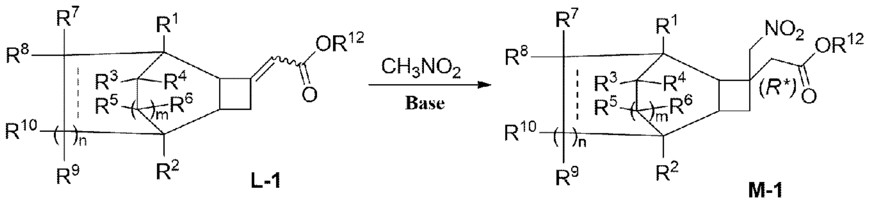

L-1
M-1

For **M-1,** it can be divided into the following three cases:

**M-1-1**: the solid line and the dashed line between the C atom connected to $R^8$ and the C atom connected to $R^{10}$ represent that the chemical bond between the C atom connected to $R^8$ and the C atom connected to $R^{10}$ is the double bond;
**M-1-2:** the C atom connected to $R^8$ and the C atom connected to $R^{10}$ form cyclopropyl together with $R^8$ and $R^{10}$;
**M-1-3:** the solid line and the dashed line between the C atom connected to $R^8$ and the C atom connected to $R^{10}$ represent that the chemical bond between the C atom connected to $R^8$ and the C atom connected to $R^{10}$ is the single bond.

For **M-1-1:** an ester bond in **M-1-1** is subjected to hydrolysis with an acid or a base, etc., to obtain **N-1-1, and N-1-1** is subjected to simultaneous reduction of a nitro group and a C=C double bond by using a catalytic hydrogenation method to obtain (*R**)-**I-1-3.** *(R*)*-**I-1-3** is a specific form of the compound of general formula **I** in the present application. (*R**)-**I-1-3** reacts with acid HA to obtain corresponding salt (*R**)-**I-1-3**·HA, wherein the acid HA is selected from various inorganic acids and organic acids. **M-1-1** is firstly subjected to reduction of a nitro group with iron powder to obtain **P-1-1,** and **P-1-1** is subjected to hydrolysis of an ester bond with an acid to obtain *(R*)*-**I-1-1**. *(R*)*-**I-1-1** is a specific form of the compound of general formula **I** in the present application. (R*)-I-1-1 reacts with acid HA to obtain corresponding salt (*R**)-**I-1-1**·HA, wherein the acid HA is selected from various inorganic acids and organic acids.

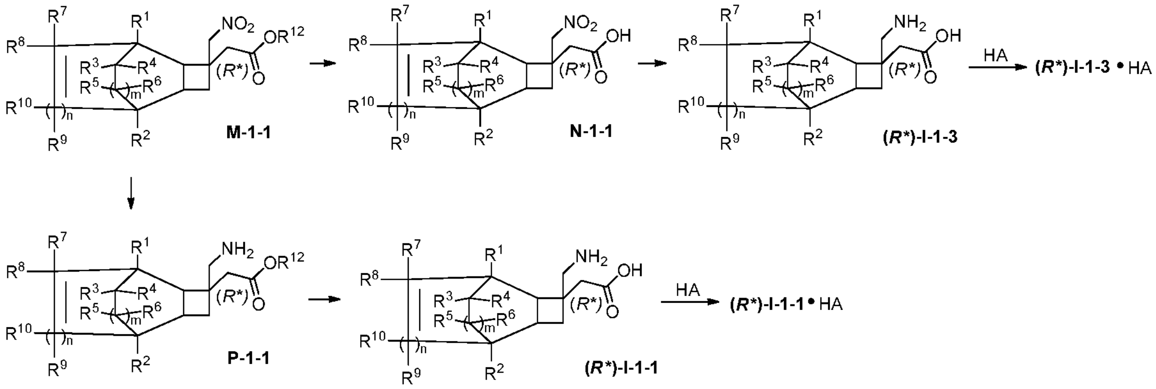

M-1-1 N-1-1 (*R**)-I-1-3 (*R**)-I-1-3 • HA
P-1-1 (*R**)-I-1-1 (*R**)-I-1-1 • HA

For **M-1-2:** an ester bond in **M-1-2** is subjected to hydrolysis with an acid or a base, etc., to obtain **N-1-2, and N-1-2** is subjected to reduction of a nitro group by using a catalytic hydrogenation method to obtain (*R**)-**I-1-2**. (*R**)-**I-1-2** is a specific form of the compound of general formula **I** in the present application. (*R**)-**I-1-2** reacts with acid HA to obtain corresponding salt (*R**)-**I-1-2**·HA, wherein the acid HA is selected from various inorganic acids and organic acids. **M-1-2** is firstly subjected to reduction of a nitro group with iron powder to obtain **P-1-2,** and **P-1-2** is subjected to hydrolysis of an ester bond with an acid to obtain (*R**)-**I-1-2**. (*R**)-**I-1-2** is a specific form of the compound of general formula **I** in the present application.

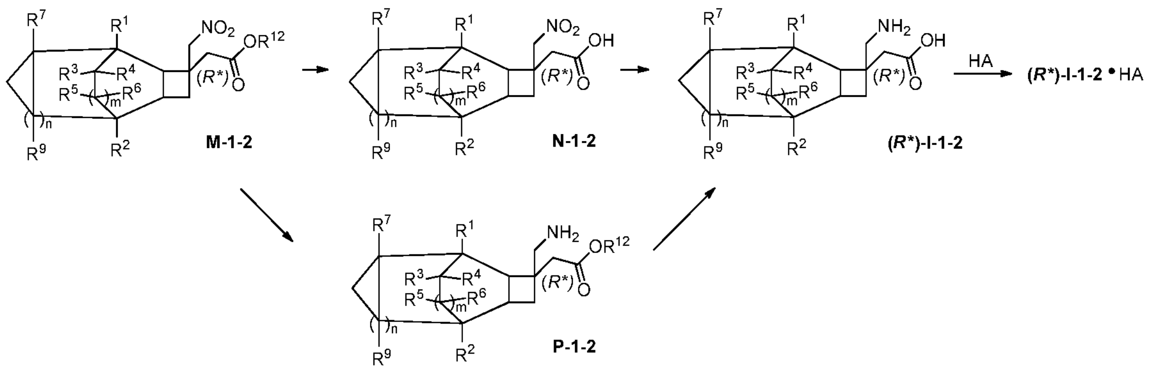

M-1-2 N-1-2 (*R**)-I-1-2 (*R**)-I-1-2 • HA
P-1-2

(*R**)-**I-1-2** reacts with acid HA to obtain the corresponding salt (*R**)-**I-1-2**·HA, wherein the acid HA is selected from various inorganic acids and organic acids.

For **M-1-3:** an ester bond in **M-1-3** is subjected to hydrolysis with an acid or a base, etc., to obtain **N-1-3,** and **N-1-3** is subjected to reduction of a nitro group by using a catalytic hydrogenation method to obtain (*R**)-**I-1-3**. (*R**)-**I-1-3** is a specific form of the compound of general formula **I** in the present application. (*R**)-**I-1-3** reacts with acid HA to obtain the corresponding salt (*R**)-**I-1-3**·HA, wherein the acid HA is selected from various inorganic acids and organic acids. **M-1-3** is firstly subjected to reduction of a nitro group with iron powder to obtain **P-1-3,** and **P-1-3** is subjected to hydrolysis of an ester bond with an acid to obtain (*R**)-**I-1-3**. (*R**)-**I-1-3** is a specific form of the compound of general formula **I** in the present application. (*R**)-**I-1-3** reacts with acid HA to obtain the corresponding salt (*R**)-**I-1-3**·HA, wherein the acid HA is selected from various inorganic acids and organic acids.

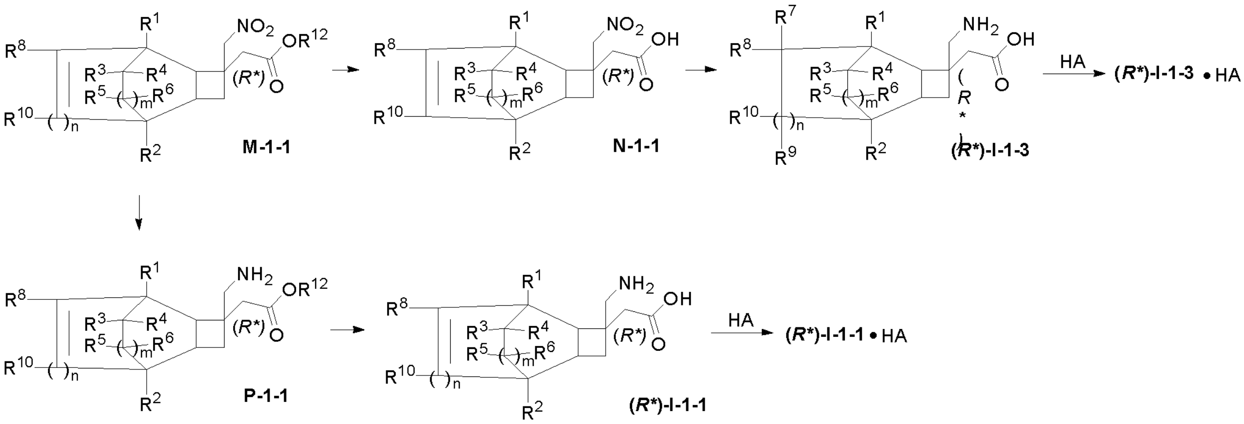


In another typical case, the ketone **K** and nitromethane undergo a Knoevenagel condensation reaction in the presence of a catalyst to obtain α,β-unsaturated nitro compound **L-2,** wherein the catalyst is selected from various inorganic bases and organic bases. **L-2** is a mixture of two cis/trans geometric isomers.

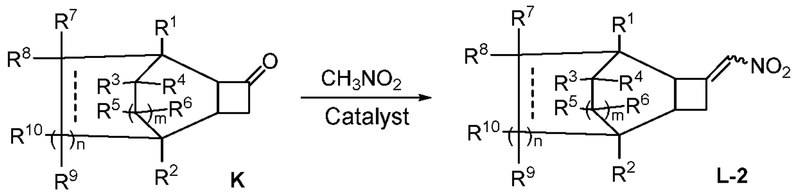


**L-2** and acetate **W2** undergo a Michael-like addition reaction in the presence of a strong base to obtain **M-2,** and a chiral center newly generated in **M-2** is affected by a chiral center derived from **K.** To be distinguished from the configuration of the chiral center generated at the corresponding position in the above reaction of generating **M-1** from **L-1,** a configuration of the chiral center newly generated in **M-2** is marked as S* herein. The strong base is selected from tert-butyl lithium, lithium diisopropylamide, lithium bis(trimethylsilyl)amide, sodium bis(trimethylsilyl)amide, and potassium bis(trimethylsilyl)amide; $R^{13}$ is selected from $C_1$-$C_6$ alkyl; and S* and R* represent that the configurations of the marked chiral centers are opposite.

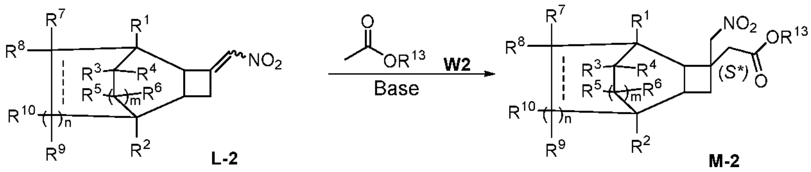


For **M-2,** it can be divided into the following three cases:

**M-2-1:** the solid line and the dashed line between the C atom connected to $R^8$ and the C atom connected to $R^{10}$ represent that the chemical bond between the C atom connected to $R^8$ and the C atom connected to $R^{10}$ is the double bond;
**M-2-2:** the C atom connected to $R^8$ and the C atom connected to $R^{10}$ form cyclopropyl together with $R^8$ and $R^{10}$;
**M-2-3:** the solid line and the dashed line between the C atom connected to $R^8$ and the C atom connected to $R^{10}$ represent that the chemical bond between the C atom connected to $R^8$ and the C atom connected to $R^{10}$ is the single bond.

For **M-2-1:** an ester bond in **M-2-1** is subjected to hydrolysis with an acid or a base, etc., to obtain **N-2-1, and N-2-1** is subjected to simultaneous reduction of a nitro group and a C=C double bond by using a catalytic hydrogenation method to obtain (*S**)-**I-2-3**. (*S**)-**I-2-3** is a specific form of the compound of general formula **I** in the present application. (*S**)-**I-2-3** reacts with acid HA to obtain corresponding salt (*S**)-**I-2-3**·HA, wherein the acid HA is selected from various inorganic acids and organic acids. **M-2-1** is firstly subjected to reduction of a nitro group with iron powder to obtain **P-2-1,** and **P-2-1** is subjected to hydrolysis of an ester bond with an acid to obtain (*S**)-**I-2-1**. (*S**)-**I-2-1** is a specific form of the compound of general formula **I** in the present application. (*S**)-**I-2-1** reacts with acid HA to obtain corresponding salt (*S**)-**I-2-1**·HA,

wherein the acid HA is selected from various inorganic acids and organic acids.

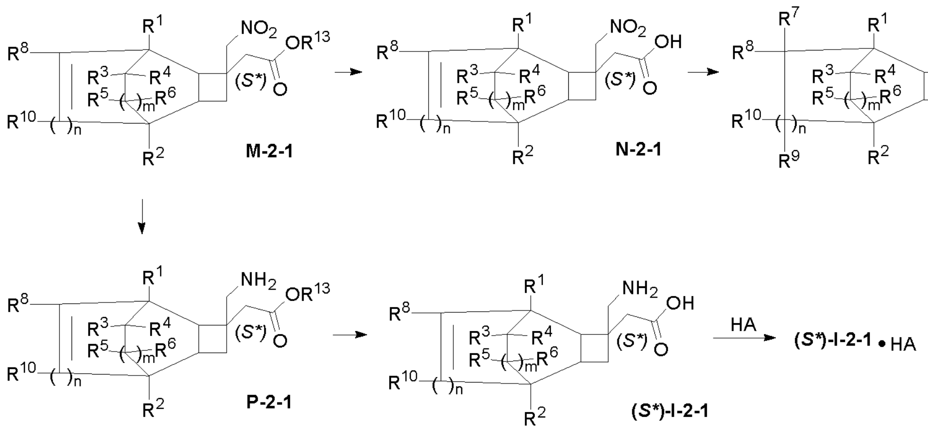


For **M-2-2:** an ester bond in **M-2-2** is subjected to hydrolysis with an acid or a base, etc., to obtain **N-2-2,** and **N-2-2** is subjected to reduction of a nitro group by using a catalytic hydrogenation method to obtain (*S**)-**I-2-2**. (*S**)-**I-2-2** is a specific form of the compound of general formula **I** in the present application. (*S**)-**I-2-2** reacts with acid HA to obtain corresponding salt (*S**)-**I-2-2**·HA, wherein the acid HA is selected from various inorganic acids and organic acids. **M-2-2** is firstly subjected to reduction of a nitro group with iron powder to obtain **P-2-2,** and **P-2-2** is subjected to hydrolysis of an ester bond with an acid to obtain (*S**)-I-**2-2.** (*S**)-**I-2-2** is a specific form of the compound of general formula **I** in the present application. (*S**)-**I-2-2** reacts with acid HA to obtain the corresponding salt (*S**)-**I-2-2**·HA, wherein the acid HA is selected from various inorganic acids and organic acids.

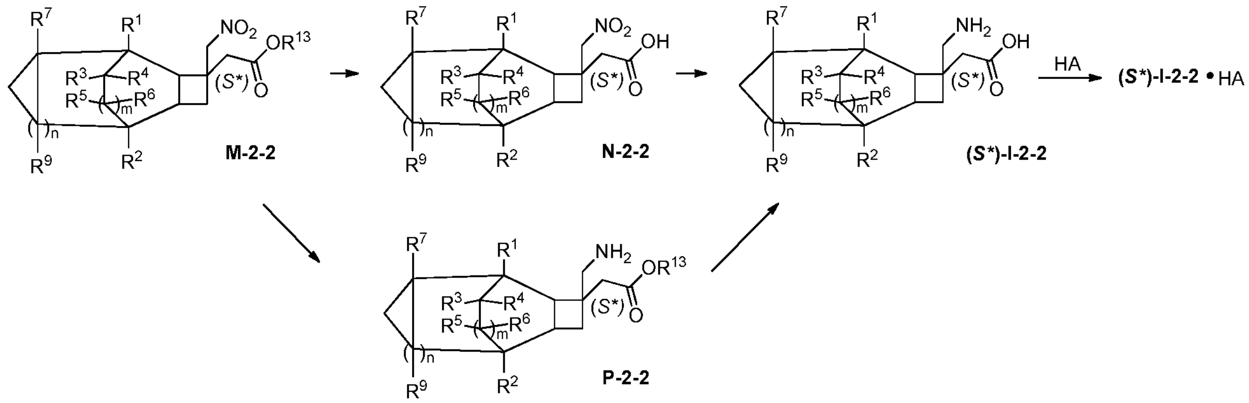


For **M-2-3:** an ester bond in **M-2-3** is subjected to hydrolysis with an acid or a base, etc., to obtain **N-2-3,** and **N-2-3** is subjected to simultaneous reduction of a nitro group by using a catalytic hydrogenation method to obtain (*S**)-**I-2-3**. (*S**)-**I-2-3** is a specific form of the compound of general formula **I** in the present application. (*S**)-**I-2-3** reacts with acid HA to obtain the corresponding salt (*S**)-**I-2-3**·HA, wherein the acid HA is selected from various inorganic acids and organic acids. **M-2-3** is firstly subjected to reduction of a nitro group with iron powder to obtain **P-2-3, and P-2-3** is subjected to hydrolysis of an ester bond with an acid to obtain (*S**)-**I-2-3**. (*S**)-**I-2-3** is a specific form of the compound of general formula **I** in the present application. (*S**)-**I-2-3** reacts with acid HA to obtain the corresponding salt (*S**)-**I-2-3**·HA, wherein the acid HA is selected from various inorganic acids and organic acids.

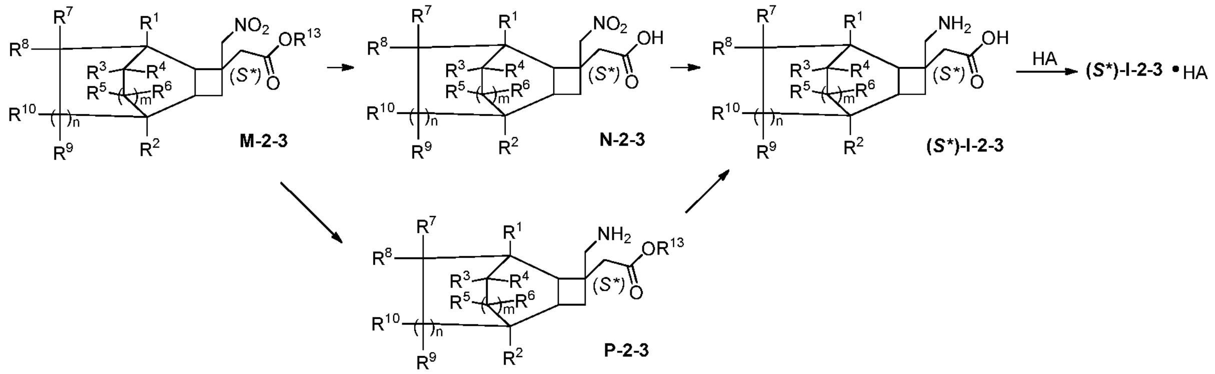


To prepare the optically pure final product **I,** a racemic intermediate composed of a pair of enantiomers with a same relative configuration can be used for resolution.

In one typical example, racemic intermediate (±)-**N-acid** composed of a pair of enantiomers with a same relative configuration in the above synthesis route is subjected to salifying resolution in a suitable solvent using suitable **chiral base-A** and **chiral base-B** to obtain precipitable salts (+)-**N-acid·chiral base-A** and (-)-**N-acid·chiral base-B** with sufficient optical purity, respectively, and the two are treated with diluted hydrochloric acid to remove the **chiral base-A** and the **chiral base-B** to obtain (+)-**N-acid** and **(-)-N-acid,** respectively. (+)-**N-acid** and **(-)-N-acid** are subjected to reduction of a nitro group by using a catalytic hydrogenation reduction method to obtain optically pure products (+)-**I-A** and **(-)-I-A,** respectively. (+)-**I-A** and **(-)-I-A** are specific forms of the compound of general formula **I** in the present application, respectively. (+)-**I-A** and **(-)-I-A** react with acid HA to obtain corresponding salts (+)-**I-A**·HA and (-)-**I-A**·HA, respectively, wherein the acid HA is selected from various inorganic acids and organic acids.

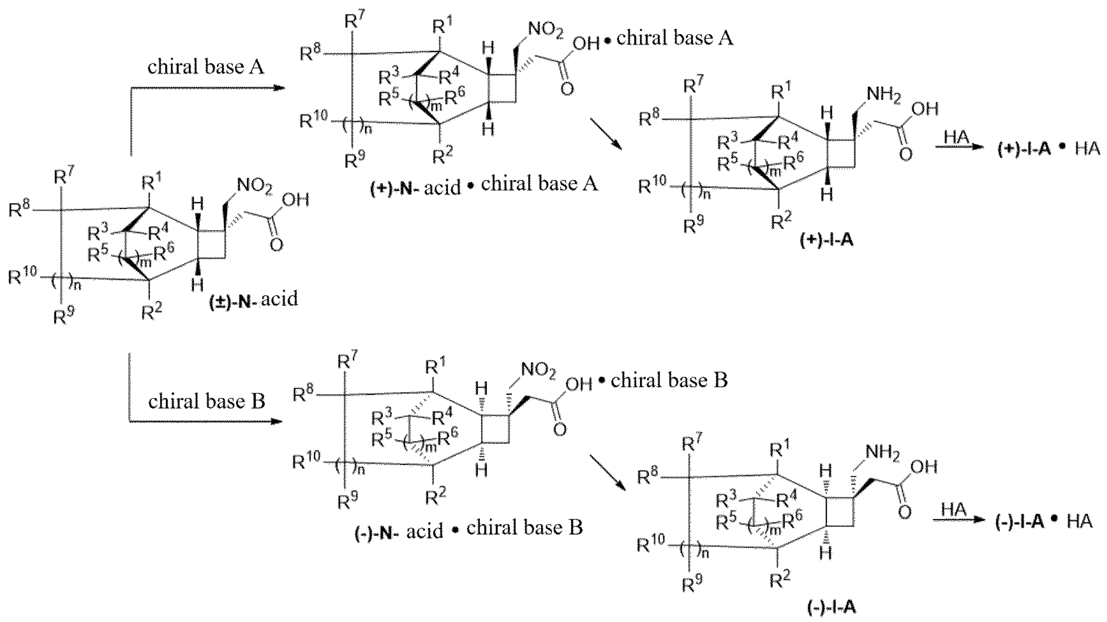


In another typical example, racemic intermediate (±)-**P-NH2** composed of a pair of enantiomers with a same relative configuration in the above synthesis route is subjected to salifying resolution in a suitable solvent using suitable **chiral acid-A** and **chiral acid-B** to obtain precipitable salts (+)-**P-NH2·chiral acid-A** and (-)-**P-NH2·chiral acid-B** with sufficient optical purity, respectively, and the two are treated with a sodium bicarbonate aqueous solution to remove the **chiral acid-A** and the **chiral acid-B** to obtain (+)-**P-NH2** and **(-)-P-NH2,** respectively. (+)-**P-NH2** and **(-)-P-NH2** are subjected to hydrolysis of tert-butyl ester with an acid to obtain optically pure products (+)-**I-A1** and (-)-**I-A1**, respectively. (+)-**I-A1** and (-)-**I-A1** are specific forms of the compound of general formula I in the present application, respectively. (+)-**I-A1** and (-)-**I-A1** react with acid HA to obtain corresponding salts **(+)-I-A1**·HA and (-)-**I-A1**·HA, respectively, wherein the acid HA is selected from various inorganic acids and organic acids.

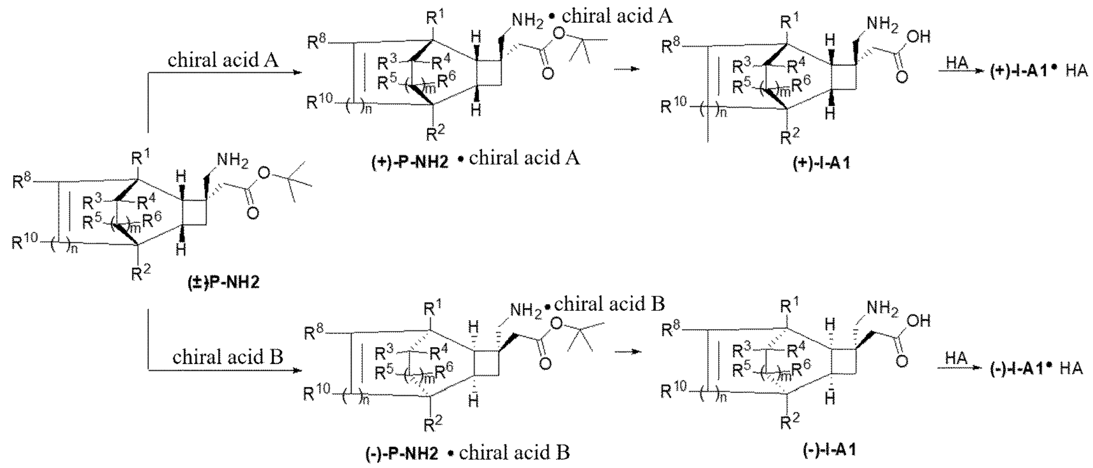

(±)P-NH2
(+)-P-NH2 • chiral acid A
(+)-I-A1
(+)-I-A1• HA
(-)-P-NH2 • chiral acid B
(-)-I-A1
(-)-I-A1• HA

In one typical example, **K-1** can be synthesized according to the following method, and **K-1** is a specific form of the aforementioned compound of general formula **K.** Compound **CDE** and compound **Q-1** undergo a Diels-Alder reaction to obtain compound **R**, wherein Z is selected from NH, O, and S, $R^{14}$ is selected from H and $C_1$-$C_6$ alkyl, or $-ZR^{14}$-$R^{14}Z$-=O, and at this time, the compound **Q-1** is maleic anhydride. The compound **R** reacts with alcohol $R^{15}OH$ under the catalysis of an acid to obtain compound **S,** wherein $R^{15}$ is selected from $C_1$-$C_6$ alkyl. The compound **S** undergoes a reaction (hydroxyketone condensation reaction) with metallic sodium and trimethylchlorosilane in a refluxed inert solvent, and the resulting product is subjected to hydrolysis with an acid to obtain compound **T.** The compound **T** is treated with $PPh_3$ in refluxed $CX_4$ to obtain compound **U-1,** wherein X is selected from Cl, Br, and I. The compound **U-1** is treated with Zn powder in an acidic medium to obtain the compound **K-1.**

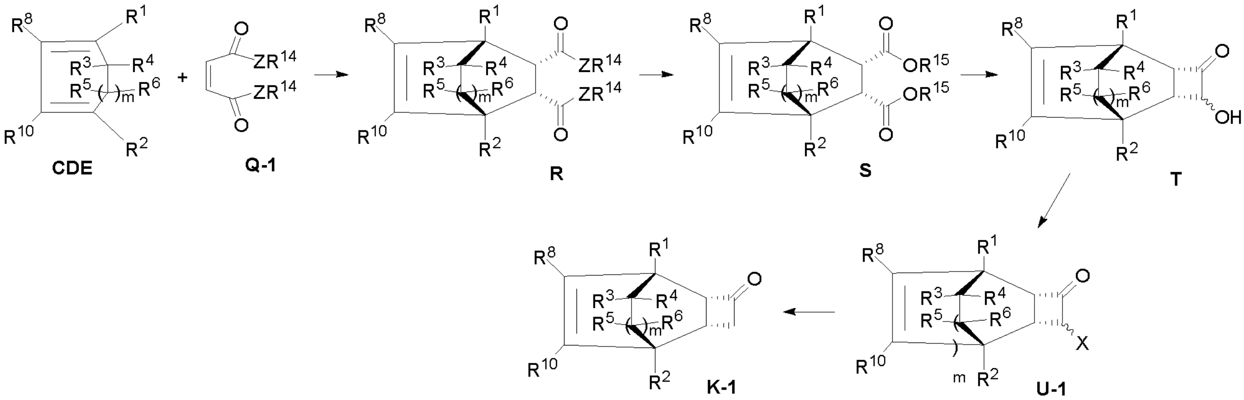
CDE Q-1 R S T U-1 K-1

In another typical example, **K-1** can be synthesized according to the following method. The compound **CDE** and compound **Q-2** undergo a Diels-Alder reaction to obtain the compound **K-1.**

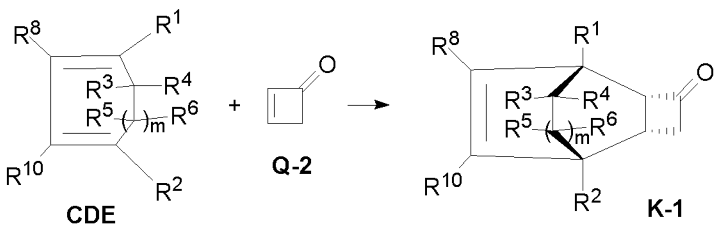
CDE Q-2 K-1

Compound **ALE** reacts with dichloroketene to obtain compound **U-2,** wherein dichloroketene can be prepared by a reaction between trichloroacetyl chloride and activated zinc powder or a reaction between dichloroacetyl chloride and triethylamine. **U-2** is treated with Zn powder in an acidic medium to obtain compound **K-2,** and **K-2** is a specific form of the aforementioned compound of general formula **K.**

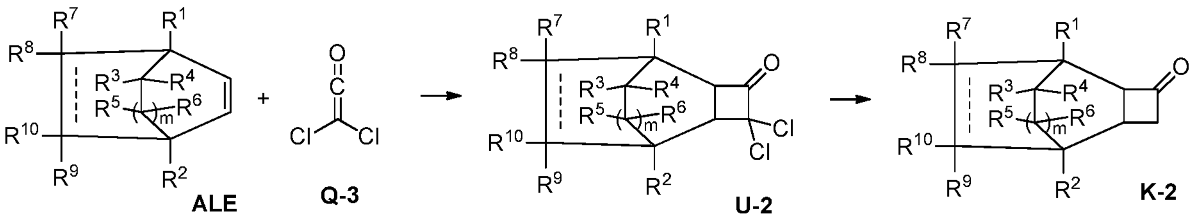

ALE Q-3 U-2 K-2

The compound **K-1** is subjected to reduction of a C=C double bond through catalytic hydrogenation to obtain compound **K-3. K-1** is converted into **K-4** using a Simmons-Smith reaction, and the Simmons-Smith reaction and similar reactions include treating a substrate containing a C=C double bond with $CH_2I_2/Et_2Zn$, $CH_2I_2/Et_2Zn$/trifluoroacetic acid, or $CH_2I_2$/Cu-Zn to obtain a cyclopropyl product. **K-3** and **K-4** are specific forms of the aforementioned compound of general formula **K,** respectively.

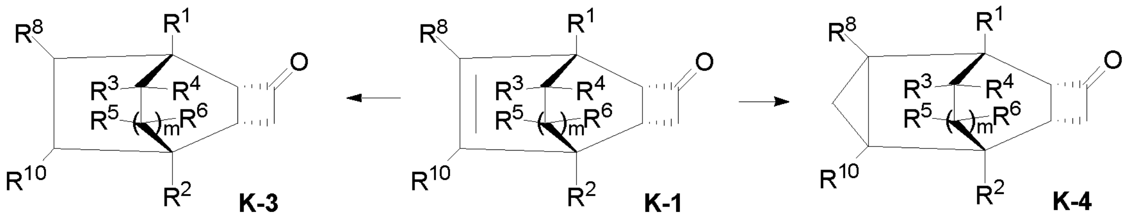

K-3 K-1 K-4

The "halogen" in the present application refers to a fluorine atom, a chlorine atom, a bromine atom, or an iodine atom.

The "alkyl" in the present application refers to a group derived from a branched or linear saturated aliphatic alkane with a specified number of carbon atoms after removal of one hydrogen. For example, "$C_{1-6}$ alkyl" refers to the inclusion of $C_1$, $C_2$, $C_3$, $C_4$, $C_5$, and $C_6$ alkyl; and specific examples include, but are not limited to, methyl, ethyl, n-propyl, isopropyl, sec-butyl, 2-methylbutyl, 1,1-dimethylbutyl, etc.

The "alkoxy" in the present application means that the alkyl as defined herein is connected to other groups through an oxygen atom, i.e., "alkyl-O-", including "$C_{1-6}$ alkoxy" (with the structure of $C_{1-6}$ alkyl-O-) and "$C_{1-4}$ alkoxy"; specific examples include, but are not limited to, methoxy, ethoxy, propoxy, 1-methylethoxy, butoxy, 2-methylbutoxy, 3-methylbutoxy, 1,1-dimethylpropoxy, 1,2-dimethylpropoxy, etc.; and preferably, the "alkoxy" in the present application is $C_{1-4}$ alkoxy, more preferably $C_{1-3}$ alkoxy.

The "cycloalkyl" in the present application refers to a saturated cyclic alkyl derived from a cycloalkane after removal of one hydrogen atom. The cycloalkyl includes "3-6 membered cycloalkyl", "3-5 membered cycloalkyl". Preferably, the cycloalkyl has a monocyclic saturated structure; and specific examples include, but are not limited to, cyclopropyl, cyclobutyl, cyclopentyl, and cyclohexyl.

The pharmaceutically acceptable salt of the compound of general formula **I** in the present application includes, but is not limited to, pharmaceutically acceptable salts generated by the compound of general formula **I** with various inorganic bases, e.g., NaOH, KOH, $Mg(OH)_2$, $Ca(OH)_2$, $Sr(OH)_2$, $Al(OH)_3$, etc., or inorganic carbonates, e.g., $Na_2CO_3$, $K_2CO_3$, $MgCO_3$, $CaCO_3$, $SrCO_3$, etc., or organic bases, e.g., amino acids, etc., or inorganic acids, e.g., hydrochloric acid, sulfuric acid, nitric acid, hydrobromic acid, hydroiodic acid, etc., or organic acids, e.g., methanesulfonic acid, trifluoromethanesulfonic acid, benzenesulfonic acid, p-toluenesulfonic acid, trifluoroacetic acid, malic acid, citric acid, etc.

The compound of general formula **I** in the present application can be combined with one or more of pharmaceutically acceptable auxiliary materials to prepare a pharmaceutical composition. The pharmaceutical composition can be prepared into a solid oral preparation, a liquid oral preparation, an injection and other dosage forms. The solid oral preparation and the liquid oral preparation include a tablet, a dispersible tablet, a sugar-coated formulation, a granule, dry powder, a capsule, and a solution. The injection includes a small volume parenteral (SVP) solution, an infusion solution, water for injection, freeze-dried powder for injection, etc.

The compound of the present application has chiral isomers, such as enantiomers, diastereoisomers, racemic mixtures and other mixtures, and all of these mixtures fall within the scope of the present application.

The term "enantiomer" refers to stereoisomers with a mirror image relationship.

The term "diastereoisomer" refers to stereoisomers in which molecules have two or more chiral centers and the molecules are in a non-mirror image relationship.

(±) represents that a certain compound is a racemic mixture, and at this time, a configuration in a chemical structure of the compound is a relative configuration. (+) or (-) represents that a certain compound is optically pure, an optical rotation symbol represents dextrorotation or levorotation, respectively, and at this time, a configuration in a chemical structure of the compound is an absolute configuration.

The chiral isomers of the compound of the present application can be prepared according to chiral synthesis or chiral reagents of the present application as described above or other conventional technologies. The separation of optically pure compounds in the present application is usually completed by chiral resolution. An optically pure chiral acid and a racemic base are crystallized in a suitable solvent after salifying to obtain an optically pure base and a salt of a chiral acid, so as to achieve the purpose of separating a chiral compound. The term "optically pure" means that a content of an isomer or enantiomer is greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

An absolute stereoscopic configuration of a compound can be confirmed by a conventional technical means in the art. For example, a single crystal X-ray diffraction method is adopted. The absolute configuration of the compound can also be confirmed according to a chiral structure of a raw material and a reaction mechanism of asymmetric synthesis.

According to the composition of the present application, the pharmaceutically acceptable auxiliary material or an acceptable auxiliary material in food science is selected from a carrier, an excipient, a diluent, a binder, a filler, a disintegrant, a lubricant, a glidant, an effervescing agent, a flavoring agent, a preservative, and a coating material.

According to the composition of the present application, the excipient is a substance that is non-toxic, compatible with an active ingredient and applicable to organisms in other biological properties. The selection of a particular excipient depends on an administration mode for treating a particular patient or a disease type and state. Examples of the excipient include, but are not limited to, conventional solvents, dispersants, suspending agents, surfactants, isotonic agents, thickeners, emulsifiers, stabilizers, hydrating agents, emulsifying accelerators, buffers, absorbents, colorants, ion exchangers, release agents, coating agents, antioxidants, etc. in the pharmaceutical field. The filler includes a composition of one or more of lactose, dextrin, starch, pre-gelatinized starch, mannitol, sorbitol, calcium hydrogen phosphate, calcium sulfate, calcium carbonate, and microcrystalline cellulose; the binder includes a composition of one or more of sucrose, povidone, sodium carboxymethyl cellulose, hydroxypropyl methyl cellulose, hydroxypropyl cellulose, methyl cellulose, polyethylene glycol, ethanol, and water; and the disintegrant includes a composition of one or more of crosslinked povidone, crosslinked sodium carboxymethyl cellulose, low-substituted hydroxypropyl cellulose, sodium carboxymethyl cellulose, and an effervescent disintegrant. The compound of general formula **I** in the present application has a binding effect on a voltage-gated calcium ion channel $\alpha 2\delta$ and can be used as an effective ingredient in preparation of a medicament for treatment of chronic neuropathic pain, epilepsy and anxiety. The activity of the compound of general formula **I** in the present application at the *in vitro* level is verified through *in vitro* inhibition of the binding of [$^3$H] gabapentin to a human recombinant calcium ion channel $Ca_V2.2/\beta 3/\alpha 2\delta$-1 receptor expressed on CHO cells, and at the *in vivo* level is verified through an analgesic effect on an animal chronic pain model and an antiepileptic effect on an animal epilepsy model.

The compound of general formula **I** in the present application is effective within a quite wide dose range. For example, a daily dose taken is within a range of about 1-3000 mg/person, and administration is performed for one time or several times. An actually taken dose of the compound of general formula **I** in the present application can be determined by a physician according to actual situations of a patient.

## BRIEF DESCRIPTION OF THE DRAWINGS

The present application is further described below in conjunction with drawings, in which

FIG. 1 shows a chemical structure of compound **(+)-32-LAC** in single crystal diffraction (ORTEP diagram).

FIG. 2 shows a chemical structure of compound (+)-**22-LAC** in single crystal diffraction (ORTEP diagram).

FIG. 3A is a mechanical pain threshold time chart of pharmacodynamic evaluation results of compounds (±)-**I-7,** (±)-**I-3** and (±)-**I-4** in a rat spared nerve injury model; FIG. 3B is a chart of an area under a mechanical pain threshold-time curve of the pharmacodynamic evaluation results of the compounds (±)-**I-7**, **(±)-I-3** and **(±)-I-4** in the rat spared nerve injury model.

FIG. 4A is a mechanical pain threshold time chart of pharmacodynamic evaluation of compounds **(-)-I-3,** (+)-**I-3**, **(-)-I-4** and (+)-**I-4** in a rat spared nerve injury model; FIG. 4B is a chart of an area under a mechanical pain threshold-time curve of the pharmacodynamic evaluation of the compounds (-)-**I-3**, (+)-**I-3**, (-)-**I-4** and (+)-**I-4** in the rat spared nerve injury model.

FIG. 5 is a fitting curve of median effective doses ($ED_{50}$) of compounds for animal protection in a maximum electroshock model of mice calculated by a least square method (Graphpad Prism 5) adopting an administration

dose-protection rate curve.

FIG. 6 shows an antiepileptic effect of compounds (+)-**I-3** and (+)-**I-4** in a mouse epilepsy model (maximum electroshock model (MES)).

FIG. 7 is a curve of impacts of compounds **(+)-I-3** p-toluenesulfonate and (+)-**I-4** p-toluenesulfonate on animal motor functions as measured by a rotarod test.

**EMBODIMENTS OF THE INVENTION**

The contents of the present application are further described in details below through specific examples. It should be noted that the following examples are only used for illustration and are not intended to limit the present application. Various changes made by those skilled in the art in accordance with teachings of the present application shall be within the protection scope claimed by the claims of the present application.

A melting point is measured by using an SGW X-4A microscopic melting point tester (INESA Physico-optical Instrument Co., Ltd, Shanghai, China), without calibrating the thermometer. $^1H$ NMR and $^{13}C$ NMR are detected by a Bruker Ascend 500 NMR spectrometer (Bruker Swiss AG, Fällanden, Swiss) using $CDCl_3$, DMSO-$d_6$, $CD_3OD$ or $D_2O$ as a solvent and a known chemical shift of a carbon signal of TMS (for $^1H$ NMR) or a deuterated solvent (for $^{13}C$ NMR) as an internal standard. A high-resolution mass spectrum is tested by using an electrospray ionization (ESI) technology with a Thermo Q Exactive Plus mass spectrometer (Thermo Fisher Scientific, Bremen, Germany). An optical rotation is determined by using an Anton Paar MCP4100 polarimeter.

Enantiomeric excess (%ee) determination method (chiral HPLC method): Determination is performed using a Daicel Chiralpak AS-RH 4.6 mm×250 mm chromatographic column (5 μm) in an Agilent 1260 infinity Type II liquid chromatograph with a detector wavelength of 220 nm, a mobile phase of acetonitrile/0.1% $KH_2PO_4$-KOH buffer solution (pH=7.0) = 70/30, a flow rate of 1 mL/min, a sample injection concentration of 0.5 mg/mL, and a sample injection volume of 3 μL.

Single crystal diffraction method: A Rigaku XtaLAB Pro single crystal diffractometer is used for diffraction with Cu Kα rays at a temperature of 100.00 (10) K, CrysAlisPro 1.171.39.33c (Rigaku OD, 2017) is used for collecting diffraction data and performing data reduction, and an SHELXL program is used for performing structural analysis and refinement.

Dry solvents are prepared from corresponding analytical pure solvents using a standard drying method.

**Example 1 Synthesis of compound (±)-I-1**

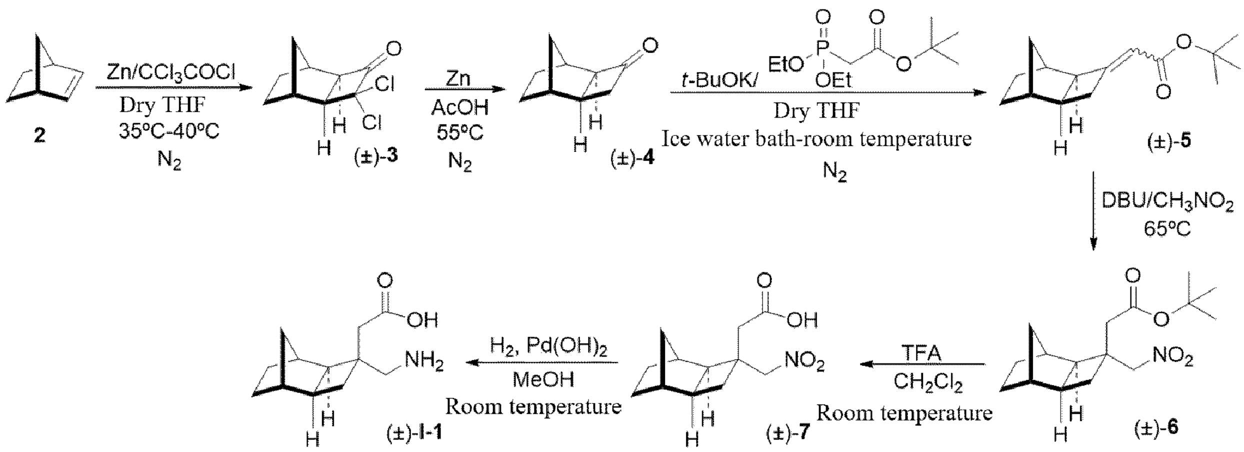


Step 1: Synthesis of compound (±)-**3**

Steps for preparation of activated zinc powder are as follows: Dry $CuSO_4$ (40.00 g, 0.25 mol) was added into water (1 L) and stirred for dissolved clarification. Zinc powder (600.00 g, 9.17 mol) was added, stirred at room temperature for 3-4 h, and filtered. A filter cake was sequentially washed with water (300 mL×2) and acetone (750 mL×2) and dried in a vacuum drying oven at a temperature of 50°C (about 10 mmHg) for 24-48 h.

Under the conditions of $N_2$ environment and ice water bath, compound **2** (14.00 g, 0.15 mol) and the activated

zinc powder (14.00 g, 0.21 mol) were sequentially added into dry tetrahydrofuran (140 mL) and stirred. A dry tetrahydrofuran (20 mL) solution of trichloroacetyl chloride (12.00 g, 66 mmol) was added thereto dropwise. The system released heat during the dropping, and the internal temperature of the reaction system was maintained at 35°C-40°C by controlling a dropping speed. After the dropping was completed, the internal temperature of the reaction system was maintained at 35°C, and stirring was performed overnight. TLC monitoring showed that the reaction was completed. The reaction solution was cooled to room temperature. Filtration was performed with the assistance of diatomite. A filtrate was concentrated under reduced pressure on a rotary evaporator to obtain a brown oily substance, and then purified by a column chromatography [V(EtOAc)/V(n-hexane)=0/1→9/91] to obtain target product (±)-**3**, a light yellow oily substance, 6.00 g. The product was directly used in a next reaction without characterization.

Step 2: Synthesis of compound (±)-**4**

Zinc powder (12.00 g, 0.18 mol) and glacial acetic acid (65 mL) were mixed and stirred, and a newly formulated glacial acetic acid (12 mL) solution of the compound (±)-**3** (6.00 g, 29 mmol) was added dropwise under an ice water bath. After the dropping was completed, under $N_2$ environment, the reaction mixture was placed in an oil bath and stirred at 55°C for 2 h. TLC monitoring showed that a reaction was completed. The reaction mixture was cooled to room temperature and filtered. A filtrate was poured into ice water (350 mL) and extracted with $CH_2Cl_2$ (200 mL×2). Organic phases were combined, washed with water (300 mL×3), dried ($MgSO_4$), and filtered to remove the drying agent. The filtrate was concentrated under reduced pressure on a rotary evaporator to obtain a brown oily substance, and then purified by a column chromatography [V(EtOAc)/V(n-hexane)=0/1→1/9] to obtain target product (±)-**4**, a light yellow oily substance, 3.00 g (a combined yield of **2**→(±)-**4** was 15%).

$^1H$ NMR ($CDCl_3$, 500 MHz) δ: 3.08-3.10 (m, 1H), 3.03 (ddd, 1H, *J* = 3.8 Hz, 8.8 Hz and 18.5 Hz), 2.53 (dt, 1H, *J* = 3.5 Hz and 18.5 Hz), 2.43-2.45 (m, 1H), 2.39-2.41 (m, 1H), 2.25-2.28 (m, 1H), 1.58-1.65 (m, 1H), 1.53-1.56 (m, 1H), 1.46-1.53 (m, 1H), 1.24-1.27 (m, 1H), 1.18-1.24 (m, 1H), 1.10-1.13 (m, 1H).

$^{13}C$ NMR ($CDCl_3$, 126 MHz) δ: 213.08, 68.21, 50.44, 39.70, 37.41, 32.62, 31.38, 28.46, 26.87.

Step 3: Synthesis of compound (±)-**5**

Under $N_2$ environment, potassium tert-butoxide (*t*-BuOK) (3.8 g, 34 mmol) was added into dry THF (25 mL) and stirred into a suspension under an ice water bath, and then tert-butyl diethylphosphonoacetate (7.00 g, 28 mmol) was added dropwise thereto. After the dropping was completed, a reaction was carried out under the ice water bath for 40 min, and a newly formulated dry THF (10 mL) solution of the compound (±)-**4** (3.00 g, 22 mmol) was added dropwise. After the dropping was completed, a reaction was carried out at room temperature for 2 h, and TLC monitoring showed that the reaction was completed. The reaction solution was poured into water (200 mL) and extracted with EtOAc (120 mL×2). Organic phases were combined, dried ($MgSO_4$), filtered to remove the drying agent, concentrated under reduced pressure on a rotary evaporator to obtain a deep yellow oily substance, and then purified by a column chromatography [V(EtOAc)/V(n-hexane)=0/1→1/9] to obtain target product (±)-**5**, a light yellow oily substance, 3.70 g. The product was directly used in a next reaction without characterization.

Step 4: Synthesis of compound (±)-**6**

At room temperature, (±)-**5** (3.70 g, 16 mmol) was dissolved in $CH_3NO_2$ (22 mL) and stirred, and 1,8-diazabicycloundec-7-ene (DBU) (4.80 g, 32 mmol) was added dropwise thereto. After the dropping was completed, under $N_2$ environment, the reaction mixture was stirred overnight in an oil bath at 65°C. TLC monitoring showed that a reaction was completed. The reaction solution was cooled to room temperature, poured into water (200 mL), and extracted with $CH_2Cl_2$ (150 mL×2). Organic phases were combined, dried ($MgSO_4$), and filtered to remove the drying agent. A filtrate was concentrated under reduced pressure on a rotary evaporator to obtain a brownish black oily substance, and then purified by a column chromatography [V(EtOAc)/V(n-hexane)=0/1→1/9] to obtain target product (±)-**6**, a light yellow oily substance, 3.80 g (a combined yield of (±)-**4**→(±)-**6** was 58%).

$^1H$ NMR ($CDCl_3$, 500 MHz) δ: 4.74 (dd, 1H, *J* = 1.0 Hz and 11.5 Hz), 4.68 (d, 1H, *J* = 11.5 Hz), 2.57 (d, 1H, *J= 17.0* Hz), 2.52 (d, 1H, *J* = 17.0 Hz), 2.31-2.35 (m, 1H), 2.12-2.13 (m, 1H), 2.03-2.05 (m, 2H), 1.99 (ddd, 1H, *J* = 2.0 Hz, 9.0 Hz and 14.0 Hz), 1.87-1.90 (m, 1H), 1.57-1.61 (m, 1H), 1.45-1.50 (m, 11H), 1.29-1.32 (m, 1H), 1.02-1.06 (m, 2H).

$^{13}C$ NMR ($CDCl_3$, 126 MHz) δ: 170.83, 82.52, 81.10, 48.40, 39.78, 37.97, 37.43, 36.35, 35.03, 34.25, 32.03, 28.88, 28.23, 27.52.

ESI-HRMS: *(m/z)* calcd. for $C_{16}H_{26}NO_4$ ($[M+H]^+$) 296.1856, found: 296.1852.

Step 5: Synthesis of compound (±)-**7**

The compound (±)-**6** (1.70 g, 5.8 mmol) was dissolved in $CH_2Cl_2$ (18 mL), trifluoroacetic acid (TFA) (10 mL) was added dropwise thereto under an ice water bath, and after the dropping was completed, stirring was performed at room temperature until TLC monitoring showed that a reaction was completed (usually 2-4 h). The reaction solution was concentrated under reduced pressure on a rotary evaporator to obtain a brown oily substance, and then purified by a column chromatography [V(EtOAc)/V(n-hexane)=0/1→17/33] to obtain a light yellow oily substance. N-hexane (1 mL) was added into the oily substance, ultrasonic pulverization was performed to precipitate a solid, filtration was performed after stirring at room temperature for 1 h, and the solid was collected and dried to obtain target product (±)-**7**, a white solid (72%), 1.00 g, with a melting point of 93.7°C-95.5°C.

$^1H$ NMR ($CDCl_3$, 500 MHz) δ: 4.76 (d, 1H, *J* = 12.0 Hz), 4.71 (d, 1H, *J* = 12.0 Hz), 2.77 (d, 1H, *J* = 18.0 Hz), 2.72 (d, 1H, *J* = 18.0 Hz), 2.34-2.38 (m, 1H), 2.15-2.16 (m, 1H), 2.03-2.07 (m, 2H), 1.99 (ddd, 1H, *J* = 2.0 Hz, 8.5 Hz and 14.0 Hz), 1.86-1.88 (m, 1H), 1.62 (dd, 1H, *J* = 6.0 Hz and 14.0 Hz), 1.48-1.52 (m, 2H), 1.32-1.34 (m,1H), 1.02-1.08 (m, 2H).

$^{13}C$ NMR (DMSO-$d_6$, 126 MHz) δ: 172.30, 82.18, 47.56, 38.90, 37.26, 36.77, 35.60, 33.76, 33.59, 31.27, 28.38, 27.06.

ESI-HRMS: *(m/z)* calcd. for $C_{12}H_{18}NO_4$ ($[M+H]^+$) 240.1230, found: 240.1227.

Step 6: Synthesis of compound (±)-**I-1**

(±)-**7** (1.00 g, 4.2 mmol) was dissolved in $CH_3OH$ (10 mL), 10% $Pd(OH)_2$/C (0.27 g) was added thereto, air in a reaction vessel was replaced with hydrogen (balloon) according to standard operations, and stirring was performed overnight at room temperature. TLC monitoring showed that a reaction was completed (the reaction was generally completed within 12 h). The reaction mixture was filtered with the assistance of diatomite, a filtrate was concentrated under reduced pressure on a rotary evaporator to obtain an oily residue, EtOAc (10 mL) was added thereto and stirred to precipitate a solid, and stirring was performed at room temperature for 1 h. The solid was collected by filtration and dried to obtain target product (±)-**I-1,** a white solid (34%), 0.30 g, with a melting point of 180.2°C-184.0°C.

$^1H$ NMR ($CD_3OD$, 500 MHz) δ: 3.04 (s, 2H), 2.63 (d, 1H, *J* = 16.0 Hz), 2.45 (d, 1H, *J* = 16.0 Hz), 2.29-2.34 (m, 1H), 2.21-2.22 (m, 2H), 2.01-2.06 (m, 2H), 1.86-1.87 (m, 1H), 1.79 (ddd, 1H, *J* = 2.0 Hz, 8.5 Hz and 13.0 Hz), 1.49-1.55 (m, 3H), 1.30-1.32 (m, 1H), 1.03-1.07 (m, 2H).

$^{13}C$ NMR ($CD_3OD$ + $D_2O$ (1 drop), 126 MHz) δ: 180.68, 51.20, 49.46, 42.56, 39.00, 38.90, 37.88, 37.29, 34.69, 32.76, 29.48, 28.39.

ESI-HRMS: *(m/z)* calcd. for $C_{12}H_{20}NO_2$ ($[M+H]^+$) 210.1489, found: 210.1483.

The compound (±)-**I-1** is a specific form of the compound of general formula **I** in the present application.

## Example 2 Synthesis of compound (±)-I-2

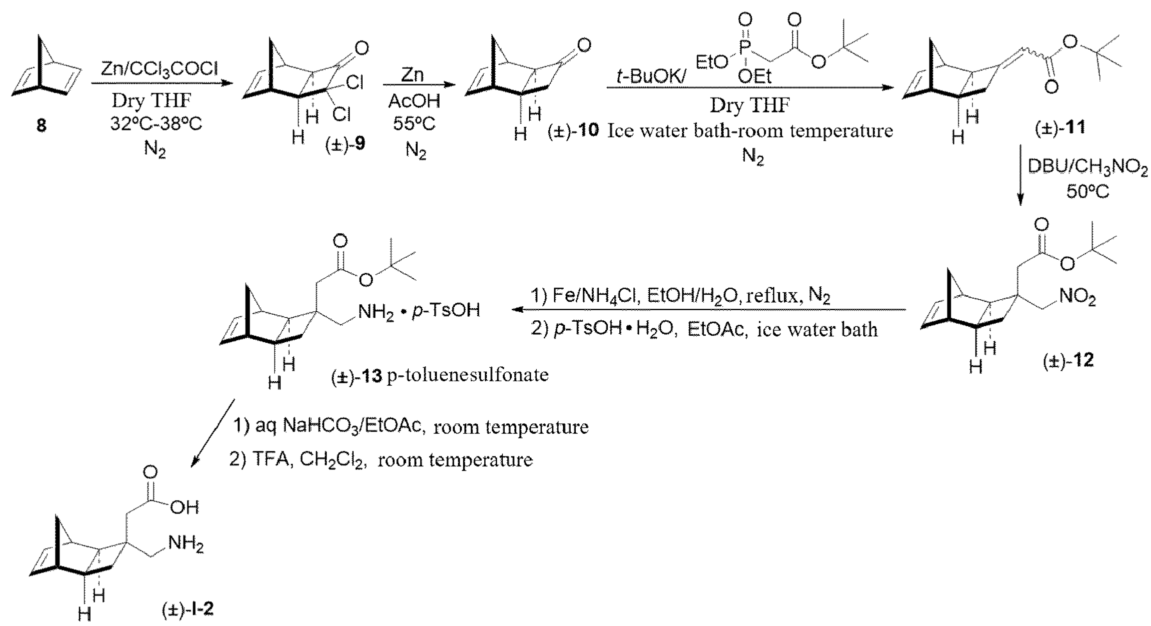

Step 1: Synthesis of compound (±)-**9**

Under the conditions of $N_2$ environment and ice water bath, compound **8** (42.00 g, 0.46 mol) and activated zinc powder (42.00 g, 0.64 mol) were sequentially added into dry tetrahydrofuran (350 mL) and stirred. A dry tetrahydrofuran (150 mL) solution of trichloroacetyl chloride (36.00 g, 0.20 mol) was added dropwise thereto. The reaction system released heat during the dropping, and the internal temperature of the reaction system was maintained at 32°C-38°C. After the dropping was completed, the internal temperature of the reaction system was maintained at 34.5°C, and stirring was performed overnight. TLC monitoring showed that a reaction was completed. The reaction solution was cooled to room temperature. Filtration was performed with the assistance of diatomite. A filtrate was concentrated under reduced pressure on a rotary evaporator to obtain a residue, and then purified by a column chromatography [V(EtOAc)/V(n-hexane)=0/1 →2/23] to obtain target product (±)-**9**, a light yellow oily substance, 10.54 g. The product was directly used in a next reaction without characterization.

Step 2: Synthesis of compound (±)-**10**

After zinc powder (21.00 g, 0.32 mol) and glacial acetic acid (120 mL) were stirred and mixed, a newly formulated glacial acetic acid (25 mL) solution of the compound (±)-**9** (10.54 g, 52 mmol) was added dropwise thereto under an ice water bath. After the dropping was completed, under $N_2$ environment, the reaction mixture was placed in an oil bath at 55°C and stirred overnight. TLC monitoring showed that a reaction was completed. The reaction mixture was cooled to room temperature and filtered. A filtrate was diluted with water (400 mL) and extracted with $CH_2Cl_2$ (300 mL). An organic phase was sequentially washed with water (400 mL×3) and a saturated $NaHCO_3$ solution (400 mL) until a pH of an aqueous phase was > 7, dried ($MgSO_4$), and filtered to remove the drying agent. The filtrate was concentrated under reduced pressure on a rotary evaporator to obtain a brown oily substance, and then purified by a column chromatography [V(EtOAc)/V(n-hexane)=0/1-2/23] to obtain target product (±)-**10**, a light yellow oily substance, 2.73 g (a combined yield of **8**→(±)-**10** was 4%).

$^1H$ NMR ($CDCl_3$, 500 MHz) δ: 6.29-6.31 (m, 1H), 6.12-6.14 (m, 1H), 3.05-3.08 (m, 2H), 3.00-3.01 (m, 1H), 2.84 (ddd, 1H, *J* = 3.3 Hz, 9.0 Hz and 19.3 Hz), 2.28-2.33 (m, 2H), 1.54-1.56 (m, 1H), 1.40-1.43 (m, 1H). $^{13}C$ NMR ($CDCl_3$, 126 MHz) δ: 211.83, 139.86, 136.19, 66.31, 45.70, 44.17, 43.18, 41.06, 30.28. ESI-HRMS: *(m/z)* calcd. for $C_9H_{11}O$ ($[M+H]^+$) 135.0804, found: 135.0803.

Step 3: Synthesis of compound (±)-**11**

Under $N_2$ environment, *t*-BuOK (4.52 g, 40 mmol) was added into dry THF (90 mL) and stirred into a suspension under an ice water bath, and then tert-butyl diethylphosphonoacetate (10.16 g, 40 mmol) was added dropwise thereto. After the dropping was completed, a reaction was carried out under the ice water bath for 1 h, and a newly formulated dry THF (30 mL) solution of the compound (±)-**10** (2.70 g, 20 mmol) was added dropwise thereto. After the dropping was completed, stirring was performed overnight at room temperature. TLC monitoring showed that the reaction was completed. The reaction solution was poured into water (400 mL) and extracted with EtOAc (300 mL×3). Organic phases were combined, washed with a saturated brine (300 mL), dried ($MgSO_4$), and filtered to remove the drying agent. A filtrate was concentrated under reduced pressure on a rotary evaporator to obtain a deep yellow oily substance, and then purified by a column chromatography [V(EtOAc)/V(n-hexane)=0/1→2/23] to obtain target product (±)-**11**, a light yellow oily substance, 3.91 g. The product was directly used in a next reaction without characterization.

Step 4: Synthesis of compound (±)-**12**

At room temperature, (±)-**11** (3.91 g, 17 mmol) was dissolved in $CH_3NO_2$ (40 mL) and stirred, and DBU (7.76 g, 51 mmol) was added dropwise thereto. After the dropping was completed, under $N_2$ environment, the reaction mixture was stirred overnight in an oil bath at 50°C. TLC monitoring showed that a reaction was completed. The reaction solution was cooled to room temperature, poured into water (250 mL), and extracted with $CH_2Cl_2$ (200 mL×2). Organic phases were combined, dried ($MgSO_4$), and filtered. A filtrate was concentrated under reduced pressure on a rotary evaporator to obtain a brownish black oily substance, and then purified by a column chromatography [V(EtOAc)/V(n-hexane)=0/1-2/23] to obtain target product (±)-**12,** a light yellow oily substance, 3.06 g (a combined yield of (±)-**10**→(±)-**12** was 52%).

$^1H$ NMR ($CDCl_3$, 500 MHz) δ: 6.02-6.04 (m, 1H), 5.99-5.01 (m, 1H), 4.74 (d, 1H, *J* = 11.5 Hz), 4.70 (d, 1H, *J* = 11.5 Hz), 2.72-2.74 (m, 2H), 2.55 (s, 2H), 2.09-2.14 (m, 1H), 2.04 (ddd, 1H, *J* = 2.0 Hz, 8.5 Hz and 13.5Hz), 1.87-1.88 (m, 1H), 1.71-1.73 (m, 1H), 1.36-1.48 (m, 11H).

$^{13}C$ NMR ($CDCl_3$, 126 MHz) δ: 170.87, 136.29, 136.18, 82.78, 81.22, 44.44, 43.34, 41.94, 41.90, 36.16, 34.70, 32.56, 30.46, 28.24.

ESI-HRMS: (*m*/*z*) calcd. for $C_{16}H_{24}NO_4$ ($[M+H]^+$) 294.1700, found: 294.1693.

Step 5: Synthesis of compound (±)**-13** p-toluenesulfonate

At room temperature, the compound (±)**-12** (0.70 g, 2.4 mmol) was dissolved in EtOH (10 mL), added into water (5 mL) and stirred, then iron powder (0.67 g, 12 mmol) and $NH_4Cl$ (0.26 g, 4.9 mmol) were sequentially added thereto, air in a reaction vessel was replaced with nitrogen (balloon) according to standard operations, and stirring was performed in an oil bath at 85°C for 6-7 h. TLC monitoring showed that a reaction was completed. The reaction solution was cooled to room temperature and filtered. A filtrate was washed with a saturated $NaHCO_3$ solution (100 mL) and extracted with EtOAc (30 mL×3). Organic phases were combined, dried ($MgSO_4$), and filtered to remove the drying agent. A filtrate was concentrated under reduced pressure on a rotary evaporator to obtain a brown oily residue. At room temperature, EtOAc (8 mL) was added to dilute the residue, then *p*-TsOH·$H_2O$ (0.49 g, 2.6 mmol) was added and stirred for dissolution, and the system was transferred into an ice water bath, stirred to precipitate a white solid, and continuously stirred under the ice water bath for 1 h. A filter cake was collected by filtration and dried with a vacuum oil pump to obtain p-toluenesulfonate of (±)**-13,** a white solid , 0.66 g (64%).

$^1$H NMR (DMSO-$d_6$, 500 MHz) δ: 7.75 (brs, 3H), 7.48 (d, 2H, *J* = 8.0 Hz), 7.11 (d, 2H, *J* = 8.0 Hz), 6.03-6.05 (m, 1H), 6.00-6.02 (m, 1H), 3.04-3.08 (m, 2H), 2.65-2.67 (m, 2H), 2.43-2.44 (m, 2H), 2.29 (s, 3H), 1.98-2.02 (m, 1H), 1.82 (ddd, 1H, *J* = 1.8 Hz, 8.5 Hz and 13.3 Hz), 1.41 (s, 9H), 1.22-1.27 (m, 2H).

$^{13}$C NMR (DMSO-$d_6$, 126 MHz) δ: 170.40, 145.70, 137.63, 135.92, 135.89, 128.06, 125.50, 80.33, 47.12, 43.51, 42.79, 41.55, 41.32, 34.16, 33.97, 31.69, 29.31, 27.78, 20.79.

ESI-HRMS: *(m/z)* calcd. for $C_{16}H_{26}NO_2$ ([M(free base)+H]$^+$) 264.1958, found: 264.1953.

Step 6: Synthesis of compound (±)-**I-2**

At room temperature, the (±)-**13** p-toluenesulfonate (0.66 g, 1.5 mmol) and a saturated $NaHCO_3$ solution (100 mL) were stirred for 20 min (suspension state), and EtOAc (30 mL×3) was added for extraction. Organic phases were combined, dried ($MgSO_4$), and filtered to remove the drying agent. A filtrate was concentrated under reduced pressure on a rotary evaporator to obtain a brown oily substance, and the oily substance was dissolved in $CH_2Cl_2$ (5 mL). Under an ice water bath, TFA (2.5 mL) was slowly added dropwise. After the dropping was completed, a reaction was carried out at room temperature for 2 h. TLC monitoring showed that the reaction was completed. The reaction solution was concentrated under reduced pressure on a rotary evaporator to obtain a yellow oily substance. The oily substance was dried with a vacuum oil pump and then solidified, methyl tert-butyl ether (5 mL) was added for trituration and slurrying at room temperature for 30 min, and a solid was collected by filtration and dried with a vacuum oil pump to obtain compound (±)-**I-2**, a white solid (64%), 0.20 g, with a melting point of 141.8°C-146.6°C.

$^1$H NMR ($CD_3OD$, 500 MHz) δ: 6.02-6.05 (m, 2H), 3.24 (d, 1H, *J* = 13.0 Hz), 3.21 (d, 1H, *J* = 13.0 Hz), 2.76-2.77 (m, 1H), 2.70-2.71 (m, 1H), 2.64 (d, 1H, *J* = 17.0 Hz), 2.55 (d, 1H, *J* = 16.5 Hz), 2.09-2.14 (m, 1H), 1.89 (ddd, 1H, *J* = 2.0 Hz, 8.5 Hz and 13.5 Hz), 1.78-1.80 (m, 1H), 1.73-1.75 (m, 1H), 1.41 (dd, 1H, *J* = 5.5 Hz and 13.5 Hz), 1.35-1.38 (m, 1H).

$^{13}$C NMR ($CD_3OD$, 126 MHz) δ: 175.47, 137.19, 137.05, 49.86, 45.37, 44.46, 42.88, 42.60, 35.34, 35.19, 33.43, 30.99.

ESI-HRMS: *(m/z)* calcd. for $C_{12}H_{18}NO_2$ ([M+H]$^+$) 208.1332, found: 208.1327.

The compound (±)**-I-2** is a specific form of the compound of general formula **I** in the present application.

## Example 3 Synthesis of compound (+)-1-3

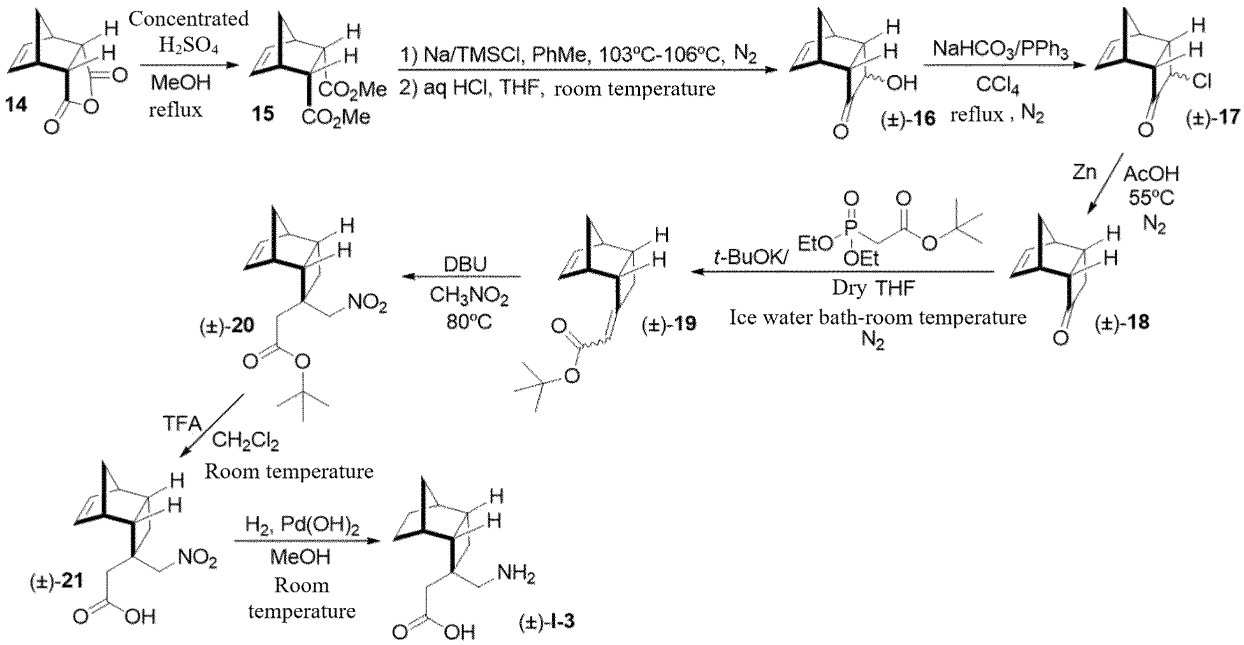


Step 1: Synthesis of compound **15**

Compound **14** (30.00 g, 0.18 mol) was dissolved in dry $CH_3OH$ (300 mL), and then concentrated $H_2SO_4$ (3 mL) was added thereto and heated to reflux for 24 h. TLC monitoring showed that a reaction was completed. The reaction solution was cooled to room temperature, directly concentrated to 1/3 of the original volume under reduced pressure on a rotary evaporator, and then poured into ice water (400 mL). A resulting mixture was extracted with $CH_2Cl_2$ (300 mL×2). Organic phases were combined, washed with a saturated $NaHCO_3$ solution (400 mL), dried ($MgSO_4$), filtered to remove the drying agent, and concentrated under reduced pressure on a rotary evaporator to obtain target compound **15,** a yellow oily substance, 37.00 g (96%). $^1H$ NMR ($CDCl_3$, 500 MHz) δ: 6.26-6.27 (m, 2H), 3.61 (s, 6H), 3.29-3.30 (m, 2H), 3.16-3.17 (m, 2H), 1.47-1.49 (m, 2H), 1.32-1.35 (m, 2H).

Step 2: Synthesis of compound (±)**-16**

Metallic sodium (19.00 g, 0.83 mol) was added into dry toluene (370 mL) and heated under $N_2$ environment until the sodium was completely melted, and stirring was performed at an internal temperature maintained at 103°C-106°C for 20 min. A dry toluene (100 mL) solution of the compound **15** (37.00 g, 0.18 mol) and trimethylchlorosilane (TMSCl) (85.00 g, 0.78 mol) was added dropwise under stirring. The system released heat during the dropping, and the internal temperature of the reaction system was maintained at 103°C-106°C. After the dropping was completed, the internal temperature of the reaction system was maintained at 103°C-106°C, and stirring was performed overnight. TLC monitoring showed that a reaction was completed. The reaction solution was cooled to room temperature. Filtration was performed with the assistance of diatomite. A filtrate was concentrated under reduced pressure on a rotary evaporator to obtain a brown oily substance. The oily substance was dissolved in THF (200 mL), and 1 M HCl (20 mL) was added dropwise under an ice water bath. After the dropping was completed, a reaction was carried out at room temperature for 0.5 h. TLC monitoring showed that the reaction was completed. The reaction solution was poured into water (400 mL) and extracted with EtOAc (300 mL×2). Organic phases were combined, dried ($MgSO_4$), and filtered to remove the drying agent. A filtrate was concentrated under reduced pressure on a rotary evaporator to obtain a brown oily substance, and then purified by a column chromatography [V(EtOAc)/V(n-hexane)=0/1→2/3] to obtain target product (±)**-16**, a white solid, 14.3 g (54%), with a melting point of 74.7°C-77.5°C. The product was directly used in a next reaction without characterization.

Step 3: Synthesis of compound (±)**-17**

The compound (±)**-16** (5.00 g, 33 mmol) was dissolved in $CCl_4$ (60 mL), and triphenylphosphine (10.00 g, 38 mmol) and $NaHCO_3$ (0.40 g, 4.8 mmol) were sequentially added thereto under a stirring state. Air in a reaction vessel was replaced with nitrogen (balloon) according to standard operations, and stirring was performed overnight in an oil bath at 75°C. TLC monitoring showed that a reaction was completed. The reaction solution was cooled to room temperature and

filtered. A filtrate was concentrated under reduced pressure on a rotary evaporator to obtain a brownish black oily substance, and then purified by a column chromatography [V(EtOAc)/V(n-hexane)=0/1→9/91] to obtain target product (±)**-17**, a light yellow oily substance, 4.40 g. The product was directly used in a next reaction without characterization.

Step 4: Synthesis of compound (±)**-18**

After zinc powder (8.00 g, 0.12 mol) and glacial acetic acid (40 mL) were stirred and mixed, a newly formulated glacial acetic acid (6 mL) solution of the compound (±)**-17** (4.40 g, 26 mmol) was added dropwise at room temperature. After the dropping was completed, under $N_2$ environment, the reaction mixture was stirred in an oil bath at 55°C for 1.5 h. TLC monitoring showed that a reaction was completed. The reaction mixture was cooled to room temperature and filtered. A filtrate was diluted with ice water (150 mL) and extracted with $CH_2Cl_2$ (70 mL×2). Organic phases were combined, washed with water (100 mL×3), dried ($MgSO_4$), and filtered to remove the drying agent. The filtrate was concentrated under reduced pressure on a rotary evaporator to obtain a yellow oily substance, and then purified by a column chromatography [V(EtOAc)/V(n-hexane)=0/1→1/9] to obtain target product (±)**-18**, a light yellow semi-solid, 1.30 g (a combined yield of (±)**-16**→(±)**-18** was 29%).

$^1$H NMR ($CDCl_3$, 500 MHz) δ: 6.16-6.17 (m, 2H), 3.71-3.75 (m, 1H), 3.12-3.14 (m, 1H), 3.04-3.06 (m, 1H), 2.79-2.84 (m, 1H), 2.72 (dddd, 1H, *J* = 1.0 Hz, 3.0 Hz, 8.5 Hz and 18.0 Hz), 2.15 (dt, 1H, *J* = 3.8 Hz and 18.5 Hz), 1.75-1.77 (m, 1H), 1.45-1.47 (m, 1H).

$^{13}$C NMR ($CDCl_3$, 126 MHz) δ: 211.48, 135.81, 132.74, 66.65, 54.50, 46.42, 46.21, 44.13, 26.93.

Step 5: Synthesis of compound (±)**-19**

Under $N_2$ environment, *t*-BuOK (1.90 g, 17 mmol) was added into dry THF (15 mL) and stirred into a suspension under an ice water bath, and then tert-butyl diethylphosphonoacetate (3.50 g, 14 mmol) was added dropwise. After the dropping was completed, a reaction was carried out under the ice water bath for 40 min, and a newly formulated dry THF (5 mL) solution of the compound (±)**-18** (1.40 g, 10 mmol) was added dropwise. After the dropping was completed, stirring was performed at room temperature for 1.5 h. TLC monitoring showed that a reaction was completed. The reaction solution was poured into water (100 mL) and extracted with EtOAc (70 mL×2). Organic phases were combined, dried ($MgSO_4$), and filtered. A filtrate was concentrated under reduced pressure on a rotary evaporator to obtain a deep yellow oily substance, and then purified by a column chromatography [V(EtOAc)/V(n-hexane)=0/1→2/23] to obtain target product (±)**-19**, a light yellow oily substance, 2.42 g. The product was directly used in a next reaction without characterization.

Step 6: Synthesis of compound (±)**-20**

At room temperature, the above crude product (±)**-19** (2.42 g, calculated as 10 mmol) was dissolved in $CH_3NO_2$ (15 mL) and stirred, and DBU (3.00 g, 20 mmol) was added dropwise thereto. After the dropping was completed, under $N_2$ environment, the reaction mixture was stirred overnight in an oil bath at 80°C. TLC monitoring showed that the raw materials were not reacted completely. The reaction solution was cooled to room temperature, poured into water (150 mL), and extracted with $CH_2Cl_2$ (70 mL×2). Organic phases were combined, dried ($MgSO_4$), and filtered. A filtrate was concentrated under reduced pressure on a rotary evaporator to obtain a brownish black oily substance, and then purified by a column chromatography [V(EtOAc)/V(n-hexane)=0/1→1/9] to obtain target product (±)**-20,** a light yellow oily substance, 1.00 g (a combined yield of (±)**-18**→(±)**-20** was 33%).

$^1$H NMR ($CDCl_3$, 500 MHz) δ: 6.40-6.41 (m, 1H), 6.28-6.29 (m, 1H), 4.82 (dd, 1H, *J* = 1.0 Hz and 11.5 Hz), 4.60 (dd, 1H, *J* = 1.0 Hz and 11.5 Hz), 2.94-2.96 (m, 1H), 2.81-2.87 (m, 2H), 2.52-2.56 (m, 1H), 2.47 (dd, 1H, *J* = 0.8 Hz and 17.8 H), 2.38 (d, 1H, *J* = 17.5 Hz), 2.04 (ddd, 1H, *J* = 1.5 Hz, 8.0 Hz and 13.0 Hz), 1.56-1.60 (m, 1H), 1.47 (s, 9H), 1.32-1.36 (m, 1H), 1.11-1.13 (m, 1H).

$^{13}$C NMR ($CDCl_3$, 126 MHz) δ: 170.92, 137.50, 136.91, 83.36, 80.92, 53.22, 47.71, 45.82, 44.83, 38.78, 36.17, 34.10, 32.83, 28.24.

ESI-HRMS: *(m/z)* calcd. for $C_{16}H_{24}NO_4$ ($[M+H]^+$) 294.1700, found: 294.1777.

Step 7: Synthesis of compound (±)**-21**

The compound (±)**-20** (1.00 g, 3.4 mmol) was dissolved in $CH_2Cl_2$ (10 mL), and TFA (6 mL) was slowly added dropwise thereto under an ice water bath. After the dropping was completed, a reaction was carried out at room temperature for 2 h. TLC monitoring showed that the reaction was completed. The reaction solution was concentrated under reduced pressure on a rotary evaporator to obtain a yellow oily substance, and then purified by a column chromatography [V(EtOAc)/V(n-hexane)=0/1→2/3] to obtain a light yellow oily substance. n-hexane (1 mL) was added into the oily substance, ultrasonic treatment was performed to solidify the oily substance, which was then slurried at room

temperature for 30 min. A solid was collected by filtration, and a filter cake was dried with a vacuum oil pump to obtain compound (±)-**21**, a white solid, 0.80 g (99%), with a melting point of 84.6°C-87.7°C.

$^1$H NMR ($CDCl_3$, 500 MHz) δ: 6.43-6.44 (m, 1H), 6.31-6.32 (m, 1H), 4.79 (dd, 1H, *J* = 1.0 Hz and 11.5 Hz), 4.65 (d, 1H, *J* = 11.5 Hz), 2.97-2.99 (m, 1H), 2.86-2.89 (m, 2H), 2.67 (d, 1H, *J* = 18.5 Hz), 2.57 (d, 1H, *J* = 18.5 Hz), 2.54-5.56 (m, 1H), 2.03-2.07 (m, 1H), 1.61-1.63 (m, 1H), 1.38 (dd, 1H, *J* = 6.0 Hz and 13.5 Hz), 1.13-1.15 (m, 1H).

$^{13}$C NMR ($CDCl_3$, 126 MHz) δ: 177.12, 137.88, 136.78, 83.09, 53.34, 47.56, 45.68, 44.84, 38.34, 34.58, 34.17, 32.70.

ESI-HRMS: *(m/z)* calcd. for $C_{12}H_{16}NO_4$ ([M+H]$^+$) 238.1074, found: 238.1071.

Step 8: Synthesis of compound (±)-**I-3**

The compound (±)-**21** (0.23 g, 0.97 mmol) was dissolved in $CH_3OH$ (9 mL), 10% $Pd(OH)_2/C$ (0.15 g) was added thereto, air in a reaction vessel was replaced with hydrogen (balloon) according to standard operations, and stirring was performed overnight at room temperature. TLC monitoring showed that a reaction was completed (the reaction was generally completed within 12 h). Filtration was performed. A filtrate was concentrated under reduced pressure on a rotary evaporator to obtain a white solid, and $CH_3OH$ (1 mL)/EtOAc (2 mL) was added, and stirred and slurried at room temperature for 5 min. The solid was collected by filtration and dried to obtain target product (+)-**I-3**, a white solid, 0.12 g (60%), with a melting point of 186.2°C-190.5°C.

$^1$H NMR ($CD_3OD$, 500 MHz) δ: 3.07 (d, 1H, *J* = 12.5 Hz), 2.98 (d, 1H, *J* = 12.5 Hz), 2.71 (d, 1H, *J* = 16.0 Hz), 2.65 (d, 1H, *J* = 16.0 Hz), 2.50-2.55 (m, 1H), 2.44-2.46 (m, 1H), 2.22-2.26 (m, 2H), 1.99-2.05 (m, 1H), 1.91 (dd, 1H, *J* = 7.5 Hz and 13.0 Hz), 1.75-1.81 (m, 2H), 1.54-1.61 (m, 1H), 1.46-1.53 (m, 1H), 1.40-1.43 (m, 1H), 1.24-1.27 (m, 1H).

$^{13}$C NMR ($CD_3OD$, 126 MHz) δ: 180.05, 52.82, 49.93, 47.58, 42.78, 41.02, 40.22, 37.39, 34.81, 33.73, 26.23, 25.26.

ESI-HRMS: *(m/z)* calcd. for $C_{12}H_{20}NO_2$ ([M+H]$^+$) 210.1489, found: 210.1484.

The compound (±)-**I-3** is a specific form of the compound of general formula **I** in the present application.

**Example 4 Synthesis of compound (±)-I-4 and a p-toluenesulfonate thereof**

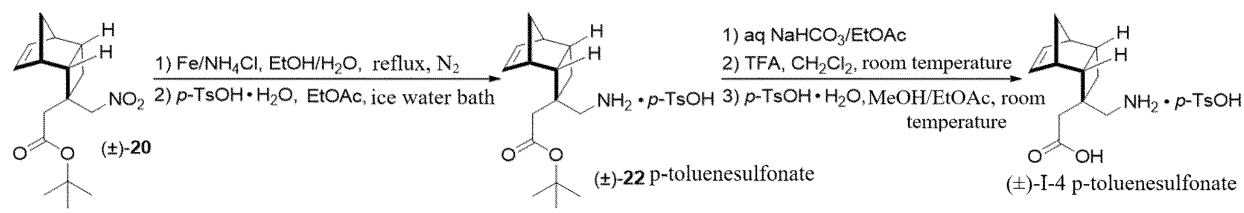


(±)-**20**  (±)-**22** p-toluenesulfonate  (±)-I-4 p-toluenesulfonate

Step 1: Synthesis of compound (±)-**22** p-toluenesultonate

At room temperature, the compound (±)-**20** (1.50 g, 5.1 mmol) was dissolved in EtOH (15 mL), water (7 mL) was added thereto and stirred, then iron powder (1.50 g, 27 mmol) and $NH_4Cl$ (0.50 g, 9.3 mmol) were sequentially added thereto, air in a reaction vessel was replaced with nitrogen (balloon) according to standard operations, and stirring was performed in an oil bath at 85°C for 4 h. TLC monitoring showed that a reaction was completed. The reaction solution was cooled to room temperature and filtered. A filtrate was added into a saturated $NaHCO_3$ solution (100 mL) and extracted with EtOAc (40 mL×3). Organic phases were combined, dried ($MgSO_4$), and filtered to remove the drying agent. A filtrate was concentrated under reduced pressure on a rotary evaporator to obtain a yellow oily substance. At room temperature, EtOAc (15 mL) was added to dilute the oily substance, then *p*-TsOH·$H_2O$ (0.97 g, 5.1 mmol) was added and stirred for dissolution until a large amount of solid was precipitated out, and the system was transferred into an ice water bath and continuously stirred and slurried for 1 h. The solid was collected by filtration and dried with a vacuum oil pump to obtain (±)-**22** p-toluenesulfonate, a white solid, 1.60 g (72%), with a melting point of 184.4°C-187.1°C.

$^1$H NMR (DMSO-$d_6$, 500 MHz) δ: 7.71 (brs, 3H), 7.48 (d, 2H, *J* = 8.0 Hz), 7.12 (d, 2H, *J* = 8.0 Hz), 6.35-6.37 (m, 1H), 6.28-6.30 (m, 1H), 3.05-3.11 (m, 1H), 2.94-3.00 (m, 1H), 2.79-2.82 (m, 2H), 2.71-2.77 (m, 1H), 2.40-2.43 (m, 1H), 2.26-2.33 (m, 5H), 1.79 (ddd, 1H, *J* = 1.5 Hz, 8.5 Hz and 13.0 Hz), 1.45-1.48 (m, 1H), 1.43 (s, 9H), 1.11 (dd, 1H, *J* = 6.0 Hz and 13.0 Hz), 1.04-1.06 (m, 1H).

$^{13}$C NMR (DMSO-$d_6$, 126 MHz) δ: 170.51, 145.64, 137.69, 137.19, 136.49, 128.08, 125.50, 80.09, 52.46, 47.71, 46.62, 45.25, 44.08, 36.54, 36.07, 33.20, 32.20, 27.74, 20.79.

ESI-HRMS: *(m/z)* calcd. for $C_{16}H_{26}NO_2$ ([M(free base)+H]$^+$) 264.1958, found: 264.1954.

Step 2: Synthesis of compound (±)**-I-4** and a p-toluenesulfonate thereof

At room temperature, (±)**-22** p-toluenesulfonate (1.60 g, 3.7 mmol) and a saturated $NaHCO_3$ solution (100 mL) were stirred for 20 min (suspension state), and EtOAc (60 mL×3) was added for extraction. Organic phases were combined, dried ($MgSO_4$), and filtered to remove the drying agent. A filtrate was concentrated under reduced pressure on a rotary evaporator to obtain a light yellow oily substance, and the oily substance was dissolved in $CH_2Cl_2$ (10 mL). Under an ice water bath, TFA (7 mL) was slowly added dropwise. After the dropping was completed, a reaction was carried out at room temperature for 4-6 h. TLC monitoring showed that the reaction was completed. The reaction solution was concentrated under reduced pressure on a rotary evaporator to obtain a brown oily substance, and $CH_2Cl_2$ (20 mL) was added for concentration again. The oily substance was dried with a vacuum oil pump until a solid, that is, (±)**-I-4**, was completely precipitated out. The solid was dissolved with $CH_3OH$ (2 mL), and EtOAc (4 mL) was added and stirred. Then *p*-TsOH·$H_2O$ (0.65 g, 3.4 mmol) was added into the reaction solution, stirred for dissolution until a large amount of solid was precipitated out, and continuously stirred and slurried at room temperature for 2 h. The solid was collected by filtration, and a filter cake was dried with a vacuum oil pump to obtain the compound (±)**-I-4** p-toluenesulfonate, a white solid, 0.50 g (36%), with a melting point of 197.0°C-198.7°C. $^1H$ NMR ($CD_3OD$, 500 MHz) δ: 7.70 (d, 2H, $J$ = 8.5 Hz), 7.23 (d, 2H, $J$ = 8.0 Hz), 6.43-6.44 (m, 1H), 6.30-6.31 (m, 1H), 3.25 (d, 1H, $J$ = 13.0 Hz), 3.12 (d, 1H, $J$ = 12.5 Hz), 2.84-2.93 (m, 3H), 2.58 (d, 1H, $J$ = 17.5 Hz), 2.51-2.53 (m, 1H), 2.34-2.38 (m, 4H), 1.75-1.80 (m, 1H), 1.57-1.59 (m, 1H), 1.32-1.35 (m, 1H), 1.14-1.16 (m, 1H).

$^{13}C$ NMR ($CD_3OD$, 126 MHz) δ: 175.74, 143.53, 141.72, 138.51, 137.90, 129.83, 126.97, 53.83, 50.47, 48.15, 46.90, 45.86, 37.93, 37.16, 35.08, 34.08, 21.31.

ESI-HRMS: *(m/z)* calcd. for $C_{12}H_{18}NO_2$ ([M(free base)+H]$^+$) 208.1332, found: 208.1328.

The compound (±)**-I-4** is a specific form of the compound of general formula **I** in the present application.

**Example 5 Synthesis of compound (±)-I-5**

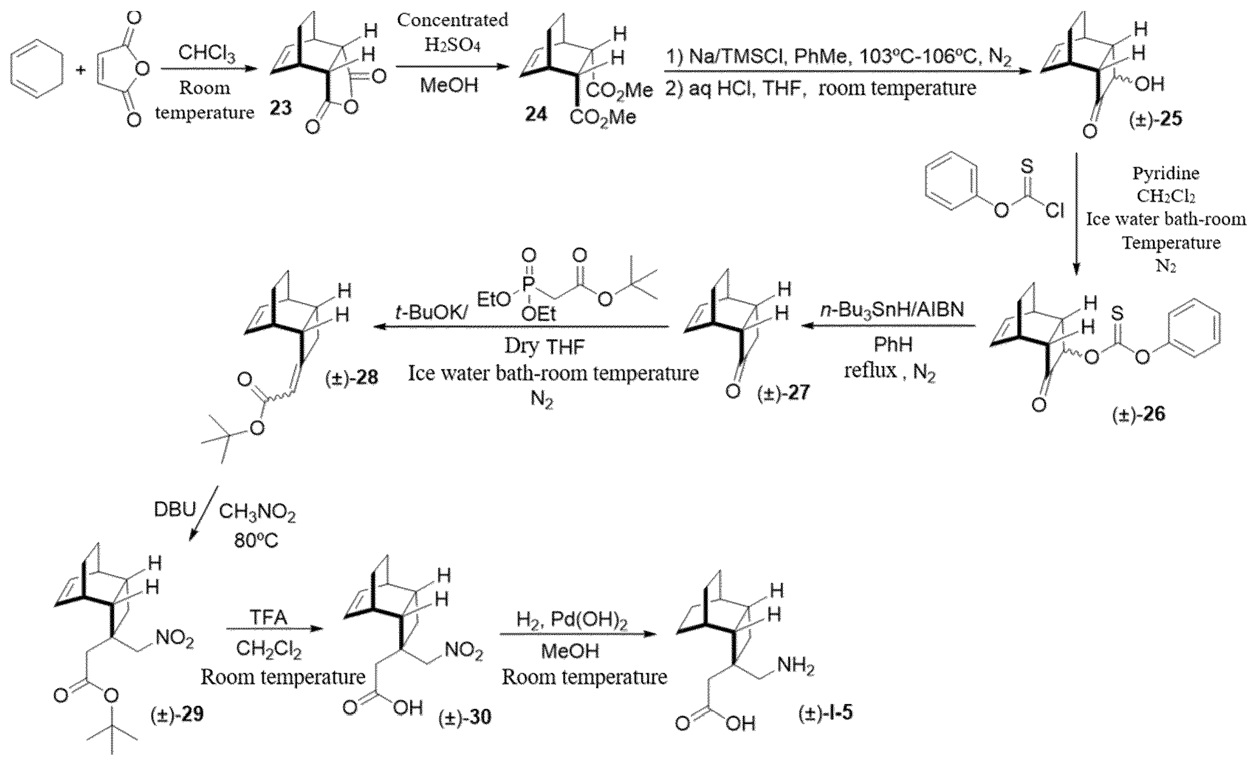


Step 1: Synthesis of compound **23**

Maleic anhydride (68.00 g, 0.69 mol) was dissolved in $CHCl_3$ (400 mL), and a $CHCl_3$ (50 mL) solution of 1,4-cyclohexadiene (78.00 g, 0.97 mol) was added dropwise thereto under an ice water bath. After the dropping was completed, stirring was performed overnight at room temperature. TLC monitoring showed that a reaction was completed. The reaction solution was concentrated under reduced pressure on a rotary evaporator to obtain an oily substance, an

EtOAc/n-hexane mixture (100 mL/400 mL) was added and slurriedat room temperature, and a solid was collected by suction filtration and dried to obtain compound **23,** 54.50 g (44%). $^1$H NMR ($CDCl_3$, 500 MHz) δ: 6.31-6.33 (m, 2H), 3.22-3.24 (m, 2H), 3.127-3.134 (m, 2H), 1.59-1.62 (m, 2H), 1.40-1.43 (m, 2H).

Step 2: Synthesis of compound **24**

The compound **23** (17.00 g, 95 mmol) was dissolved in $CH_3OH$ (170 mL) and stirred, and concentrated sulfuric acid (1.7 mL) was added thereto. Heating was performed to reflux overnight. TLC monitoring showed that a reaction was completed. The reaction solution was concentrated to 1/3 of the original volume under reduced pressure on a rotary evaporator and then poured into ice water (200 mL). A resulting mixture was extracted with $CH_2Cl_2$ (100 mL×3). Organic phases were combined, sequentially washed with a saturated $NaHCO_3$ solution (200 mL) and a saturated brine (200 mL), dried ($MgSO_4$), and filtered to remove the drying agent. A filtrate was concentrated under reduced pressure on a rotary evaporator to obtain solid compound **24,** a white solid, 20.00 g (93%). $^1$H NMR ($CDCl_3$, 500 MHz) δ: 6.31-6.35 (m, 2H), 3.60 (s, 6H), 3.02-3.03 (m, 2H), 2.89-2.91 (m, 2H), 1.53-1.57 (m, 2H), 1.30-1.33 (m, 2H).

Step 3: Synthesis of compound (±)**-25**

Metallic sodium (10.00 g, 0.43 mol) was added into dry toluene (200 mL) and heated under $N_2$ environment until the sodium was completely melted, stirring was initiated, and continuous stirring was performed at an internal temperature maintained at 103°C-106°C for 20 min. A dry toluene (30 mL) solution formulated with the compound **24** (20.00 g, 89 mmol) and TMSCl (48.00 g, 0.44 mol) was added dropwise. The system released heat during the dropping, and the internal temperature of the reaction system was maintained at 103°C-106°C by controlling a dropping speed. After the dropping was completed, the internal temperature of the reaction system was maintained at 103°C-106°C, and stirring was performed overnight. TLC monitoring showed that a reaction was completed. The reaction solution was cooled to room temperature. Filtration was performed with the assistance of diatomite. A filtrate was concentrated under reduced pressure on a rotary evaporator to obtain a brown oily substance. The oily substance was dissolved in THF (100 mL), and 1 M HCl (16 mL) was added dropwise under an ice water bath. After the dropping was completed, a reaction was carried out at room temperature for 3 h. TLC monitoring showed that the reaction was completed. The reaction solution was poured into water (100 mL) and extracted with EtOAc (100 mL×2). Organic phases were combined, dried ($MgSO_4$), and filtered to remove the drying agent. A filtrate was concentrated under reduced pressure on a rotary evaporator to obtain a brown oily substance, and the oily substance was placed at room temperature and then solidified. n-hexane (15 mL) was added into the above solidifying system and heated to reflux, and EtOAc was added dropwise until a solid was completely dissolved. Stirring was performed when being cooled to room temperature, a crystal was precipitated, and continuous stirring was performed for 5 h. Filtration was performed. A filter cake was dried with a vacuum oil pump to obtain target compound (±)**-25**, a white solid 7.60 g (52%), with a melting point of 106.1°C-110.4°C. The product was directly used in a next reaction without characterization.

Step 4: Synthesis of compound (±)**-26**

The compound (±)**-25** (2.00 g, 12 mmol) was dissolved in $CH_2Cl_2$ (20 mL), pyridine (1.93 g, 24 mmol) and 4-dimethylaminopyridine (DMAP) (0.74 g, 6.1 mmol) were sequentially added into the system, and air in a reaction vessel was replaced with nitrogen (balloon) according to standard operations. Under an ice water bath, phenyl chlorothionocarbonate (3.15 g, 18 mmol) was slowly added dropwise into the system. After the dropping was completed, a reaction was carried out at room temperature for 1 h. TLC monitoring showed that the reaction was completed. The reaction solution was poured into ice water (100 mL) and extracted with $CH_2Cl_2$ (50 mL×2). Organic phases were combined, sequentially washed with 1 M HCl (100 mL) and a saturated brine (100 mL), dried ($MgSO_4$), filtered to remove the drying agent, and concentrated under reduced pressure on a rotary evaporator to obtain a crude product of compound (±)**-26**, a yellow solid, 3.66 g. The product was directly used in a next reaction without characterization.

Step 5: Synthesis of compound (±)**-27**

The compound (±)**-26** (3.66 g, 12 mmol) was added into benzene (40 mL), and air in a reaction vessel was replaced with nitrogen (balloon) according to standard operations. Heating was performed to 90°C to reflux, and a benzene (15 mL) solution of *n*-$Bu_3SnH$ (5.30 g, 18 mmol) and azodiisobutyronitrile (AIBN) (0.20 g, 1.2 mmol) was slowly added dropwise. After the dropping was completed, a reaction was carried out in an oil bath maintained at 90°C overnight. TLC monitoring showed that the reaction was completed. The reaction solution was cooled to room temperature, concentrated under reduced pressure on a rotary evaporator to obtain an oily substance, and then purified by a column chromatography [V(EtOAc)/V(n-hexane)=0/1-1/9] to obtain target product (±)**-27**, a light yellow oily substance, 1.20 g (a

combined yield of (±)**-25**→(±)**-27** was 66%).

$^1H$ NMR ($CDCl_3$, 500 MHz) δ: 6.20-6.25 (m, 2H), 3.31-3.34 (m, 1H), 2.89-2.95 (m, 1H), 2.85-2.89 (m, 1H), 2.78-2.81 (m, 1H), 2.40-2.47 (m, 1H), 1.47-1.53 (m, 1H), 1.37-1.44 (m, 2H), 1.23-1.29 (m, 1H).

$^{13}C$ NMR ($CDCl_3$, 126 MHz) δ: 212.49, 133.54, 131.54, 64.18, 50.49, 32.04, 31.01, 26.48, 24.54, 22.54.

Step 6: Synthesis of compound (±)**-28**

Under $N_2$ environment, t-BuOK (0.90 g, 8.0 mmol) was added into dry THF (10 mL) and stirred into a suspension under an ice water bath, and then tert-butyl diethylphosphonoacetate (1.70 g, 6.7 mmol) was added dropwise. After the dropping was completed, a reaction was carried out under the ice water bath for 40 min, and a newly formulated dry THF (5 mL) solution of the compound (±)**-27** (0.60 g, 4.0 mmol) was added dropwise. After the dropping was completed, stirring was performed at room temperature for 1.5 h. TLC monitoring showed that the reaction was completed. The reaction solution was poured into ice water (100 mL) and extracted with EtOAc (50 mL×2). Organic phases were combined, dried ($MgSO_4$), and filtered to remove the drying agent. A filtrate was concentrated under reduced pressure on a rotary evaporator to obtain a deep yellow oily substance, and then purified by a column chromatography [V(EtOAc)/V(n-hexane) =0/1→1/9] to obtain target product (±)**-28**, a light yellow oily substance, 0.90 g. The product was directly used in a next reaction without characterization.

Step 7: Synthesis of compound (±)**-29**

At room temperature, the compound (±)**-28** (0.90 g, 3.7 mmol) was dissolved in $CH_3NO_2$ (9 mL), and DBU (2.00 g, 13 mmol) was added dropwise. After the dropping was completed, under $N_2$ environment, the reaction mixture was stirred overnight in an oil bath at 80°C. TLC monitoring showed that a reaction was basically completed. The reaction solution was cooled to room temperature, poured into water (100 mL), and extracted with $CH_2Cl_2$ (50 mL×2). Organic phases were combined, dried ($MgSO_4$), and filtered to remove the drying agent. A filtrate was concentrated under reduced pressure on a rotary evaporator to obtain a brownish black oily substance, and then purified by a column chromatography [V(EtOAc)/V(n-hexane)=0/1→1/9] to obtain target product (±)**-29**, a light yellow oily substance, 0.50 g (a combined yield of (±)**-27**→(±)**-29** was 40%).

$^1H$ NMR ($CDCl_3$, 500 MHz) δ: 6.45-6.49 (m, 1H), 6.26-6.30 (m, 1H), 4.78 (dd, 1H, *J* = 0.5 Hz and 11.5 Hz), 4.65 (d, 1H, *J* = 11.5 Hz), 2.60 (d, 1H, *J* = 17.5 Hz), 2.52-2.61 (m, 3H), 2.54 (d, 1H, *J* = 17.5 Hz), 2.27-2.30 (m, 1H), 1.94 (ddd, 1H, *J* = 2.0 Hz, 8.5 Hz and 13.0 Hz), 1.64 (dd, 1H, *J* = 7.3 Hz and 13.3 Hz), 1.45 (s, 9H), 1.35-1.40 (m, 2H), 1.25-1.30 (m, 1H), 1.18-1.23 (m, 1H).

$^{13}C$ NMR ($CDCl_3$, 126 MHz) δ: 171.17, 134.53, 134.32, 82.57, 80.94, 46.80, 40.89, 36.38, 34.74, 33.09, 32.50, 30.96, 28.24, 25.21, 23.61.

ESI-HRMS: *(m/z)* calcd. for $C_{17}H_{26}NO_4$ ($[M+H]^+$) 308.1856, found: 308.1852.

Step 8: Synthesis of compound (±)**-30**

The compound (±)**-29** (0.50 g, 1.6 mmol) was dissolved in $CH_2Cl_2$ (5 mL), and TFA (3 mL) was slowly added dropwise under an ice water bath. After the dropping was completed, a reaction was carried out at room temperature for 4 h. TLC monitoring showed that the reaction was completed. After the reaction solution was concentrated under reduced pressure on a rotary evaporator to obtain a yellow oily substance, n-hexane (3 mL) was added into the oily substance, ultrasonic treatment was performed to convert the oily substance into a solid, which was stirred and slurried continuously at room temperature for 1 h. The solid was collected by suction filtration and dried with a vacuum oil pump to obtain compound (±)**-30**, a white solid, 0.33 g (80%), with a melting point of 120.0°C-123.6°C.

$^1H$ NMR ($CDCl_3$, 500 MHz) δ: 6.47-6.50 (m, 1H), 6.28-6.32 (m, 1H), 4.78 (dd, 1H, *J* = 0.8 Hz and 5.9 Hz), 4.68 (d, 1H, *J* = 11.5 Hz), 2.80 (d, 1H, *J* = 18.0 Hz), 2.73 (d, 1H, *J* = 18.0 Hz), 2.53-2.65 (m, 3H), 2.27-2.30 (m, 1H), 1.95 (ddd, 1H, *J* = 2.0 Hz, 8.5 Hz and 13.5 Hz), 1.66 (dd, 1H, *J* = 2.3 Hz and 13.3 Hz), 1.32-1.42 (m, 2H), 1.25-1.31 (m, 1H), 1.17-1.24 (m, 1H).

$^{13}C$ NMR ($CDCl_3$, 126 MHz) δ: 176.37, 134.66, 134.33, 82.31, 46.58, 40.37, 34.65, 32.97, 32.78, 30.85, 25.17, 23.55.

ESI-HRMS: *(m/z)* calcd. for $C_{13}H_{18}NO_4$ ($[M+H]^+$) 252.1230, found: 252.1229.

Step 9: Synthesis of compound (±)**-I-5**

The compound (±)**-30** (0.30 g, 1.2 mmol) was dissolved in $CH_3OH$ (10 mL), 10% $Pd(OH)_2/C$ (0.20 g) was added thereto, air in a reaction vessel was replaced with hydrogen (balloon) according to standard operations, and stirring was performed overnight at room temperature. TLC monitoring showed that a reaction was completed (the reaction was generally completed within 12 h). Filtration was performed to remove a solid. A filtrate was concentrated under reduced

pressure on a rotary evaporator to obtain a white solid, and $CH_3OH$ (2 mL)/EtOAc (3 mL) was added and stirred at room temperature for 0.5 h. The solid was collected by suction filtration and dried to obtain target product (±)**-I-5**, a white solid, 0.13 g (49%), with a melting point of 176.6°C-178.2°C.

$^1$H NMR ($CD_3OD$, 500 MHz) δ: 3.16 (d, 1H, *J* = 13.0 Hz), 3.07 (d, 1H, *J* = 13.0 Hz), 2.74 (d, 1H, *J* = 16.5 Hz), 2.67 (d, 1H, *J* = 16.0 Hz), 2.45-2.52 (m, 1H), 2.20-2.27 (m, 2H), 2.01-2.04 (m, 1H), 1.90-1.96 (m, 1H), 1.85 (ddd, 1H, *J* = 3.0 Hz, 8.5 Hz and 12.5 Hz), 1.74-1.76 (m, 1H), 1.46-1.70 (m, 6H), 1.36-1.43 (m, 1H). $^{13}$C NMR ($CD_3OD$, 126 MHz) δ: 180.15, 51.74, 46.55, 44.33, 40.14, 33.41, 33.09, 27.72, 26.94, 26.50, 23.84, 22.75.

ESI-HRMS: *(m/z)* calcd. for $C_{13}H_{22}NO_2$ ($[M+H]^+$) 224.1645, found: 224.1641.

The compound **(±)-I-5** is a specific form of the compound of general formula **I** in the present application.

## Example 6 Synthesis of compound (±)-I-6 and a p-toluenesulfonate thereof

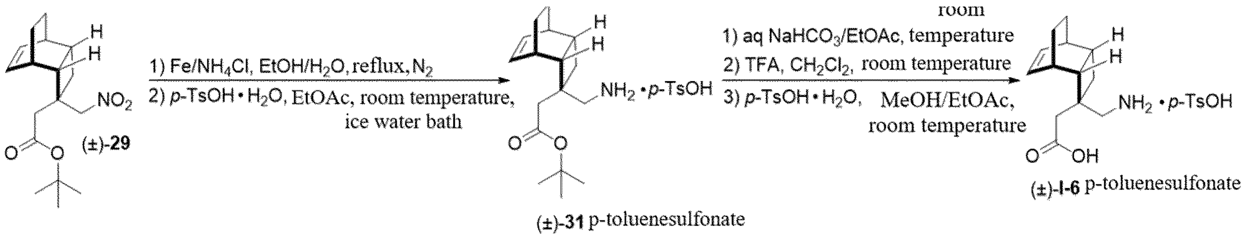


(±)-**29**　(±)-**31** p-toluenesulfonate　(±)-**I-6** p-toluenesulfonate

Step 1: Synthesis of the compound (±)**-31** p-toluenesulfonate

At room temperature, the compound (±)**-29** (1.10 g, 3.6 mmol) was dissolved in EtOH (10 mL), water (5 mL) was added thereto and stirred, then iron powder (1.30 g, 23 mmol) and $NH_4Cl$ (0.45 g, 8.4 mmol) were sequentially added thereto, air in a reaction vessel was replaced with nitrogen (balloon) according to standard operations, and refluxing was performed under stirring for 5 h. TLC monitoring showed that a reaction was completed. The reaction solution was cooled to room temperature and filtered to remove a solid. A filtrate was washed with a saturated $NaHCO_3$ solution (120 mL) and extracted with EtOAc (50 mL×3). Organic phases were combined, dried ($MgSO_4$), and filtered to remove the drying agent. The filtrate was concentrated under reduced pressure on a rotary evaporator to obtain a yellow oily substance. At room temperature, EtOAc (15 mL) was added to dilute the oily substance, then *p*-TsOH·$H_2O$ (0.70 g, 3.7 mmol) was added and stirred for dissolution until a large amount of solid was precipitated out, and the system was transferred into an ice water bath and stirred for 1 h. The solid was collected by filtration and dried with a vacuum oil pump to obtain (±)**-31** p-toluenesulfonate, a white solid, 1.30 g (81%), with a melting point of 190.1°C-192.5°C.

$^1$H NMR (DMSO-$d_6$, 500 MHz) δ: 7.69 (brs, 3H), 7.48 (d, 2H, *J* = 6.5 Hz), 7.12 (d, 2H, *J* = 8.0 Hz), 6.44-6.46 (m, 1H), 6.25-6.28 (m, 1H), 3.07-3.11 (m, 1H), 2.96-3.00 (m, 1H), 2.40-2.54 (m, 5H), 2.29 (s, 3H), 2.12-2.14 (m, 1H), 1.74 (ddd, 1H, *J* = 2.0 Hz, 8.5 Hz and 13.0 Hz), 1.38-1.42 (m, 10H), 1.31-1.33 (m, 2H), 1.17-1.21 (m, 1H), 1.09-1.14 (m, 1H).

$^{13}$C NMR (DMSO-$d_6$, 126 MHz) δ: 170.55, 145.64, 137.66, 134.31, 133.80, 128.07, 125.49, 80.09, 46.72, 45.55, 38.85, 35.92, 33.77, 32.38, 31.83, 30.37, 27.74, 24.67, 23.21, 20.78.

ESI-HRMS: *(m/z)* calcd. for $C_{17}H_{28}NO_2$ ([M(free base)+H]$^+$) 278.2115, found: 278.2111.

Step 2: Synthesis of the compound (±)-**I-6** and a p-toluenesulfonate thereof

At room temperature, (±)**-31** p-toluenesulfonate (1.30 g, 2.9 mmol) and a saturated $NaHCO_3$ solution (100 mL) were stirred for 20 min (suspension state), and EtOAc (60 mL×3) was added for extraction. Organic phases were combined, dried ($MgSO_4$), and filtered to remove the drying agent. A filtrate was concentrated under reduced pressure on a rotary evaporator to obtain a light yellow oily substance, and the oily substance was dissolved in $CH_2Cl_2$ (10 mL). Under an ice water bath, TFA (7 mL) was slowly added dropwise. After the dropping was completed, a reaction was carried out at room temperature for 4 h. TLC monitoring showed that the reaction was completed. The reaction solution was concentrated under reduced pressure on a rotary evaporator to obtain a brown oily substance, and $CH_2Cl_2$ (20 mL) was added for concentration again. The oily substance was dried with a vacuum oil pump until a solid, that is, (±)**-I-6**, was completely precipitated out. The solid was dissolved with $CH_3OH$ (3 mL), and EtOAc (8 mL) was added and stirred. Then *p*-TsOH·$H_2O$ (0.60 g, 3.2 mmol) was added into the reaction solution, stirred for dissolution until a large amount of solid was precipitated out, and continuously stirred at room temperature for 0.5 h. The solid was collected by filtration and dried with a vacuum oil pump to obtain the compound (±)**-I-6** p-toluenesulfonate, a white solid, 0.70 g (62%), with a melting point of 192.7°C-194.0°C.

$^1H$ NMR ($CD_3OD$, 500 MHz) δ: 7.71 (d, 2H, $J$ = 8.0 Hz), 7.23 (d, 2H, $J$ = 8.5 Hz), 6.48-6.52 (m, 1H), 6.28-6.32 (m, 1H), 3.26 (d, 1H, $J$ = 12.5 Hz), 3.14 (d, 1H, $J$ = 13.0 Hz), 2.66 (d, 1H, $J$ = 17.0 Hz), 2.56 (d, 1H, $J$ = 17.0 Hz), 2.56-2.67 (m, 2H), 2.51-2.53 (m, 1H), 2.19-2.22 (m, 1H), 1.74-1.79 (m, 1H), 1.58-1.64 (m, 1H), 1.40-1.43 (m, 2H), 1.27-1.32 (m, 1H), 1.19-1.24 (m, 1H).

$^{13}C$ NMR ($CD_3OD$, 126 MHz) δ: 175.74, 143.57, 141.67, 135.55, 135.31, 129.80, 126.97, 49.41, 47.40, 40.12, 36.92, 35.65, 33.98, 33.67, 32.16, 25.98, 24.39, 21.30.

ESI-HRMS: *(m/z)* calcd. for $C_{13}H_{20}NO_2$ ([M(free base)+H]$^+$) 222.1489, found: 222.1485.

The compound (±)**-I-6** is a specific form of the compound of general formula **I** in the present application.

**Example 7 Synthesis of compounds (-)-1-3 and (+)-1-3 and p-toluenesulfonates thereof**

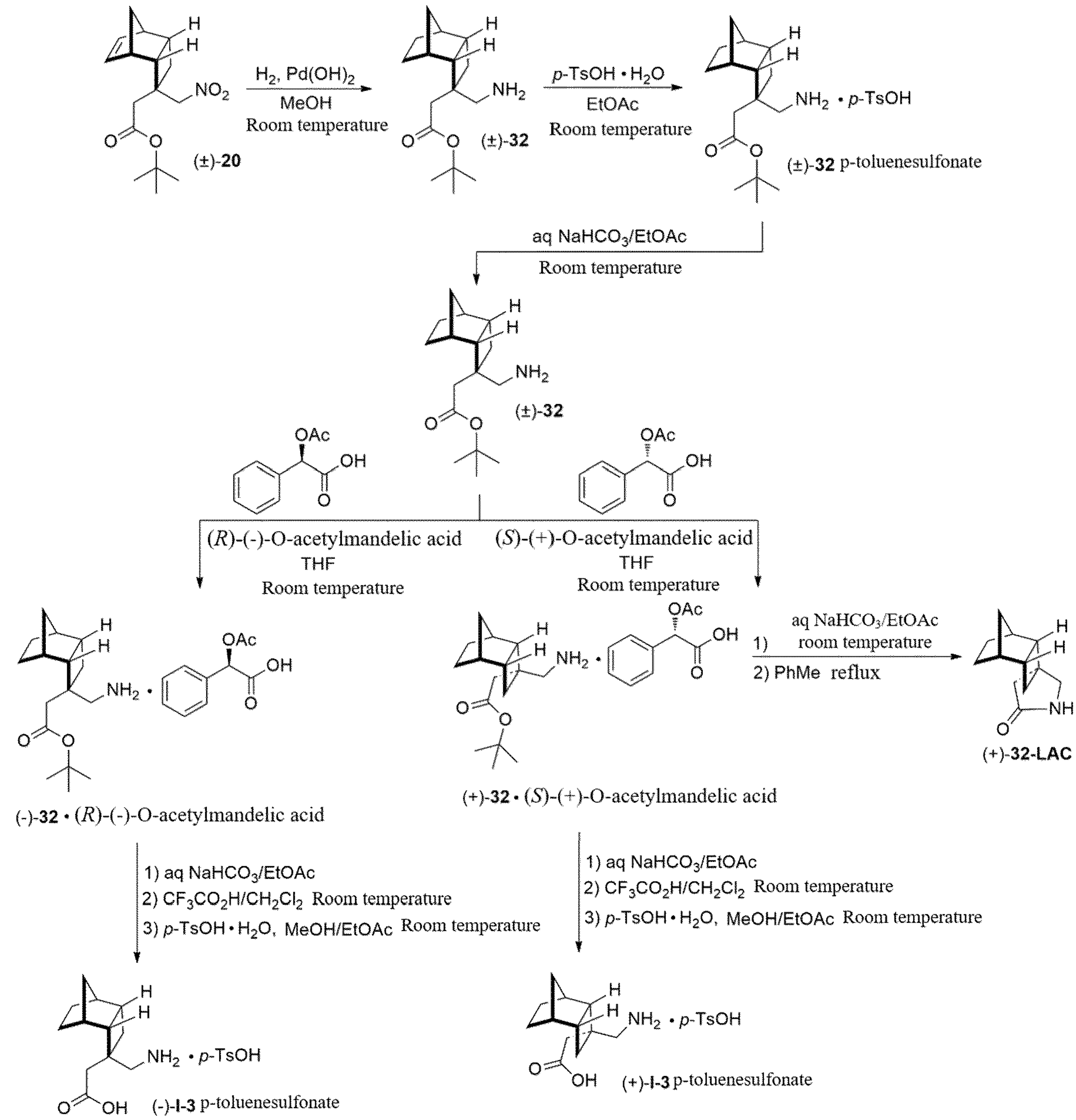


Step 1: Synthesis of the compound (±)**-32** p-toluenesulfonate

The compound (±)**-20** (140.00 g, 0.48 mol) was dissolved in $CH_3OH$ (800 mL), 10% $Pd(OH)_2/C$ (22.00 g) was added thereto, air in a reaction vessel was replaced with hydrogen (balloon) according to standard operations, and stirring was performed at room temperature for 12 h. TLC monitoring showed that a reaction was completed. Filtration was

performed. A filtrate was concentrated under reduced pressure on a rotary evaporator to obtain a crude product of compound (±)-**32**. The compound (±)-**32** was diluted with $CH_2Cl_2$ (800 mL), a saturated $NaHCO_3$ solution (2 L) was added for washing, an organic phase was separated, and an aqueous phase was extracted with $CH_2Cl_2$ (500 mL×2). Organic phases were combined, dried ($MgSO_4$), and filtered to remove the drying agent. A filtrate was concentrated under reduced pressure on a rotary evaporator to obtain a yellow oily substance. At room temperature, EtOAc (800 mL) was added to dilute the oily substance, then *p*-TsOH·$H_2O$ (70.00 g, 0.37 mol) was added and stirred for dissolution until a large amount of solid was precipitated out, and stirring was performed at room temperature for 3 h. The solid was collected by suction filtration and dried with a vacuum oil pump to obtain (±)-**32** p-toluenesulfonate, a white solid, 75.20 g (36%), with a melting point of 170.1°C-174.4°C.

$^1$H NMR (DMSO-$d_6$, 500 MHz) δ: 7.73 (brs, 3H), 7.48 (d, 2H, *J* = 8.0 Hz), 7.12 (d, 2H, *J* = 7.5 Hz), 3.02-3.07 (m, 1H), 2.91-2.95 (m, 1H), 2.61 (d, 1H, *J* = 17.0 Hz), 2.57 (d, 1H, *J* = 17.0 Hz), 2.39-2.46 (m, 1H), 2.27-2.29 (m, 4H), 2.18-2.20 (m, 1H), 2.10-2.13 (m, 1H), 1.83-1.88 (m, 1H), 1.68-1.79 (m, 3H), 1.41-1.53 (m, 11H), 1.31-1.33 (m, 1H), 1.13-1.16 (m, 1H).

$^{13}$C NMR (DMSO-$d_6$, 126 MHz) δ: 170.59, 145.54, 137.76, 128.11, 125.51,80.17, 48.51, 48.43, 48.23, 41.52, 38.50, 35.42, 35.39, 33.04, 30.92, 27.74, 24.64, 23.74, 20.80.

3,5-dinitrobenzoylated derivatization: synthesis of a 3,5-dinitrobenzoylated derivative of the compound (±)-**32**

At room temperature, the compound (±)-**32** p-toluenesulfonate (1.00 g, 2.3 mmol) was added into a saturated $NaHCO_3$ solution (200 mL), stirred into a suspension, and then extracted with EtOAc (100 mL). An organic phase was washed again with a saturated $NaHCO_3$ solution (200 mL) and separated by extraction, and an aqueous phase was extracted with EtOAc (50 mL). Organic phases were combined, dried ($MgSO_4$), and filtered to remove the drying agent. A filtrate was concentrated under reduced pressure on a rotary evaporator to obtain light yellow oily compound (±)-**32.** The above oily compound was dissolved in $CH_2Cl_2$ (7 mL) and stirred, 3,5-dinitrobenzoyl chloride (0.6 g, 2.6 mmol) was added, then triethylamine (0.6 mL) was added dropwise, and after the dropping was completed, a reaction was carried out at room temperature for 1 h. TLC monitoring showed that the reaction was completed. The reaction solution was directly purified by a column chromatography [V(EtOAc)/V(n-hexane)=0/1→1/4] to obtain a light yellow oily substance. n-hexane (10 mL) was added into the oily substance to precipitate a solid, which was slurried at room temperature for 1 h. The solid was collected by suction filtration and dried under vacuum to obtain a 3,5-dinitrobenzoylated derivative of (±)-**32**, a white solid, 0.70 g (67%), with a melting point of 113.9°C-115.4°C.

$^1$H NMR ($CDCl_3$, 500 MHz) δ: 9.40 (brs, 1H), 9.20 (d, 2H, *J* = 2.0 Hz), 9.15 (t, 1H, *J* = 2.0 Hz), 3.70 (dd, 1H, *J* = 4.5 Hz and 13.5 Hz), 3.43 (dd, 1H, *J* = 3.3 Hz and 13.3 Hz), 2.84 (dd, 1H, *J* = 1.0 Hz and 12.0 Hz), 2.71 (d, 1H, *J* = 17.0 Hz), 2.54-2.60 (m, 1H), 2.36-2.38 (m, 1H), 2.30-2.33 (m, 1H), 2.26-2.28 (m, 1H), 1.86-1.94 (m, 2H), 1.75-1.82 (m, 2H), 1.50-1.62 (m, 11H), 1.38-1.40 (m, 1H), 1.21-1.24 (m, 1H).

ESI-HRMS: *(m/z)* calcd. for $C_{23}H_{28}N_3O_7$ ($[M-H]^-$) 458.1933, found: 458.1939.

The 3,5-dinitrobenzoylated derivative of (±)-**32** was used as a reference substance for testing the optical purity of (-)-**32** and (+)-**32** obtained after chiral acid resolution of (±)-**32** in the following steps 2 and 3 using a chiral HPLC method.

Step 2: Synthesis of an (*R*)-(-)-O-acetylmandelate of the compound (-)-**32**

(±)-**32** p-toluenesulfonate (22.80 g, 52 mmol) was added into a saturated $NaHCO_3$ solution (200 mL× 2), stirred, and extracted with EtOAc (200 mL×2). Organic phases were combined, dried ($MgSO_4$), and filtered to remove the drying agent. A filtrate was concentrated under reduced pressure on a rotary evaporator to obtain a light yellow oily substance. At room temperature, THF (100 mL) was added to dilute the oily substance, and then a THF (50 mL) solution of (*R*)-(-)-O-acetylmandelic acid (5.00 g, 26 mmol) was added dropwise. After the dropping was completed, a large amount of solid was gradually precipitated out, THF (8 mL) was supplemented, and stirring was performed overnight at room temperature. The solid was collected by suction filtration (a filtrate was recovered). A filter cake was dried with a vacuum oil pump to obtain a white solid (7.35 g, 16 mmol), that is, an (*R*)-(-)-O-acetylmandelate of the compound (-)-**32.**

3,5-dinitrobenzoylated derivatization: synthesis of a 3,5-dinitrobenzoylated derivative of the compound (-)-**32**. The above white solid (0.1 g, 0.22 mmol) was added into a saturated $NaHCO_3$ solution (50 mL× 2), stirred, and extracted with EtOAc (25 mL×2). Organic phases were combined, dried ($MgSO_4$), and filtered to remove the drying agent. A filtrate was concentrated under reduced pressure on a rotary evaporator to obtain a light yellow oily substance. At room temperature, $CH_2Cl_2$ (3 mL) was added into the above oily substance, then 3,5-dinitrobenzoyl chloride (0.1 g, 0.43 mmol) was added and stirred, triethylamine (2-3 drops) was added dropwise, and after the dropping was completed, a reaction was carried out at room temperature for 10 min. TLC monitoring showed that the reaction was completed. The reaction solution was directly purified by a column chromatography [V(EtOAc)/V(n-hexane)=0/1→1/4] to obtain a light yellow oily substance, and then dried under vacuum to obtain a white foam solid of a 3,5-dinitrobenzoylated derivative of (-)-**32**. An ee value of the derivative determined by chiral HPLC was 80.54%.

Enantiomeric excess (%ee) determination method (chiral HPLC method): Determination was performed using a Daicel Chiralpak AS-RH 4.6 mm×250 mm chromatographic column (5 μm) in an Agilent 1260 infinity Type II liquid chromatograph with a detector wavelength of 220 nm, a mobile phase of acetonitrile / 0.1% $KH_2PO_4$-KOH buffer solution

(pH=7.0) = 70/30, a flow rate of 1 mL/min, a sample injection concentration of 0.5 mg/mL, and a sample injection volume of 3 μL.

At room temperature, the above (*R*)-(-)-O-acetylmandelate of the compound (-)-**32** (6.35 g, 14 mmol) as the white solid obtained after first resolution was fully stirred with THF (65 mL), then transferred into an oil bath, and stirred at 40°C for 10 min (the system was slightly dissolved). Then, the system was placed at room temperature, THF (30 mL) was supplemented, and stirring was performed overnight. A solid was collected by suction filtration and dried with a vacuum oil pump to obtain a white solid (4.90 g, 11 mmol). The above recrystallization method was repeated to crystallize again, to obtain an (*R*)-(-)-O-acetylmandelate of the compound (-)-**32**. A derivative was obtained according to the above 3,5-dinitrobenzoylated derivatization steps, and an ee value thereof was 99.34% as determined by chiral HPLC, a white solid, 4.40 g (37%). The product was directly used in a next reaction without characterization.

Step 3: Synthesis of an (*S*)-(+)-O-acetylmandelate of the compound (+)-**32**

A filtrate obtained after the filtration of the resolved crystals in step 2 was concentrated, into which a saturated $NaHCO_3$ solution (100 mL×2) was added, stirred, and extracted with EtOAc (100 mL×2). Organic phases were combined, dried ($MgSO_4$), filtered, and concentrated under reduced pressure on a rotary evaporator to obtain a light yellow oily substance (12.85 g, calculated in 48 mmol). At room temperature, THF (100 mL) was added to dilute the oily substance, and then a THF (50 mL) solution of (*S*)-(+)-O-acetylmandelic acid (4.70 g, 24 mmol) was added dropwise (a large amount of solid was gradually precipitated out when half of the solution was dropped). After the dropping was completed, THF (30 mL) was supplemented, and stirring was performed at room temperature for 2 h. Filtration was performed. A filter cake was dried with a vacuum oil pump to obtain an (*S*)-(+)-O-acetylmandelate of (+)-**32** as a white solid (6.00 g, 13 mmol). A derivative was obtained according to the above 3,5-dinitrobenzoylated derivatization steps, and an ee value thereof was 90.77% as determined by chiral HPLC.

At room temperature, the above white solid was fully stirred with THF (60 mL), then transferred into an oil bath, and stirred at 40°C for 10 min (the system was slightly dissolved). Then, the system was placed at room temperature, THF (30 mL) was supplemented, and stirring was performed overnight. The solid was collected by suction filtration and dried with a vacuum oil pump to obtain an (S)-(+)-O-acetylmandelate of the compound (+)-**32**. A derivative was obtained according to the above 3,5-dinitrobenzoylated derivatization steps, and an ee value thereof was 99.17% as determined by chiral HPLC, a white solid, 4.40 g (40%). The product was directly used in a next reaction without characterization.

Step 4: Synthesis of compound (-)-**I-3** and a p-toluenesulfonate thereof

At room temperature, the (R)-(-)-O-acetylmandelate of the compound (-)-**32** (1.50 g, 3.3 mmol) was added into a saturated $NaHCO_3$ solution (100 mL) and stirred for 20 min (suspension state), and EtOAc (50 mL×3) was added for extraction. Organic phases were combined, dried ($MgSO_4$), and filtered. After a filtrate was concentrated under reduced pressure on a rotary evaporator to obtain a yellow oily substance, the oily substance was dissolved in $CH_2Cl_2$ (10 mL), and TFA (7 mL) was slowly added dropwise under an ice water bath. After the dropping was completed, a reaction was carried out at room temperature for 4-6 h. TLC monitoring showed that the reaction was completed. After the reaction solution was concentrated under reduced pressure on a rotary evaporator to obtain a brown oily substance, $CH_2Cl_2$ (10 mL) was added for concentration again, and the resulting oily substance was dried with a vacuum oil pump to obtain (-)-32. The above (-)-**32** sample was dissolved in $CH_3OH$ (2.5 mL), then EtOAc (12 mL) was added and stirred, p-TsOH·$H_2O$ (0.70 g, 3.7 mmol) was added into the solution and stirred for dissolution until a large amount of solid was precipitated out, and stirring was performed continuously at room temperature for 1 h. The solid was collected by suction filtration and dried with a vacuum oil pump to obtain compound (-)-**I-3** p-toluenesulfonate, a white solid, 1.00 g (80%), with a melting point of 180.6°C-183.5°C, $[\alpha]_D^{20}$ =-22.8 (c=2.50, $CH_3OH$).

$^1H$ NMR ($CD_3OD$, 500 MHz) δ: 7.70 (d, 2H, *J* = 8.0 Hz), 7.23 (d, 2H, *J* = 8.0 Hz), 3.20 (d, 1H, *J* = 13.0 Hz), 3.10 (d, 1H, *J* = 13.0 Hz), 2.79 (d, 1H, *J* = 17.5 Hz), 2.70 (d, 1H, *J* = 17.5 Hz), 2.49-2.57 (m, 1H), 2.41-2.43 (m, 1H), 2.37 (s, 3H), 2.22-2.27 (m, 2H), 1.92-1.97 (m, 1H), 1.84-1.89 (m, 2H), 1.76-1.81 (m, 1H), 1.57-1.63 (m, 1H), 1.49-1.55 (m, 1H), 1.41-1.44 (m, 1H), 1.25-1.28 (m, 1H).

$^{13}C$ NMR ($CD_3OD$, 126 MHz) δ: 177.10, 143.14, 140.84, 130.31, 126.34, 50.73, 49.15, 42.42, 40.52, 40.21, 39.64, 36.05, 34.47, 32.47, 25.62, 24.79, 21.41.

The compound (-)-**I-3** is a specific form of the compound of general formula **I** in the present application, and is also one of (±)-**I-3,** which is an optically pure compound having a same relative configuration as the (±)-**I-3** and having levorotatory optical rotation.

Step 5: Synthesis of compound (+)-**I-3** and a p-toluenesulfonate thereof

At room temperature, the (S)-(+)-O-acetylmandelate of the compound (+)-**32** (1.40 g, 3.0 mmol) was added into a

saturated $NaHCO_3$ solution (100 mL) and stirred for 20 min (suspension state), and EtOAc (50 mL×3) was added for extraction. Organic phases were combined, dried ($MgSO_4$), and filtered to remove the drying agent. After a filtrate was concentrated under reduced pressure on a rotary evaporator to obtain a yellow oily substance, the oily substance was dissolved in $CH_2Cl_2$ (10 mL), and TFA (7 mL) was slowly added dropwise under an ice water bath. After the dropping was completed, a reaction was carried out at room temperature for 4-6 h. TLC monitoring showed that the reaction was completed. After the reaction solution was concentrated under reduced pressure on a rotary evaporator to obtain a brown oily substance, $CH_2Cl_2$ (10 mL) was added for concentration again, and the resulting oily substance was dried with a vacuum oil pump to obtain (+)-**I-3.** The above (+)-**I-3** sample was dissolved in $CH_3OH$ (2 mL), and EtOAc (6 mL) was added again and stirred. Then *p*-TsOH·$H_2O$ (0.60 g, 3.2 mmol) was added into the solution, stirred for dissolution until a large amount of solid was precipitated out, and continuously stirred at room temperature for 1 h. The solid was collected by suction filtration and dried with a vacuum oil pump to obtain a p-toluenesulfonate of the compound (+)-**I-3,** a white solid 0.85 g (73%), with a melting point of 179.5°C-181.8°C, $[\alpha]_D^{20}$=+25.09 (c=2.55, $CH_3OH$).

$^1$H NMR ($CD_3OD$, 500 MHz) δ: 7.71 (d, 2H, *J* = 8.0 Hz), 7.23 (d, 2H, *J* = 8.0 Hz), 3.20 (d, 1H, *J* = 13.0 Hz), 3.10 (d, 1H, *J* = 13.0 Hz), 2.78 (d, 1H, *J* = 17.5 Hz), 2.70 (d, 1H, *J* = 17.5 Hz), 2.48-2.55 (m, 1H), 2.40-2.42 (m, 1H), 2.37 (s, 3H), 2.21-2.26 (m, 2H), 1.90-1.95 (m, 1H), 1.83-1.88 (m, 2H), 1.76-1.80 (m, 1H), 1.56-1.62 (m, 1H), 1.49-1.54 (m, 1H), 1.40-1.42 (m, 1H), 1.24-1.27 (m, 1H).

$^{13}$C NMR ($CD_3OD$+$D_2O$ (1 drop), 126 MHz) δ: 176.94, 142.97, 141.11, 130.25, 126.38, 50.74, 49.21, 42.44, 40.56, 40.17, 39.67, 36.10, 34.50, 32.47, 25.65, 24.81, 21.40.

The compound (+)-**I-3** is a specific form of the compound of general formula **I** in the present application, and is also one of (±)-**I-3,** which is an optically pure compound having a same relative configuration as (±)-**I-3** and having dextrorotatory optical rotation.

A method of determining an absolute configuration of the compound (+)-**I-3** is as follows: converting the (*S*)-(+)-o-acetylmandelate of (+)-**32** having the same absolute configuration into the lactam thereof (+)**-32-LAC,** and determining the absolute configuration of (+)-**32-LAC** by using an X-ray single crystal diffraction method, wherein the absolute configuration is the same as that of (+)**-32**. The absolute configuration of (+)-**I-3** can be indirectly determined by the method.

A specific experimental method is as follows:

Synthesis of (+)-**32-LAC:** The above (S)-(+)-O-acetylmandelate of (+)-**32** with the ee value of 99.17% (1.00 g, 2.2 mmol) was added into a saturated $NaHCO_3$ solution (100 mL) and stirred for 10 min, and then EtOAc (30 mL×3) was added for extraction. Organic phases were combined, washed with a brine, dried ($MgSO_4$), and filtered to remove the drying agent. A filtrate was evaporated to dryness on a rotary evaporator. A resulting residue was dissolved with toluene (7 mL) and heated to reflux overnight. TLC showed that a reaction was completed. The reaction system was cooled to room temperature, the solvent was removed by evaporation on a rotary evaporator, EtOAc/n-hexane (1/10 by v/v, 3 mL in total) was added into the residue for slurrying, and a crystal was collected by suction filtration and dried to obtain (+)**-32-LAC,** 0.28 g (67%), with a melting point of 189.3°C-191.5°C, $[\alpha]_D^{20}$=+62.6° (*c*=1.15, $CH_3OH$).

$^1$H NMR ($CDCl_3$, 500 MHz) δ: 6.05 (brs, 1H), 3.45 (d, 1H, *J* = 9.5 Hz), 3.36 (d, 1H, *J* = 9.5 Hz), 2.47-2.52 (m, 1H), 2.43 (d, 1H, *J* = 17.0 Hz), 2.39-2.42 (m, 1H), 2.34 (d, 1H, *J* = 17.0 Hz), 2.23-2.27 (m, 2H), 2.02 (dd, 1H, *J* = 7.0 Hz and 13.0 Hz), 1.93-1.98 (m, 1H), 1.70-1.75 (m, 1H), 1.60-1.66 (m, 1H), 1.51-1.58 (m, 1H), 1.42-1.49 (m, 1H), 1.39-1.41 (m, 1H), 1.20-1.23 (m, 1H).

$^{13}$C NMR ($CDCl_3$, 126 MHz) δ: 178.29, 58.68, 51.24, 41.99, 41.78, 39.62, 39.51, 38.99, 35.31, 33.54, 25.59, 23.95.

ESI-HRMS: *(m/z)* calcd. for $C_{12}H_{18}NO$ ($[M+H]^+$) 192.1383, found: 192.1380.

Culture and X-ray diffraction of a (+)**-32-LAC** single crystal: 10 mg of the (+)**-32-LAC** sample was weighed and dissolved in $CH_2Cl_2$ (1 mL), then n-hexane (2 mL) was added and shaken evenly, filtration was performed, and a filtrate was put into a small glass triangular flask and slowly volatilized at room temperature for 2-3 days to obtain a single crystal suitable for X-ray diffraction. A single crystal with a size of 0.12×0.1 ×0.08 mm was selected for diffraction with CuKα rays at a temperature of 100.00 (10) K on a Rigaku XtaLAB Pro single crystal diffractometer, CrysAlisPro 1.171.39.33c (Rigaku OD, 2017) was used for collecting diffraction data and performing data reduction, and an SHELXL program was used for performing structural analysis and refinement.

A chemical structure of the compound (+)**-32-LAC** in single crystal diffraction (ORTEP diagram) is shown in FIG. 1.

Crystal test and structural refinement relevant parameters of (+)**-32-LAC** are shown in the following table.

| | |
|---|---|
| CCDC deposition number | 2209119 |
| Radiation type | Cu Kα |
| Radiation wavelength/Å | λ = 1.54184 |
| Crystal size/mm$^3$ | 0.12 × 0.1 × 0.08 |

(continued)

| | |
|---|---|
| 2 θ range for data collection/° | 8.364 to 148.950 |
| Empirical formula | $C_{12}H_{17}NO$ |
| Formula weight | 191.26 |
| Temperature/K | 100.00(10) |
| Crystal system | Monoclinic |
| Space group | $P2_1$ |
| a/Å | 7.5507(1) |
| b/Å | 6.2505(1) |
| c/Å | 10.6250(1) |
| α/° | 90 |
| β/° | 95.800(1) |
| γ/° | 90 |
| Volume/Å$^3$ | 498.887(11) |
| Z | 2 |
| $\rho_{calcd}$g/cm$^3$ | 1.273 |
| μ/mm$^{-1}$ | 0.628 |
| F(000) | 208.0 |
| Index ranges | $-9 \le h \le 9$, $-7 \le k \le 7$, $-13 \le l \le 13$ |
| Reflections collected | 13898 |
| Independent reflections | 2017 [$R_{int}$ = 0.0382, $R_{sigma}$ = 0.0208] |
| Data/restraints/parameters | 2017/1/127 |
| Goodness-of-fit on F$^2$ | 1.059 |
| Final R indexes [I≥2σ (I)] | $R_1$ = 0.0394, $wR_2$ = 0.1056 |
| Final R indexes [all data] | $R_1$ = 0.0404, $wR_2$ = 0.1063 |
| Largest diff. peak/hole/e Å$^{-3}$ | 0.24/-0.13 |
| Flack parameter | 0.05(12) |

**Example 8 Synthesis of compounds (-)-I-4 and (+)-I-4 and p-toluenesulfonates thereof**

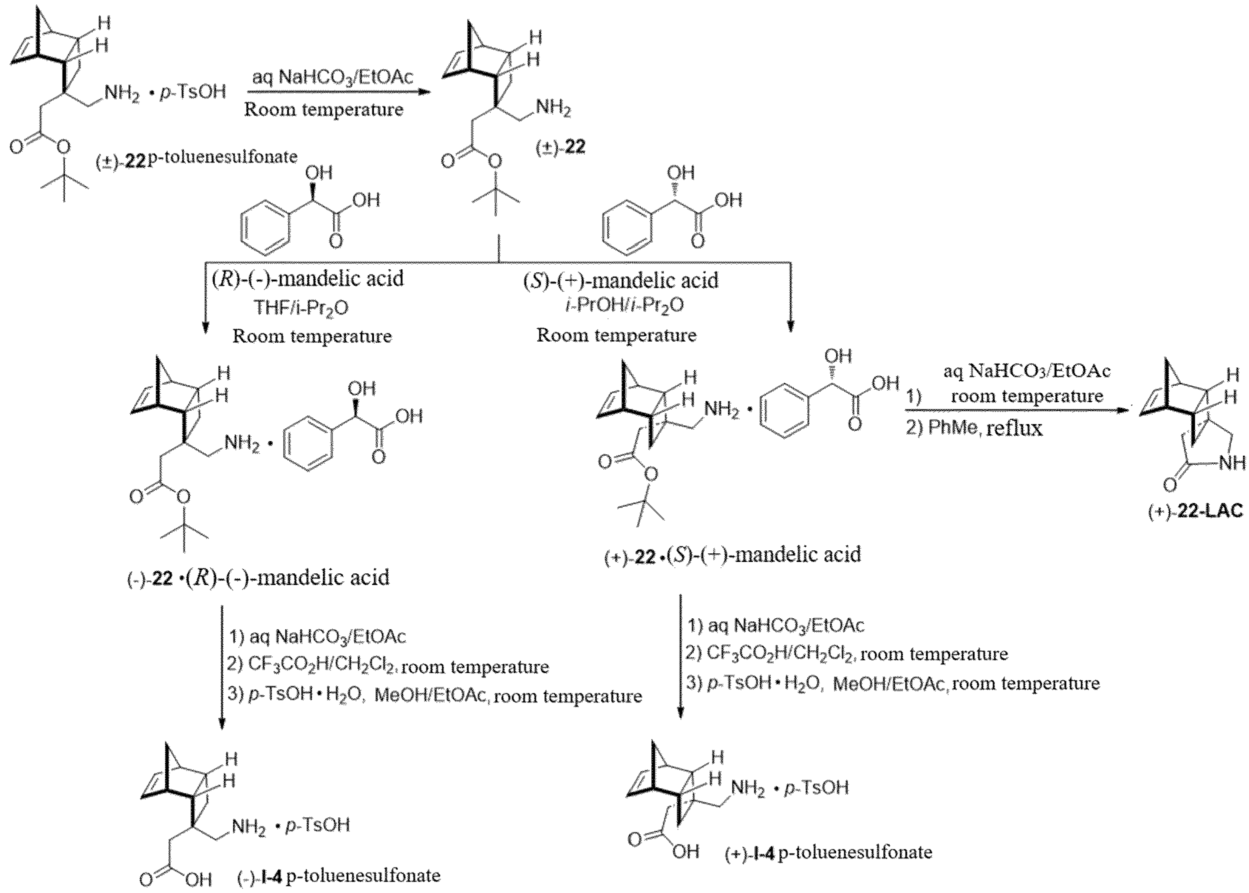


Step 1: Synthesis of compound (±)-**22**

At room temperature, (±)-**22** p-toluenesulfonate (60.00 g, 0.14 mol) was added into a saturated $NaHCO_3$ solution (600 mL×2), stirred, and extracted with EtOAc (400 mL×2). Organic phases were combined, dried ($MgSO_4$), filtered to remove the drying agent, and concentrated under reduced pressure on a rotary evaporator to obtain compound (±)-**22**, a yellow oily substance, 36 mL. The product was directly used in a next reaction without characterization.

3,5-dinitrobenzoylated derivatization: synthesis of a 3,5-dinitrobenzoylated derivative of the compound (±)-**22**

At room temperature, the compound (±)-**22** p-toluenesulfonate (1.00 g, 2.3 mmol) was added into a saturated $NaHCO_3$ solution (200 mL), stirred, and extracted with EtOAc (100 mL×2). Organic phases were combined, dried ($MgSO_4$), and filtered to remove the drying agent. A filtrate was concentrated under reduced pressure on a rotary evaporator to obtain light yellow oily compound (±)-**22.** The above oily compound was dissolved in $CH_2Cl_2$ (7 mL) and stirred, and then 3,5-dinitrobenzoyl chloride (0.60 g, 2.6 mmol) was added. Triethylamine (0.6 mL) was added dropwise, and after the dropping was completed, a reaction was carried out at room temperature for 1 h. TLC monitoring showed that the reaction was completed. The reaction solution was directly purified by a column chromatography [V(EtOAc)/V(n-hexane)=0/1→1/4] to obtain a light yellow oily substance. n-hexane (10 mL) was added into the oily substance, and slurried at room temperature for 1 h. A solid was collected by suction filtration and dried under vacuum to obtain the target product, 3,5-dinitrobenzoylated derivative of (±)-**22**, a white solid, 0.60 g (57%), with a melting point of 118.9°C-119.8°C.

$^1H$ NMR ($CDCl_3$, 500 MHz) δ: 9.46 (brs, 1H), 9.19 (d, 2H, *J* = 2.0 Hz), 9.16 (t, 1H, *J* = 2.0 Hz), 6.40-6.42 (m, 1H), 6.31-6.33 (m, 1H), 3.82 (dd, 1H, *J* = 5.5 Hz and 13.5 Hz), 3.36 (dd, 1H, *J* = 3.3 Hz and 13.8 Hz), 2.86-2.90 (m, 3H), 2.69 (dd, 1H, *J* = 1.0 Hz and 17.0 Hz), 2.58-2.60 (m, 1H), 2.37 (d, 1H, *J* = 17.5 Hz), 1.77-1.81 (m, 1H), 1.58-1.61 (m, 10H), 1.30 (dd, 1H, *J* = 5.8 Hz and 12.3 Hz), 1.11-1.13 (m, 1H).

$^{13}C$ NMR ($CDCl_3$, 126 MHz) δ: 175.42, 162.99, 148.82, 138.26, 138.12, 136.52, 127.59, 120.86, 82.84, 54.11, 53.02, 46.82, 45.87, 44.76, 42.21, 37.02, 35.75, 34.57, 28.14.

ESI-HRMS: (*m*/*z*) calcd. for $C_{23}H_{27}N_3NaO_7$ ($[M+Na]^+$) 458.1741, found: 458.1735.

The 3,5-dinitrobenzoylated derivative of (±)-**22** was used as a reference substance for testing the optical purity of **(-)-22** and (+)-**22** obtained after chiral acid resolution of (±)-**22** in the following steps 2 and 3 using a chiral HPLC method.

Step 2: Synthesis of an (*R*)-(-)-mandelate of the compound **(-)-22**

At room temperature, the compound (±)-**22** (18.14 g, 69 mmol) was dissolved in THF (90 mL), and then a THF solution (90 mL) of (*R*)-(-)-mandelic acid (4.50 g, 30 mmol) was added dropwise. After the dropping was completed, stirring was performed continuously for 30 min. Then, isopropyl ether (180 mL) was added dropwise, and stirring was performed overnight at room temperature until a crystal was precipitated out. When no solid was precipitated out, an appropriate amount of a seed crystal could be added into the system until the solid was gradually precipitated out. Then, a THF solution (9 mL) of (*R*)-(-)-mandelic acid (2.25 g, 15 mmol) was added dropwise, stirring was performed at room temperature for 1 h, and then isopropyl ether (180 mL) was added dropwise. After the dropping was completed, the solid amount in the system was increased, and stirring was performed at room temperature for 1 h. The solid was collected by suction filtration (a filtrate was recovered) and dried with a vacuum oil pump to obtain an (*R*)-(-)-mandelate of (-)-**22** (8.60 g, 21 mmol) as a white solid. A small amount of the sample was treated according to the above 3,5-dinitrobenzoylated derivatization steps and determined to have an ee value of 87.05% by chiral HPLC. At room temperature, the above (*R*)-(-)-mandelate of **(-)-22** (8.40 g, 20 mmol) as the white solid was fully stirred with a mixed solvent of THF (40 mL) and isopropyl ether (40 mL), then transferred into an oil bath and stirred at 65°C. The mixed solvent of THF (10 mL) and isopropyl ether (10 mL) was supplemented and added dropwise until the system was slightly dissolved, and stirring was performed continuously for 30 min. Then, the system was placed at room temperature and stirred overnight. A solid was collected by suction filtration and dried with a vacuum oil pump to obtain a white solid (6.60 g, 16 mmol). The solid was subjected to crystallization again by repeating the above recrystallization method with a solvent of THF/isopropyl ether = 1/2 (v/v) to obtain a pure product of the (*R*)-(-)-mandelate of the compound **(-)-22,** a small amount of which was treated according to the above 3,5-dinitrobenzoylated derivatization steps and determined to have an ee value of 99.10% by chiral HPLC, a white solid, 6.00 g (43%). The product was directly used in a next reaction without characterization.

Step 3: Synthesis of an (*S*)-(+)-mandelate of the compound (+)-**22**

At room temperature, the compound (±)-**22** (18.14 g, 69 mmol) was dissolved in isopropanol (45 mL), and then an isopropanol solution (45 mL) of (*S*)-(+)-mandelic acid (9.00 g, 59 mmol) was added dropwise. After the dropping was completed, no obvious changes were observed, stirring was performed continuously for 30 min, and isopropyl ether (270 mL) was added dropwise. After the dropping was completed, stirring was performed until a large amount of solid was generated. When no crystal was generated, an appropriate amount of a seed crystal was added into the system. The crystallization system was stirred overnight at room temperature. The crystal was collected by suction filtration (a filtrate was recovered) and dried with a vacuum oil pump to obtain an (*S*)-(+)-mandelate of (+)-**22** as a white solid (9.00 g, 22 mmol). A small amount of the sample was treated according to the above 3,5-dinitrobenzoylated derivatization steps and determined to have an ee value of 30.88% by chiral HPLC. At room temperature, the above (*S*)-(+)-mandelate of (+)-**22** (9.00 g, 22 mmol) was fully stirred with a mixed solvent of isopropanol/isopropyl ether = 1/2 (v/v, 50 mL in total), then transferred into an oil bath and stirred at 60°C (the system was slightly dissolved). Stirring was performed continuously for 10 min. Then, the system was placed at room temperature until a large amount of white solid was gradually precipitated out, and the mixed solvent of isopropanol/isopropyl ether =1/2 (v/v, 15 mL in total) was added dropwise and stirred continuously for 1-2 h. The solid was collected by suction filtration and dried with a vacuum oil pump to obtain a white solid (5.40 g, 13 mmol). The above recrystallization operation was repeated for twice to obtain an (*S*)-(+)-mandelate of the compound (+)-**22**, a small amount of which was treated according to the above 3,5-dinitrobenzoylated derivatization steps and determined to have an ee value of 98.34% by chiral HPLC, a white solid, 2.40 g (17%). The product was directly used in a next reaction without characterization.

Step 4: Synthesis of compound **(-)-I-4** and a p-toluenesulfonate thereof

At room temperature, the (*R*)-(-)-mandelate of the compound **(-)-22** (3.00 g, 7.2 mmol) was added into a saturated $NaHCO_3$ solution (100 mL) and stirred for 20 min, and EtOAc (80 mL×3) was added for extraction. Organic phases were combined, dried ($MgSO_4$), and filtered to remove the drying agent. After a filtrate was concentrated under reduced pressure on a rotary evaporator to obtain a yellow oily substance, the oily substance was dissolved in $CH_2Cl_2$ (20 mL), and TFA (15 mL) was slowly added dropwise under an ice water bath. After the dropping was completed, a reaction was carried out at room temperature for 4-6 h. TLC monitoring showed that the reaction was completed. After the reaction solution was concentrated under reduced pressure on a rotary evaporator to obtain a brown oily substance, $CH_2Cl_2$ (20 mL) was added for concentration again, and the resulting oily substance was dried with a vacuum oil pump to obtain **(-)-I-4.** The above **(-)-I-4** was dissolved in $CH_3OH$ (2.5 mL), then EtOAc (12 mL) was added and fully stirred, *p*-TsOH·$H_2O$ (1.40 g, 7.4 mmol) was added into the solution and stirred for dissolution until a large amount of solid was precipitated out, and stirring was performed continuously at room temperature for 1 h. The solid was collected by suction filtration, and a filter cake was dried with a vacuum oil pump to obtain a p-toluenesulfonate of the compound (-)-**I-4**, a white solid, 1.80 g (66%), with a melting

point of 180.3°C-183.5°C, $[\alpha]_D^{20}$=-35.47 (c=2.65, $CH_3OH$).

$^1H$ NMR ($CD_3OD$, 500 MHz) δ: 7.70 (d, 2H, *J* = 8.5 Hz), 7.23 (d, 2H, *J* = 8.0 Hz), 6.42-6.44 (m, 1H), 6.29-6.31 (m, 1H), 3.24 (d, 1H, *J* = 13.0 Hz), 3.12 (d, 1H, *J* = 13.0 Hz), 2.92-2.94 (m, 1H), 2.81-2.87 (m, 2H), 2.57 (d, 1H, *J* = 17.5 Hz), 2.49-2.53 (m, 1H), 2.37 (s, 3H), 2.36 (d, 1H, *J* = 17.5 Hz), 1.75-1.80 (m, 1H), 1.57-1.59 (m, 1H), 1.34 (dd, 1H, *J* = 4.5 Hz and 13.5 Hz), 1.14-1.15 (m, 1H).

$^{13}C$ NMR ($CD_3OD$, 126 MHz) δ: 175.72. 143.52, 141.71, 138.50, 137.88, 129.82, 126.96, 53.81, 50.45, 48.14, 46.89, 45.84, 37.91, 37.15, 35.07, 34.06, 21.31.

The compound **(-)-I-4** is a specific form of the compound of general formula **I** in the present application, and is also one of (±)-**I-4**, which is an optically pure compound having a same relative configuration as (±)-**I-4** and having levorotatory optical rotation.

Step 5: Synthesis of compound (+)-**I-4** and a p-toluenesulfonate thereof

At room temperature, the compound (+)-**22** (*S*)-(+)-mandelate (2.40 g, 5.8 mmol) was added into a saturated $NaHCO_3$ solution (100 mL) and stirred for 20 min, and EtOAc (60 mL×3) was added for extraction. Organic phases were combined, dried ($MgSO_4$), and filtered to remove the drying agent. After a filtrate was concentrated under reduced pressure on a rotary evaporator to obtain a yellow oily substance, the oily substance was dissolved in $CH_2Cl_2$ (18 mL), and TFA (14 mL) was slowly added dropwise under an ice water bath. After the dropping was completed, a reaction was carried out at room temperature for 4-6 h. TLC monitoring showed that the reaction was completed. After the reaction solution was concentrated under reduced pressure on a rotary evaporator to obtain a brown oily substance, $CH_2Cl_2$ (20 mL) was added for concentration again, and the resulting oily substance was dried with a vacuum oil pump to obtain (+)-**I-4.** The dried oily substance was dissolved in $CH_3OH$ (2 mL), and EtOAc (8 mL) was added again and stirred. Then *p*-TsOH·$H_2O$ (1.10 g, 5.8 mmol) was added into the reaction solution, stirred for dissolution until a large amount of solid was precipitated out, and continuously stirred at room temperature for 1 h. The solid was collected by suction filtration, and a filter cake was dried with a vacuum oil pump to obtain a p-toluenesulfonate of the compound (+)-**I-4**, a white solid, 1.60 g (73%), with a melting point of 182.1°C-184.7°C, $[\alpha]_D^{20}$=+37.81 (c=2.75, $CH_3OH$).

$^1H$ NMR ($CD_3OD$, 500 MHz) δ: 7.70 (d, 2H, *J* = 8.0 Hz), 7.23 (d, 2H, *J* = 8.0 Hz), 6.42-6.44 (m, 1H), 6.29-6.31 (m, 1H), 3.24 (d, 1H, *J* = 13.0 Hz), 3.12 (d, 1H, *J* = 13.0 Hz), 2.92-2.94 (m, 1H), 2.81-2.87 (m, 2H), 2.57 (d, 1H, *J* = 17.5 Hz), 2.50-2.53 (m, 1H), 2.37 (s, 3H), 2.36 (d, 1H, *J* = 17.5 Hz), 1.75-1.80 (m, 1H), 1.57-1.59 (m, 1H), 1.34 (dd, 1H, *J* = 6.0 Hz and 13.5 Hz), 1.13-1.16 (m, 1H).

$^{13}C$ NMR ($CD_3OD$, 126 MHz) δ: 175.73, 143.53, 141.70, 138.50, 137.89, 129.82, 126.96, 53.81, 50.45, 48.14, 46.89, 45.85, 37.92, 37.15, 35.07, 34.07, 21.30.

The compound (+)-**I-4** is a specific form of the compound of general formula **I** in the present application, and is also one of (±)-**I-4**, which is an optically pure compound having a same relative configuration as (±)-**I-4** and having dextrorotatory optical rotation.

A method of determining an absolute configuration of the compound (+)-**I-4** is as follows: converting the (*S*)-(+)-mandelate of (+)-**22** having the same absolute configuration into lactam (+)-**22-LAC**, and determining the absolute configuration of (+)-**22-LAC** by using an X-ray single crystal diffraction method, wherein the absolute configuration is the same as that of (+)-**22**. The absolute configuration of (+)-**I-4** can be indirectly determined by the method.

A specific experimental method is as follows:

Synthesis of (+)-**22-LAC**: The above (*S*)-(+)-mandelate of (+)-**22** with the ee value of 98.34% (1.00 g, 2.4 mmol) was added into a saturated $NaHCO_3$ solution (100 mL) and stirred for 10 min, and then EtOAc (30 mL×3) was added for extraction. Organic phases were combined, washed with a brine, dried ($MgSO_4$), and filtered to remove the drying agent. A filtrate was evaporated to dryness on a rotary evaporator. A resulting residue was dissolved with toluene (7 mL) and heated to reflux overnight. TLC showed that a reaction was completed. The reaction system was cooled to room temperature, the solvent was removed by evaporation on a rotary evaporator, EtOAc/n-hexane (1/10 by v/v, 5 mL in total) was added into the residue for slurrying, and a crystal was collected by suction filtration and dried to obtain (+)-**22-LAC**, 0.40 g (88%), with a melting point of 178.7°C-180.5°C, $[\alpha]_D^{20}$=+30.2° (c=1.03, $CH_3OH$).

$^1H$ NMR ($CDCl_3$, 500 MHz) δ: 6.27-6.29 (m, 1H), 6.22-6.24 (m, 1H), 5.89 (brs, 1H), 3.40 (dd, 1H, *J* = 1.0 Hz and 9.5 Hz), 3.36 (d, 1H, *J* = 9.5 Hz), 2.97-2.99 (m, 1H), 2.81-2.83 (m, 1H), 2.69-2.74 (m, 1H), 2.54-2.57 (m, 1H), 2.13 (d, 1H, *J* = 17.0 Hz), 2.05 (d, 1H, *J* = 17.0 Hz), 1.91 (ddd, 1H, *J* = 1.0 Hz, 8.5 Hz and 12.5 Hz), 1.57-1.60 (m, 1H), 1.42 (dd, 1H, *J* = 0.8 Hz and 12.8 Hz), 1.08-1.10 (m, 1H).

$^{13}C$ NMR ($CDCl_3$, 126 MHz) δ: 178.22, 136.71, 136.25, 58.19, 52.34, 48.58, 45.59, 44.50, 40.90, 40.74, 37.63, 33.04.

ESI-HRMS: (*m*/*z*) calcd. for $C_{12}H_{16}NO$ ($[M+H]^+$) 190.1226, found: 190.1224.

Culture and X-ray diffraction of a (+)-**22-LAC** single crystal: 10 mg of the (+)-**22-LAC** sample was weighed and dissolved in $CH_2Cl_2$ (1 mL), then n-hexane (2 mL) was added and shaken evenly, filtration was performed, and a filtrate was put into a small glass triangular flask and slowly volatilized at room temperature for 2-3 days to obtain a single crystal

suitable for X-ray diffraction. The single crystal with a size of 0.3×0.09×0.08 mm was selected for diffraction with CuKα rays at a temperature of 100.00 (10) K on a Rigaku XtaLAB Pro single crystal diffractometer, CrysAlisPro 1.171.39.33c (Rigaku OD, 2017) was used for collecting diffraction data and performing data reduction, and an SHELXL program was used for performing structural analysis and refinement.

A chemical structure of the compound (+)-**22-LAC** in single crystal diffraction (ORTEP diagram) is shown in FIG. 2.

Crystal test and structural refinement relevant parameters of (+)-**22-LAC** are shown in the following table.

| | |
|---|---|
| CCDC deposition number | 2209124 |
| Radiation type | Cu Kα |
| Radiation wavelength/Å | λ = 1.54184 |
| Crystal size/$mm^3$ | 0.3 × 0.09 × 0.08 |
| 2 θ range for data collection/° | 7.198 to 148.460 |
| Empirical formula | $C_{12}H_{15}NO$ |
| Formula weight | 189.25 |
| Temperature/K | 100.00(10) |
| Crystal system | Monoclinic |
| Space group | C2 |
| a/Å | 13.3368(4) |
| b/Å | 5.9872(2) |
| c/Å | 13.5946(6) |
| α/° | 90 |
| β/° | 115.400(5) |
| γ/° | 90 |
| Volume/$Å^3$ | 980.61(7) |
| Z | 4 |
| $\rho_{calcd}g/cm^3$ | 1.282 |
| $\mu/mm^{-1}$ | 0.639 |
| F(000) | 408.0 |
| Index ranges | $-16 \leq h \leq 16$, $-7 \leq k \leq 6$ , $-16 \leq l \leq 16$ |
| Reflections collected | 12019 |
| Independent reflections | 1849 [$R_{int}$ = 0.0535, $R_{sigma}$ = 0.0697] |
| Data/restraints/parameters | 1849/1/127 |
| Goodness-of-fit on $F^2$ | 1.083 |
| Final R indexes [I≥2σ (I)] | $R_1$ = 0.0391, $wR_2$ = 0.1037 |
| Final R indexes [all data] | $R_1$ = 0.0424, $wR_2$ = 0.1063 |
| Largest diff. peak/hole/e $Å^{-3}$ | 0.17/-0.16 |
| Flack parameter | -0.2(2) |

**Example 9 Synthesis of compound (±)-I-7**

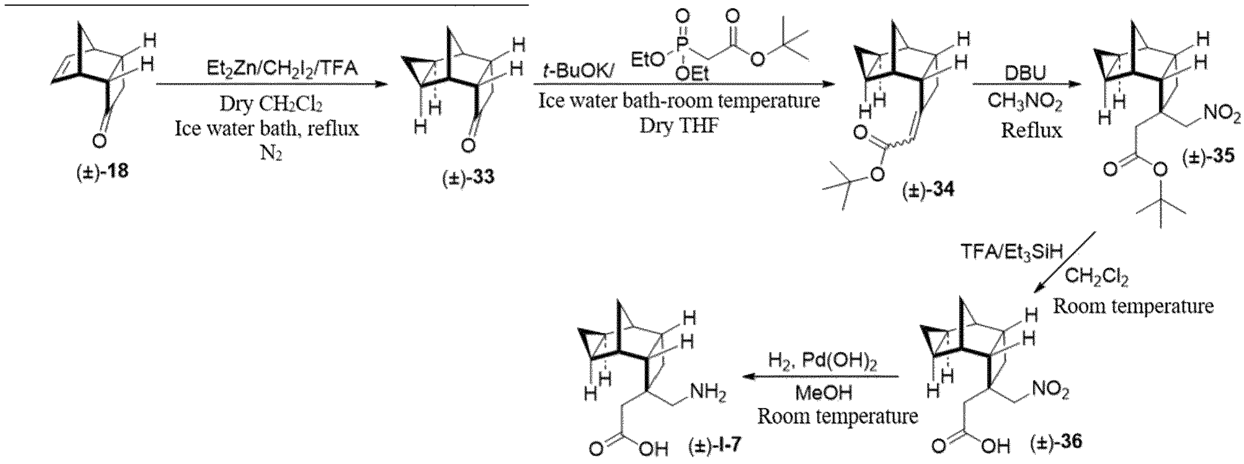


Step 1: Synthesis of compound (±)-**33**

Dry $CH_2Cl_2$ (200 mL) was added into a dry flask, and air in the reaction vessel was replaced with nitrogen (balloon) according to standard operations. Under cooling of an ice water bath, a dry $CH_2Cl_2$ (30 mL) solution of $Et_2Zn$ (1 M in n-hexane, 298 mL) and TFA (33.99 g, 0.30 mol) were sequentially and slowly added dropwise into the system to obtain a white slurry system. After the dropping was completed, stirring was performed continuously under the condition of the ice water bath for 0.5 h, and then a dry $CH_2Cl_2$ (80 mL) solution of $CH_2I_2$ (79.84 g, 0.30 mol) was added dropwise (during the dropping, a white viscous solid was gradually dissolved and clarified, and a white solid was precipitated into a suspension). After the dropping was completed, stirring was performed continuously under the condition of the ice water bath for 0.5 h, and then a dry $CH_2Cl_2$ (20 mL) solution of the compound (±)-**18** (10.00 g, 75 mmol) was added dropwise. After the dropping was completed, the reaction system was stirred at room temperature for 4-6 h and then heated to reflux overnight. TLC monitoring showed that a reaction was completed. The reaction was stopped, and the reaction system was cooled to room temperature, added into a saturated $NH_4Cl$ aqueous solution (300 mL×2), stirred, and extracted with $CH_2Cl_2$ (200 mL×2). Organic phases were combined, washed with a saturated brine (400 mL), and separated. The organic phases were combined, dried ($MgSO_4$), and filtered to remove the drying agent. A filtrate was concentrated on a rotary evaporator to obtain a brown oily substance, and then purified by a column chromatography [V(EtOAc)/V(n-hexane)=0/1→1/9] to obtain target compound (±)-**33**, a yellow oily substance (slightly impure), 3.15 g (29%).

$^1H$ NMR ($CDCl_3$, 500 MHz) δ: 3.48-3.52 (m, 1H), 2.95 (dt, 1H, *J* = 3.5 Hz and 18.5 Hz), 2.65-2.77 (m, 2H), 2.55-2.56 (m, 1H), 2.51-2.53 (m, 1H), 1.32-1.35 (m, 1H), 1.01-1.05 (m, 1H), 0.97-1.00 (m, 1H), 0.87-0.90 (m, 1H), 0.54-0.57 (m, 1H), 0.03-0.08 (m, 1H).

Step 2: Synthesis of compound (±)-**34**

Under $N_2$ atmosphere, *t*-BuOK (11.92 g, 0.11 mol) was added into dry THF (30 mL) and stirred into a suspension under an ice water bath, and then tert-butyl diethylphosphonoacetate (26.81 g, 0.11 mol) was added dropwise. After the dropping was completed, a reaction was carried out under the ice water bath for 1 h, and a newly formulated dry THF (10 mL) solution of the compound (±)-**33** (3.15 g, 21 mmol) was added dropwise. After the dropping was completed, stirring was performed overnight at room temperature. TLC monitoring showed that a reaction was completed. The reaction solution was poured into ice water (80 mL×2) and extracted with $CH_2Cl_2$ (60 mL×2). Organic phases were combined, washed with a saturated brine (150 mL) for one time, dried ($MgSO_4$), and filtered to remove the drying agent. A filtrate was concentrated under reduced pressure on a rotary evaporator to obtain a deep yellow oily substance, and then purified by a column chromatography [V(EtOAc)/V(n-hexane)=0/1→1/9] to obtain target product (±)-**34**, a light yellow oily substance 3.15 g. The product was directly used in a next reaction without characterization.

Step 3: Synthesis of compound (±)-**35**

At room temperature, the compound (±)-**34** (3.60 g, 15 mmol) was dissolved in $CH_3NO_2$ (120 mL) and stirred, and DBU (24.47 g, 0.16 mol) was added dropwise thereto. After the dropping was completed, under $N_2$ environment, the reaction mixture was refluxed for 48 h. TLC monitoring showed that a large amount of the raw materials were still not reacted. A reaction was stopped. The reaction solution was cooled to room temperature, poured into ice water, and extracted with $CH_2Cl_2$ (50 mL×2). Organic phases were combined, sequentially washed with ice water (100 mL) and a saturated brine (150 mL), dried ($MgSO_4$), and filtered to remove the drying agent. A filtrate was concentrated under

reduced pressure on a rotary evaporator to obtain a brownish black oily substance, and then purified by a column chromatography [V(EtOAc)/V(n-hexane)=0/1→1/9] to obtain target product (±)-**35**, a light yellow oily substance, 0.74 g (a combined yield of (±)-**33**-(±)-**35** was 10%).

$^1$H NMR ($CDCl_3$, 500 MHz) δ: 4.83 (dd, 1H, *J* = 1.3 Hz and 11.3 Hz), 4.55 (d, 1H, *J* = 11.0 Hz), 2.73 (s, 2H), 2.52-2.58 (m, 1H), 2.48-2.49 (m, 1H), 2.30-2.32 (m, 1H), 2.15-2.19 (m, 2H), 2.11 (ddd, 1H, *J* = 2.3 Hz, 8.5 Hz and 13.3 Hz), 1.48 (s, 9H), 1.23-1.27 (m, 1H), 1.13-1.17 (m, 2H), 0.58-0.60 (m, 1H), 0.43-0.46 (m, 1H), 0.06-0.10 (m, 1H).

$^{13}$C NMR ($CDCl_3$, 126 MHz) δ: 170.79, 83.55, 81.08, 49.08, 39.87, 39.61, 38.64, 37.19, 34.59, 29.94, 29.14, 28.26, 12.23, 11.14, 2.58.

ESI-HRMS: (*m*/*z*) calcd. for $C_{17}H_{26}NO_4$ ([M+H]$^+$) 308.1856, found: 308.1853.

Step 4: Synthesis of compound (±)-**36**

The compound (±)-**35** (0.28 g, 0.91 mmol) was dissolved in $CH_2Cl_2$ (5 mL), and $Et_3SiH$ (0.16 g, 1.4 mmol) and TFA (2 mL) were sequentially added dropwise under an ice water bath. After the dropping was completed, a reaction was carried out at room temperature for 4 h. TLC monitoring showed that the reaction was completed. The reaction mixture was poured into ice water (10 mL) and extracted with $CH_2Cl_2$ (10 mL×2). Organic phases were combined, washed with a saturated brine (20 mL×2), dried ($MgSO_4$), and filtered to remove the drying agent. A filtrate was concentrated on a rotary evaporator at a low temperature (25°C) to obtain a yellow oily substance. $CH_2Cl_2$ (10 mL× 3) was added into the oily substance for continuous concentration, drying was performed with a vacuum oil pump for 10 min until a white solid was precipitated out, and the solid was crushed and added into n-hexane (4 mL) for slurrying at room temperature for 1 h. The solid was collected by suction filtration and dried with a vacuum oil pump to obtain compound (±)-**36**, a white solid, 0.09 g (39%), with a melting point of 105.7°C-109.3°C.

$^1$H NMR (DMSO-$d_6$, 500 MHz) δ: 12.31 (brs, 1H), 4.78 (d, 1H, *J* = 12.0 Hz), 4.71 (d, 1H, *J* = 12.0 Hz), 2.74 (d, 1H, *J* = 17.5 Hz), 2.56 (d, 1H, *J* = 17.5 Hz), 2.52-2.55 (m, 1H), 2.36-2.37 (m, 1H), 2.21-2.23 (m, 1H), 2.12-2.17 (m, 2H), 2.00 (ddd, 1H, *J* = 2.0 Hz, 8.5 Hz and 13.0 Hz), 1.21-1.23 (m, 1H), 1.17-1.20 (m, 1H), 1.09-1.12 (m, 1H), 0.54-0.56 (m, 1H), 0.37-0.39 (m, 1H), -0.01-0.03 (m, 1H).

$^{13}$C NMR ($CDCl_3$, 126 MHz) δ: 176.63, 83.22, 48.89, 39.71, 38.20, 37.95, 37.13, 34.63, 29.81, 29.14, 12.42, 11.33, 2.66.

ESI-HRMS: (*m*/*z*) calcd. for $C_{13}H_{18}NO_4$ ([M+H]$^+$) 252.1230, found: 252.1228.

Step 5: Synthesis of compound (±)-**I-7**

The compound (±)-**36** (0.18 g, 0.72 mmol) was dissolved in $CH_3OH$ (4 mL), 10% $Pd(OH)_2$/C (0.06 g) was added thereto, air in a reaction vessel was replaced with hydrogen (balloon) according to standard operations, and stirring was performed overnight at room temperature. TLC monitoring showed that a reaction was completed (the reaction was generally completed within 12 h). Filtration was performed to remove a drying agent. A filtrate was concentrated under reduced pressure on a rotary evaporator to obtain a white solid, and $CH_3OH$ (1 mL)/EtOAc (3 mL) was added, and stirred and slurried at room temperature for 1-2 h. Filtration and drying were performed to obtain target product (±)-**I-7**, a white solid, 0.04 g (25%), with a melting point of 184.5°C-190.0°C.

$^1$H NMR ($CD_3OD$, 500 MHz) δ: 3.10 (dd, 1H, *J* = 1.5 Hz and 13.0 Hz), 2.96 (d, 1H, *J* = 13.0 Hz), 2.78 (dd, 1H, *J* = 1.3 Hz and 16.8 Hz), 2.60 (d, 1H, *J* = 16.5 Hz), 2.51-2.55 (m, 1H), 2.48-2.49 (m, 1H), 2.23-2.27 (m, 2H), 2.18 (dd, 1H, *J* = 6.8 Hz and 12.8 Hz), 1.69 (ddd, 1H, *J* = 2.0 Hz, 8.5 Hz and 13.0 Hz), 1.50-1.53 (m, 1H), 1.14-1.20 (m, 2H), 0.56-0.58 (m, 1H), 0.45-0.48 (m, 1H), 0.03-0.07 (m, 1H).

$^{13}$C NMR ($D_2O$, 126 MHz) δ: 179.55, 49.53, 46.11, 44.30, 37.56, 35.01, 34.80, 32.58, 29.65, 26.94, 10.37, 9.02, 0.01.

ESI-HRMS: (*m*/*z*) calcd. for $C_{13}H_{20}NO_2$ ([M+H]$^+$) 222.1489, found: 222.1487.

The compound (±)-**I-7** is a specific form of the compound of general formula I in the present application.

**Example 10 Synthesis of compound** (+)-**I-8**

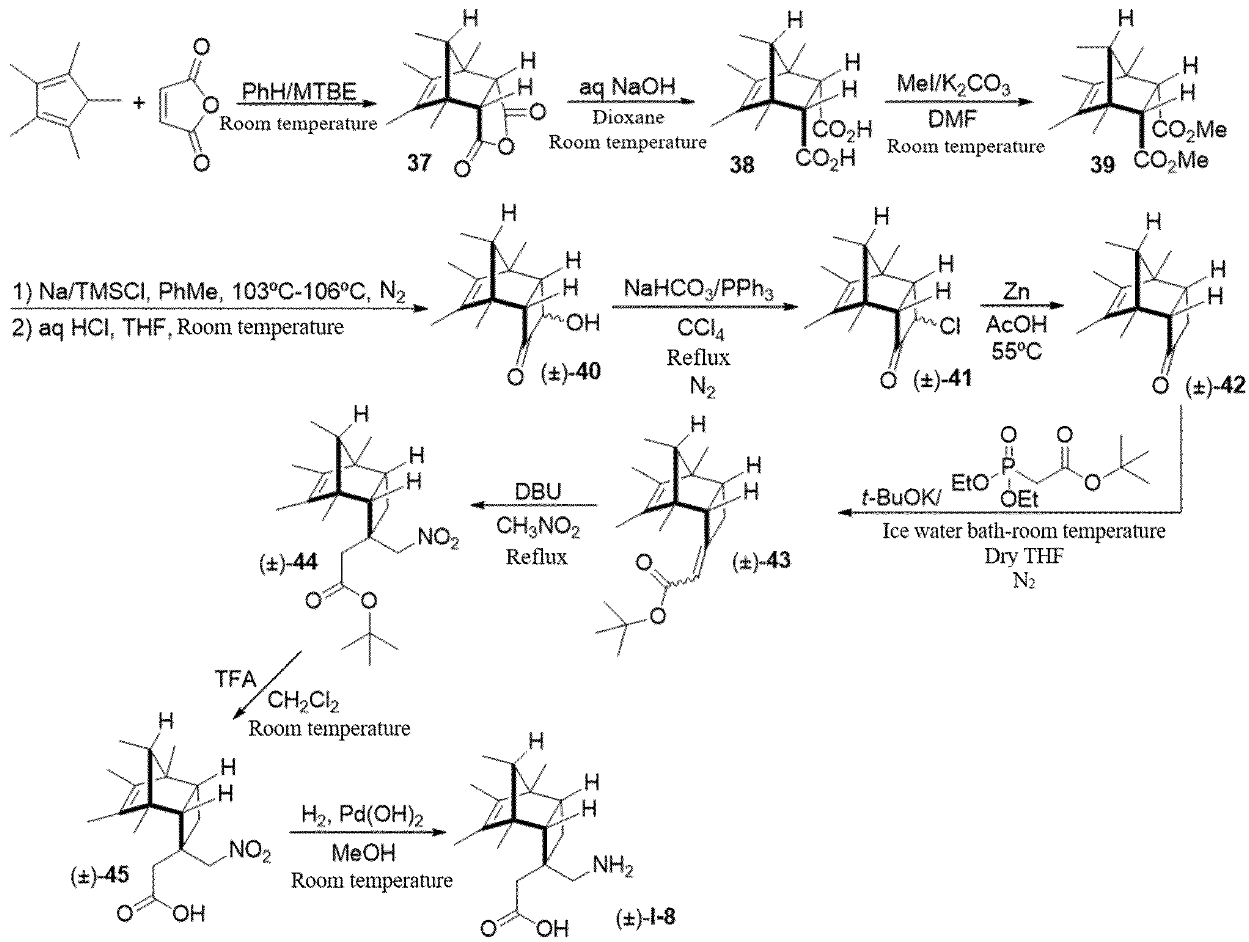


Step 1: Synthesis of compound **37**

Maleic anhydride (27.79 g, 0.28 mol) was dissolved in a mixed solvent of benzene (50 mL) and methyl tert-butyl ether (150 mL), air in a reaction vessel was replaced with nitrogen (balloon) according to standard operations, and a benzene/methyl tert-butyl ether (1/3, v/v, 50 mL in total) solution of 1,2,3,4,5-pentamethylcyclopentadiene (20.00 g, 0.15 mol) was added dropwise under an ice water bath. After the dropping was completed, stirring was performed overnight at room temperature. A reaction was stopped. The reaction solution was concentrated under reduced pressure on a rotary evaporator to obtain a purple-red oily substance. The oily substance was placed in an ice water bath for crystallization at a low temperature. After a crystal was precipitated out, n-hexane (100 mL) was added, stirred and slurried at room temperature for 1 h. The solid was collected by suction filtration, and a filter cake was dried with a vacuum oil pump to obtain a crude product of compound **37,** a white solid (containing a certain amount of impurities), 40.00 g (>100% because the solid contained a certain amount of unreacted maleic anhydride) was obtained. The product was directly used in a next step without continuous purification.

$^1H$ NMR ($CDCl_3$, 500 MHz) $\delta$: 3.18 (s, 2H), 1.55-1.59 (m, 7H), 1.35 (s, 6H), 0.62 (d, 3H, *J* = 6.5 Hz).

Step 2: Synthesis of compound **38**

The compound **37** (40.00 g, calculated as 0.17 mol) was dissolved in 1,4-dioxane (400 mL), a 50% NaOH aqueous solution (68 mL) was slowly added dropwise under an ice bath, and a white solid was gradually precipitated out during the dropping. After the dropping was completed, 1,4-dioxane (300 mL) was added into the system and stirred at room temperature for 1 h. TLC monitoring showed that a reaction was completed. Under cooling of the ice bath, 1 M HCl was slowly added dropwise into the reaction system until pH being <2, and the reaction system was saturated with sodium chloride and extracted with EtOAc (300 mL$\times$3). Organic phases were combined, dried ($MgSO_4$), and filtered to remove the drying agent. A filtrate was concentrated on a rotary evaporator to obtain a residue. n-hexane (100 mL) was added into the residue, stirred and slurried at room temperature for 1 h. A solid was collected by suction filtration and dried with a vacuum oil pump to obtain compound **38,** a white solid, 20.86 g (a combined yield of 1,2,3,4,5-pentamethylcyclopentadiene$\rightarrow$**38** was 56%). The product was directly used in a next reaction without further purification.

$^1$H NMR (DMSO-$d_6$, 500 MHz) δ: 2.90 (s, 2H), 1.54 (s, 6H), 1.37 (q, 1H, *J* = 6.3 Hz), 1.08 (s, 6H), 0.52 (d, 3H, *J* = 6.5 Hz).

Step 3: Synthesis of compound **39**

The compound **38** (20.86 g, 83 mmol) was dissolved in N,N-dimethylformamide (DMF) (210 mL), and air in a reaction vessel was replaced with nitrogen (balloon) according to standard operations. $K_2CO_3$ (34.28 g, 0.25 mol) was added under cooling of an ice water bath, and then $CH_3I$ (46.94 g, 0.33 mol) was slowly added dropwise. After the dropping was completed, a reaction was carried out at room temperature for 5-6 h. TLC monitoring showed that the reaction was completed. EtOAc (200 mL) was added into the reaction solution, filtration was performed to remove a solid, and a filtrate was washed with water (300 mL×5). All aqueous phases were combined and extracted reversely with EtOAc (200 mL×2). All organic phases were combined, washed with a saturated brine (500 mL), dried ($MgSO_4$), and filtered to remove the drying agent. A filtrate was concentrated on a rotation evaporator to obtain a yellow oily substance, and then purified by a column chromatography [V(EtOAc)/V(n-hexane)=0/1→1/9] to obtain target product **39,** a colorless transparent oily substance, 22.10 g (95%, containing certain impurities). The product was directly used in a next step without further purification.

$^1$H NMR ($CDCl_3$, 500 MHz) δ: 3.57 (s, 6H), 3.00 (s, 2H), 1.60 (s, 6H), 1.37 (q, 1H, *J* = 6.3 Hz), 1.15 (s, 6H), 0.60 (d, 3H, *J* = 6.5 Hz).

Step 4: Synthesis of compound (±)-**40**

Metallic sodium (9.97 g, 0.43 mol) was added into dry toluene (220 mL) and heated under $N_2$ environment until the sodium was completely melted, stirring was initiated, and continuous stirring was performed at an internal temperature of the reaction system maintained at 103°C-106°C for 20 min. A dry toluene (15 mL) solution of the compound **39** (22.10 g, 79 mmol) and TMSCl (45.39 g, 0.42 mol) was added dropwise. The system released heat during the dropping, and the internal temperature of the reaction system was maintained at 103°C-106°C by controlling a dropping speed. After the dropping was completed, the internal temperature of the reaction system was maintained at 103°C-106°C, and stirring was performed overnight. TLC monitoring showed that a reaction was completed. The reaction solution was cooled to room temperature. Filtration was performed with the assistance of diatomite. A filtrate was concentrated under reduced pressure on a rotary evaporator to obtain a brown oily substance. The oily substance was dissolved in THF (100 mL), and 1 M HCl (15 mL) was added dropwise under an ice water bath. After the dropping was completed, a reaction was carried out at room temperature for 30 min. TLC monitoring showed that the reaction was completed. The reaction solution was poured into water (100 mL) and extracted with EtOAc (100 mL×2). Organic phases were combined, washed with a saturated brine (300 mL), dried ($MgSO_4$), and filtered to remove the drying agent. A filtrate was concentrated under reduced pressure on a rotary evaporator to obtain a brown oily substance, and the oily substance was solidified at a low temperature. n-hexane (10 mL) was added into the solid, stirred and slurried at room temperature for 1 h. The solid was collected by filtration and dried with a vacuum oil pump to obtain target compound (±)-**40**, a white solid, 3.60 g (21%), with a melting point of 124.0°C-128.0°C.

$^1$H NMR ($CDCl_3$, 500 MHz) δ: 4.44 (dd, 1H, *J* = 3.5 Hz and 9.0 Hz), 3.16 (dd, 1H, *J* = 3.5 Hz and 7.5 Hz), 2.91 (dd, 1H, *J* = 7.5 Hz and 9.0 Hz), 1.640-1.643 (m, 3H), 1.58-1.62 (m, 2H), 1.499-1.504 (m, 3H), 1.22 (s, 3H), 1.13 (s, 3H), 0.57 (d, 3H, *J* = 6.5 Hz).

Step 5: Synthesis of compound (±)-**41**

The compound (±)-**40** (2.61 g, 12 mmol) was dissolved in $CCl_4$ (20 mL), and triphenylphosphine (3.41 g, 13 mmol) and $NaHCO_3$ (0.04 g, 0.48 mmol) were sequentially added in a stirring state. Air in a reaction vessel was replaced with nitrogen (balloon) according to standard operations, and refluxing was performed overnight under stirring. TLC monitoring showed that a reaction was completed. The reaction solution was cooled to room temperature and filtered. A filtrate was concentrated under reduced pressure on a rotary evaporator, and methyl tert-butyl ether (12 mL) was added into the concentrated residue and stirred at room temperature for 1 h. The solid was removed by filtration. The filtrate was concentrated under reduced pressure on a rotary evaporator to obtain a brownish black oily substance, and then purified by a column chromatography [V(EtOAc)/V(n-hexane)=0/1→9/91] to obtain target product (±)-**41**, a light yellow oily substance (slightly impure), 2.20 g. The product was directly used in a next reaction without characterization.

Step 6: Synthesis of compound (±)-**42**

Zinc powder (2.06 g, 32 mmol) and glacial acetic acid (10 mL) were stirred and mixed, and a newly formulated glacial acetic acid (5 mL) solution of the compound (±)-**41** (2.20 g, 9.2 mmol) was added dropwise at room temperature.

After the dropping was completed, under $N_2$ environment, the reaction mixture was stirred overnight in an oil bath at 55°C. TLC monitoring showed that a reaction was completed. The reaction mixture was cooled to room temperature and filtered. A filtrate was diluted with ice water (50 mL) and extracted with $CH_2Cl_2$ (40 mL×2). Organic phases were combined, sequentially washed with water (80 mL×3) and a saturated brine (100 mL), dried ($MgSO_4$), and filtered to remove the drying agent. The filtrate was concentrated under reduced pressure on a rotary evaporator to obtain a yellow oily substance, and then purified by a column chromatography [V(EtOAc)/V(n-hexane)=0/1→1/20] to obtain a light yellow oily substance. The oily substance was dried with a vacuum oil pump to obtain a solid. n-hexane (5 mL) was added into the solid and stirred at room temperature for 1 h. Filtration was performed. A filter cake was dried with a vacuum oil pump to obtain target compound (±)-**42**, a white solid, 1.08 g (a combined yield of (±)-**40**→(±)-**42** was 45%), with a melting point of 101.0°C-103.0°C.

$^1H$ NMR ($CDCl_3$, 500 MHz) δ: 3.34-3.37 (m, 1H), 2.62 (ddd, 1H, *J* = 3.0 Hz, 8.5Hz and 18.5 Hz), 2.49-2.52 (m, 1H), 2.14 (dt, 1H, *J* = 3.5 Hz and 18.5 Hz), 1.616-1.624 (m, 3H), 1.51-1.52 (m, 3H), 1.48 (q, 1H, *J* = 6.5 Hz), 1.14 (s, 3H), 1.13 (s, 3H), 0.58 (d, 3H, *J* = 6.5 Hz).

$^{13}C$ NMR ($CDCl_3$, 126 MHz) δ: 211.60, 134.45, 133.12, 73.07, 66.04, 56.64, 55.52, 45.28, 36.90, 15.97, 15.29, 12.49, 11.11, 8.19.

ESI-HRMS: (*m*/*z*) calcd. for $C_{14}H_{21}O$ ($[M+H]^+$) 205.1587, found: 205.1587.

Step 7: Synthesis of compound (±)-**43**

Under $N_2$ environment, *t*-BuOK (2.35 g, 21 mmol) was added into dry THF (10 mL) and stirred into a suspension under an ice water bath, and then tert-butyl diethylphosphonoacetate (5.28 g, 21 mmol) was added dropwise. After the dropping was completed, a reaction was carried out under the ice water bath for 30 min, and a newly formulated dry THF (7 mL) solution of the compound (±)-**42** (0.86 g, 4.2 mmol) was added dropwise. After the dropping was completed, stirring was performed overnight at room temperature. TLC monitoring showed that the reaction was completed. The reaction solution was poured into ice water (80 mL) and extracted with $CH_2Cl_2$ (30 mL×3). Organic phases were combined, sequentially washed with 1 M HCl (20 mL) and a saturated brine (100 mL), dried ($MgSO_4$), and filtered to remove the drying agent. A filtrate was concentrated under reduced pressure on a rotary evaporator to obtain a deep yellow oily substance, and then purified by a column chromatography [V(EtOAc)/V(n-hexane)=0/1→1/20] to obtain target product (±)-**43,** a light yellow oily substance, 0.97 g. The product was directly used in a next reaction without characterization.

Step 8: Synthesis of compound (±)-**44**

At room temperature, the compound (±)-**43** (0.97 g, 3.2 mmol) was dissolved in $CH_3NO_2$ (12 mL), and DBU (4.40 g, 29 mmol) was added dropwise. After the dropping was completed, under $N_2$ environment, the reaction mixture was refluxed for 48 h. TLC monitoring showed that about 60% of the raw materials were not reacted. A reaction was stopped. The reaction solution was cooled to room temperature, poured into ice water (100 mL), and extracted with $CH_2Cl_2$ (50 mL×2). Organic phases were combined, sequentially washed with 1 M HCl (50 mL) and a saturated brine (100 mL), dried ($MgSO_4$), and filtered to remove the drying agent. A filtrate was concentrated under reduced pressure on a rotary evaporator to obtain a brownish black oily substance, and then purified by a column chromatography [V(EtOAc)/V(n-hexane)=0/1→1/9] to obtain a light yellow oily substance. The oily substance was solidified at a low temperature, n-hexane (2 mL) was added, stirred and slurried at -18°C for 30 min, and a solid was collected by suction filtration and dried with a vacuum oil pump to obtain target product (±)-**44**, a white solid, 0.10 g (a combined yield of (+)**-42**-(%)-**44** was 7%), with a melting point of 78.6°C-81.5°C.

$^1H$ NMR ($CDCl_3$, 500 MHz) δ: 4.82 (d, 1H, *J* = 11.5 Hz), 4.66 (d, 1H, *J* = 11.5 Hz), 2.47-2.52 (m, 1H), 2.36 (d, 1H, *J* = 17.5 Hz), 2.28 (d, 1H, *J* = 17.5 Hz), 2.24-2.26 (m, 1H), 1.91 (ddd, 1H, *J* = 2.0 Hz, 8.5 Hz and 13.0 Hz), 1.73-1.74 (m, 3H), 1.605-1.612 (m, 3H), 1.44 (s, 9H), 1.17-1.24 (m, 2H), 1.00 (s, 3H), 0.97 (s, 3H), 0.55 (d, 3H, *J* = 6.0 Hz).

$^{13}C$ NMR ($CDCl_3$, 126 MHz) δ: 171.17, 137.15, 134.49, 82.91, 80.86, 66.54, 56.45, 55.96, 55.78, 42.49, 39.08, 36.58, 31.59, 28.25, 16.22, 15.25, 13.26, 12.84, 8.12.

ESI-HRMS: (*m*/*z*) calcd. for $C_{21}H_{32}NO_4$ ($[M-H]^-$) 362.2337, found: 362.2343.

Step 9: Synthesis of compound (±)-**45**

The compound (±)-**44** (0.10 g, 0.28 mmol) was dissolved in $CH_2Cl_2$ (5 mL), and TFA (1 mL) was slowly added dropwise under an ice water bath. After the dropping was completed, a reaction was carried out at room temperature for 2-3 h. TLC monitoring showed that the reaction was completed. The reaction solution was concentrated under reduced pressure on a rotary evaporator to obtain a brown oily substance, and then purified by a column chromatography [V(EtOAc)/V(n-hexane)=0/1→3/7] to obtain a light yellow solid. n-hexane (3 mL) was added to the solid, stirred and slurried at room temperature for 1 h. The solid was collected by suction filtration and dried with a vacuum oil pump to obtain

compound (±)-**45**, a white solid, 0.05 g (63%), with a melting point of 122.8°C-126.4°C.

$^1$H NMR ($CDCl_3$, 500 MHz) δ: 4.78 (d, 1H, $J$ = 12.0 Hz), 4.74 (d, 1H, $J$ = 11.5 Hz), 2.55 (d, 1H, $J$ = 18.0 Hz), 2.50-2.54 (m, 1H), 2.51 (d, 1H, $J$ = 18.0 Hz), 2.26-2.28 (m, 1H), 1.91 (ddd, 1H, $J$ = 2.0 Hz, 8.5 Hz and 13.5 Hz), 1.75-1.76 (m, 3H), 1.61-1.62 (m, 3H), 1.22-1.26 (m, 2H), 0.99 (s, 3H), 0.98 (s, 3H), 0.56 (d, 3H, $J$ = 6.5 Hz);
$^{13}$C NMR ($CDCl_3$, 126 MHz) δ: 176.32, 137.65, 134.32, 82.69, 66.66, 56.44, 56.10, 55.55, 42.44, 38.57, 34.98, 31.74, 16.34, 15.23, 13.31, 12.88, 8.11;
ESI-HRMS: (*m/z*) calcd. for $C_{17}H_{26}NO_4$ ([M+H]$^+$) 308.1856, found: 308.1850.

Step 10: Synthesis of compound (±)-**I-8**

The compound (±)-**45** (0.05 g, 0.16 mmol) was dissolved in $CH_3OH$ (3 mL), 10% $Pd(OH)_2/C$ (0.03 g) was added, air in a reaction vessel was replaced with hydrogen (balloon) according to standard operations, and stirring was performed overnight at room temperature. TLC monitoring showed that a reaction was completed (the reaction was generally completed within 12 h). Filtration was performed. A filtrate was concentrated under reduced pressure on a rotary evaporator to obtain a white solid, and $CH_3OH$ (1 mL)/EtOAc (3 mL) was added and stirred at room temperature for 0.5 h. The solid was collected by suction filtration and dried with a vacuum oil pump to obtain target product (+)-**I-8**, a white solid, 0.02 g (53%), with a melting point of 180.0°C-184.0°C.

$^1$H NMR ($CD_3OD$, 500 MHz) δ: 3.09 (dd, 1H, $J$ = 0.8 Hz and 13.3 Hz), 2.97 (d, 1H, $J$ = 13.0 Hz), 2.48-2.53 (m, 1H), 2.43 (dd, 1H, $J$ = 1.3 Hz and 17.3 Hz), 2.27 (d, 1H, $J$ = 17.0 Hz), 2.20-2.22 (m, 1H), 1.81-1.82 (m, 3H), 1.61-1.62 (m, 3H), 1.59 (ddd, 1H, $J$ = 2.0 Hz, 8.0 Hz and 12.5 Hz), 1.24 (q, 1H, $J$ = 6.3 Hz), 1.14 (dd, 1H, $J$ = 6.5 Hz and 12.5 Hz), 1.08 (s, 3H), 0.99 (s, 3H), 0.58 (d, 3H, $J$ = 6.5 Hz);
$^{13}$C NMR ($CD_3OD$, 126 MHz) δ: 180.23, 137.15, 136.15, 67.72, 57.44, 56.85, 55.52, 51.85, 45.39, 43.50, 38.85, 34.01, 16.90, 15.51, 13.16, 12.83, 8.44;
ESI-HRMS: (*m/z*) calcd. for $C_{17}H_{28}NO_2$ ([M+H]$^+$) 278.2115, found: 278.2111.

The compound (±)-**I-8** is a specific form of the compound of general formula **I** in the present application.

## Example 11 Synthesis of compound (±)-I-9 and a p-toluenesulfonate thereof

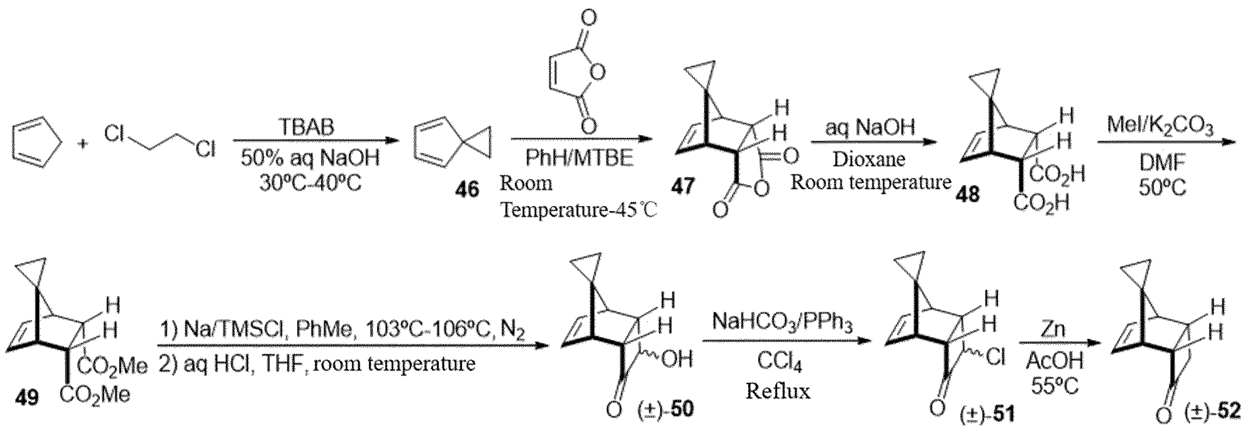

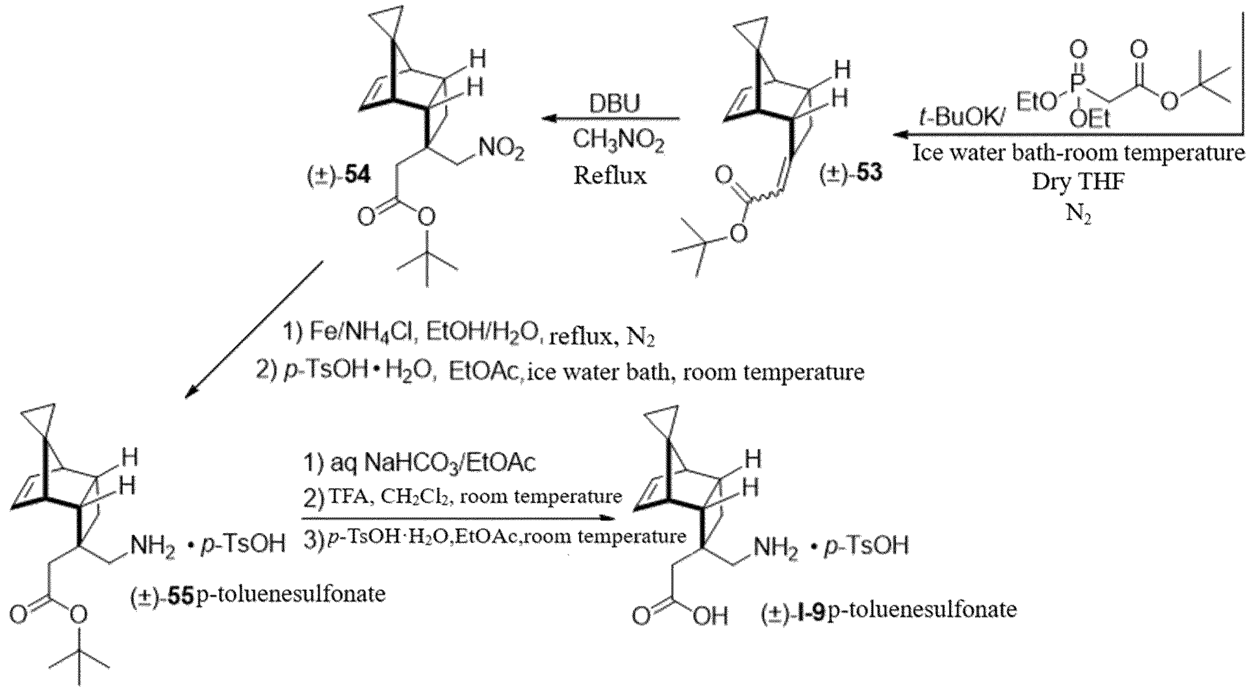


Step 1: Synthesis of compound **46**

Tetrabutylammonium bromide (TBAB) (32.64 g, 0.10 mol) was added into a 50% NaOH aqueous solution (1.2 L) and stirred, and air in a reaction vessel was replaced with nitrogen (balloon) according to standard operations. Under an ice water bath, the newly distilled cyclopentadiene (167.13 g, 2.5 mol) and 1,2-dichloroethane (250.50 g, 2.5 mol) were mixed evenly and then added dropwise into the reaction system (heat was released in a reaction, the internal temperature was maintained at 30°C-40°C by controlling the dropping speed, the color of the system was gradually changed from colorless transparent to dark red-brown during the dropping, and the system became viscous). After the dropping was completed, the reaction device was transferred to an oil bath to maintain the internal temperature of the system at 30°C-40°C, and stirring was performed for 2 h. The reaction was stopped. The reaction solution was cooled to room temperature, poured into ice water (1.0 mL), and extracted with n-pentane (300 mL×2). Organic phases were combined, sequentially washed with water (500×2), 1 M HCl (300 mL) and a saturated brine (500 mL), dried ($MgSO_4$), and filtered to remove the drying agent. A filtrate was fractionated under atmospheric pressure, and a fraction at 108°C-109°C was collected to obtain target compound **46** (containing a certain amount of n-pentane), colorless transparent liquid, 46.24 g (20%).

$^1$H NMR ($CDCl_3$, 500 MHz) δ: 6.50-6.52 (m, 2H), 6.11-6.13 (m, 2H), 1.65 (s, 4H).

Step 2: Synthesis of compound **47**

Maleic anhydride (11.28 g, 0.12 mol) was dissolved in a mixed solvent of benzene (30 mL) and methyl tert-butyl ether (90 mL), air in a reaction vessel was replaced with nitrogen (balloon) according to standard operations, and a benzene/methyl tert-butyl ether (1/3, v/v, 50 mL in total) solution of the compound **46** (13.24 g, 0.14 mol) was added dropwise under an ice water bath. After the dropping was completed, stirring was performed overnight at room temperature, and the system was placed in an oil bath to reflux and react at 45°C for 2 h. The reaction was stopped. The reaction system was cooled to room temperature and then concentrated under reduced pressure on a rotary evaporator to obtain a colorless transparent oily substance. n-hexane (100 mL×4) was added thereto, and concentration was performed for several times to obtain a white solid. n-hexane (120 mL) was added to the solid, stirred and slurried at room temperature for 1 h. The solid was collected by suction filtration and dried with a vacuum oil pump to obtain compound **47,** a white solid, 21.03 g (77%), with a melting point of 92.7°C-96.9°C.

$^1$H NMR ($CDCl_3$, 500 MHz) δ: 6.386-6.394 (m, 2H), 3.70-3.71 (m, 2H), 2.88-2.90 (m, 2H), 0.64-0.68 (m, 2H), 0.51-0.54 (m, 2H);
$^{13}$C NMR ($CDCl_3$, 126 MHz) δ: 171.25, 135.62, 51.11, 49.12, 47.54, 8.26, 7.12;
ESI-HRMS: (*m*/*z*) calcd. for $C_{11}H_{11}O_3$ ($[M+H]^+$) 191.0703, found: 191.0701.

Step 3: Synthesis of compound **48**

The compound **47** (21.00 g, 0.11 mol) was dissolved in 1,4-dioxane (500 mL) and stirred under cooling of an ice bath, a 50% NaOH aqueous solution (44 mL) was slowly added dropwise, and a white solid was gradually precipitated out during the dropping. After the dropping was completed, stirring was performed continuously at room temperature for 0.5-1 h. TLC monitoring showed that a reaction was completed. Under the ice bath, 1 M HCl was added dropwise to adjust the pH of the reaction solution being <2, and the reaction system was saturated with sodium chloride and extracted with EtOAc (400 mL×3). Organic phases were combined, dried ($MgSO_4$), and subjected to suction filtration to remove the drying agent. A filtrate was concentrated on a rotary evaporator to obtain a residue. n-hexane (120 mL) was added into the residue, stirred and slurried at room temperature for 1 h. A solid was collected by suction filtration and dried with a vacuum oil pump to obtain compound **48,** a white solid, 22.33 g (97%), with a melting point of 163.3°C-166.3°C.

$^1$H NMR ($CDCl_3$, 500 MHz) δ: 6.345-6.352 (m, 2H), 3.50-3.51 (m, 2H), 2.53-2.55 (m, 2H), 0.56-0.59 (m, 2H), 0.42-0.45 (m, 2H);
$^{13}$C NMR ($CDCl_3$, 126 MHz) δ: 179.21, 135.10, 51.32, 49.66, 44.86, 7.91, 6.55;
ESI-HRMS: (*m*/*z*) calcd. for $C_{11}H_{13}O_4$ ([M+H]$^+$) 209.0808, found: 209.0805.

Step 4: Synthesis of compound **49**

The compound **48** (42.34 g, 0.20 mol) was dissolved in DMF (500 mL), and air in a reaction vessel was replaced with nitrogen (balloon) according to standard operations. Dry $K_2CO_3$ (84.32 g, 0.61 mol) was added under an ice water bath, and then $CH_3I$ (115.44 g, 0.81 mol) was slowly added dropwise. After the dropping was completed, the reaction device was transferred to an oil bath and stirred overnight at 55°C. TLC monitoring showed that a reaction was completed. The reaction system was cooled to room temperature. EtOAc (500 mL) was added into the reaction system and stirred, a solid was removed by suction filtration, and a filtrate was washed with water (600 mL×5). All aqueous phases were combined and extracted reversely with EtOAc (300 mL×2). Then, all organic phases were combined, washed with a saturated brine (1.0 L), dried ($MgSO_4$), and subjected to suction filtration to remove the drying agent. A filtrate was concentrated on a rotary evaporator to obtain a brownish black oily substance, and then purified by a column chromatography [V(EtOAc)/V(n-hexane)=0/1→1/10] to obtain a white solid. n-hexane (150 mL) was added to the solid, stirred and slurried at room temperature for 1 h. The solid was collected by suction filtration and dried with a vacuum oil pump to obtain target compound **49,** a white solid, 40.09 g (83%), with a melting point of 71.4°C-74.2°C.

$^1$H NMR ($CDCl_3$, 500 MHz) δ: 6.34-6.35 (m, 2H), 3.62 (s, 6H), 3.45 (m, 2H), 2.52-2.53 (m, 2H), 0.54-0.57 (m, 2H), 0.41-0.44 (m, 2H);
$^{13}$C NMR ($CDCl_3$, 126 MHz) δ: 172.88, 135.07, 51.67, 51.37, 49.01, 44.46, 7.86, 6.51;
ESI-HRMS: (*m*/*z*) calcd. for $C_{13}H_{17}O_4$ ([M+H]$^+$) 237.1121, found: 237.1118.

Step 5: Synthesis of compound (±)-**50**

Metallic sodium (21.41 g, 0.93 mol) was added into dry toluene (300 mL) and heated under $N_2$ environment until the sodium was completely melted, stirring was initiated, and continuous stirring was performed at an internal temperature maintained at 103°C-106°C for 20 min. A dry toluene (200 mL) solution of the compound **49** (40.00 g, 0.17 mol) and TMSCl (97.48 g, 0.90 mol) was added dropwise. The system released heat during the dropping, and the internal temperature of the reaction system was maintained at 103°C-106°C by controlling a dropping speed. After the dropping was completed, the internal temperature of the reaction system was maintained at 103°C-106°C, and stirring was performed continuously for 3-4 h. TLC monitoring showed that a reaction was completed. The reaction solution was cooled to room temperature. Filtration was performed with the assistance of diatomite. A filtrate was concentrated under reduced pressure on a rotary evaporator to obtain a dark red-brown oily substance. The oily substance was dissolved in THF (300 mL), and 1 M HCl (20 mL) was added dropwise under an ice water bath. After the dropping was completed, a reaction was carried out at room temperature for 1 h. TLC monitoring showed that the reaction was completed. The reaction solution was poured into water (300 mL) and extracted with EtOAc (300 mL×2). Organic phases were combined, washed with a saturated brine (500 mL), dried ($MgSO_4$), and subjected to suction filtration to remove the drying agent. A filtrate was concentrated under reduced pressure on a rotary evaporator to obtain a residue. A mixed solvent of EtOAc (15 mL) and n-hexane (90 mL) was added into the residue, continuously stirred and slurried at room temperature for 1 h. A solid was collected by suction filtration and dried with a vacuum oil pump to obtain target compound (±)-**50**, a brown solid, 20.34 g (68%). The product was directly used in a next reaction without characterization.

Step 6: Synthesis of compound (±)-**51**

The compound (±)-**50** (20.34 g, 0.12 mol) was dissolved in $CCl_4$ (150 mL) and stirred, and triphenylphosphine (34.51 g, 0.13 mol) and $NaHCO_3$ (1.36 g, 16 mmol) were sequentially added. Air in a reaction vessel was replaced with nitrogen (balloon) according to standard operations, and then refluxing was performed overnight. TLC monitoring showed that a reaction was completed. The reaction solution was cooled to room temperature and subjected to suction filtration to remove a solid. A filtrate was concentrated under reduced pressure on a rotary evaporator, and a mixed solvent of EtOAc (30 mL) and n-hexane (60 mL) was added into the concentrated residue and continuously stirred at room temperature for 1 h. The solid was removed by suction filtration. The filtrate was concentrated under reduced pressure on a rotary evaporator to obtain a brownish black oily substance, and then purified by a column chromatography [V(EtOAc)/V(n-hexane)=0/1→3/97] to obtain target product (±)-**51**, a light yellow oily substance (slightly impure), 7.36 g. The product was directly used in a next reaction without characterization.

Step 7: Synthesis of compound (±)-**52**

Zinc powder (8.50 g, 0.13 mol) and glacial acetic acid (40 mL) were stirred and mixed, and a newly formulated glacial acetic acid (30 mL) solution of the compound (±)-**51** (7.36 g, 38 mmol) was added dropwise at room temperature. After the dropping was completed, under $N_2$ environment, the reaction mixture was placed in an oil bath and stirred overnight at 55°C. TLC monitoring showed that a reaction was completed. The reaction mixture was cooled to room temperature, poured into ice water (100 mL), stirred, and subjected to suction filtration to remove a solid. A filtrate was extracted with EtOAc (80 mL×3). Organic phases were combined, sequentially washed with water (200 mL×3), a saturated $NaHCO_3$ solution (200 mL) and a saturated brine (200 mL), dried ($MgSO_4$), and subjected to suction filtration to remove the drying agent. A filtrate was concentrated under reduced pressure on a rotary evaporator to obtain a yellow oily substance, and then purified by a column chromatography [V(EtOAc)/V(n-hexane)=0/1→3/97] to obtain target product (±)-**52**, a light yellow oily substance, 4.87 g (a combined yield of ±)-**50**→(±)-**52** was 26%).

$^1$H NMR ($CDCl_3$, 500 MHz) δ: 6.23-6.28 (m, 2H), 3.84-3.88 (m, 1H), 2.92-2.97 (m, 1H), 2.74 (ddd, 1H, *J* = 3.0 Hz, 8.5 Hz and 18.5 Hz), 2.50-2.52 (m, 1H), 2.42-2.45 (m, 1H), 2.22 (dt, 1H, *J* = 3.8 Hz and 18.5 Hz), 0.50-0.56 (m, 2H), 0.42-0.46 (m, 1H), 0.35-0.39 (m, 1H);
$^{13}$C NMR ($CDCl_3$, 126 MHz) δ: 211.22, 135.61, 132.91, 66.90, 51.52, 50.64, 49.45, 46.10, 27.03, 7.70, 5.60; ESI-HRMS: (*m*/*z*) calcd. for $C_{11}H_{13}O$ ($[M+H]^+$) 161.0961, found: 161.0960.

Step 8: Synthesis of compound (±)-**53**

Under $N_2$ environment, t-BuOK (3.71 g, 33 mmol) was added into dry THF (6 mL) and stirred into a suspension under an ice water bath, and then tert-butyl diethylphosphonoacetate (8.35 g, 33 mmol) was added dropwise. After the dropping was completed, a reaction was carried out continuously under the ice water bath for 1 h. A newly formulated dry THF (4 mL) solution of the compound (±)-**52** (1.06 g, 6.6 mmol) was added dropwise. After the dropping was completed, stirring was performed overnight at room temperature. TLC monitoring showed that a reaction was completed. The reaction solution was poured into ice water (50 mL) and extracted with $CH_2Cl_2$ (40 mL×2). Organic phases were combined, sequentially washed with ice water (80 mL×2) and a saturated brine (150 mL), dried ($MgSO_4$), and subjected to suction filtration to remove the drying agent. A filtrate was concentrated under reduced pressure on a rotary evaporator to obtain a deep yellow oily substance, and then purified by a column chromatography [V(EtOAc)/V(n-hexane)=0/1→3/97] to obtain target product (±)-**53**, a light yellow oily substance, 1.62 g. The product was directly used in a next reaction without characterization.

Step 9: Synthesis of compound (±)-**54**

At room temperature, the compound (±)-**53** (1.62 g, 6.3 mmol) was dissolved in $CH_3NO_2$ (20 mL), and DBU (7.64 g, 50 mmol) was added dropwise. After the dropping was completed, under $N_2$ environment, refluxing was performed for 48 h. TLC monitoring showed that about 60% of the raw materials were not reacted. A reaction was stopped. The reaction solution was cooled to room temperature, poured into ice water (30 mL), and extracted with $CH_2Cl_2$ (30 mL×2). Organic phases were combined, washed with a saturated brine (100 mL), dried ($MgSO_4$), and subjected to suction filtration to remove the drying agent. A filtrate was concentrated under reduced pressure on a rotary evaporator to obtain a brownish black oily substance, and then purified by a column chromatography [V(EtOAc)/V(n-hexane)=0/1→2/23] to obtain target compound (±)-**54**, a light brown oily substance, 0.59 g (a combined yield of (±)-**52**→(±)-**54** was 28%).

$^1$H NMR ($CDCl_3$, 500 MHz) δ: 6.49-6.51 (m, 1H), 6.38-6.40 (m, 1H), 4.84 (dd, 1H, *J* = 1.0 Hz and 11.5 Hz), 4.63 (dd, 1H,

$J$ = 1.0 Hz and 11.5 Hz), 2.95-3.01 (m, 1H), 2.66-2.70 (m, 1H), 2.53 (dd, 1H, $J$ = 0.8 Hz and 17.8 Hz), 2.42 (d, 1H, $J$ = 17.5 Hz), 2.28-2.30 (m, 1H), 2.22-2.24 (m, 1H), 2.06 (ddd, 1H, $J$ = 1.8 Hz, 8.5 Hz and 13.3 Hz), 1.46 (s, 9H), 1.41-1.44 (m, 1H), 0.44-0.47 (m, 2H), 0.25-0.33 (m, 2H).

$^{13}C$ NMR ($CDCl_3$, 126 MHz) δ: 170.98, 137.31, 136.84, 83.29, 80.91, 51.09, 50.24, 49.20, 47.98, 39.28, 36.43, 34.55, 32.34, 28.24, 7.83, 4.74.

ESI-HRMS: (*m*/*z*) calcd. for $C_{18}H_{26}NO_4$ ($[M+H]^+$) 320.1856, found: 320.1854.

Step 10: Synthesis of a p-toluenesulfonate of compound (±)-**55**

At room temperature, the compound (±)-**54** (0.59 g, 1.8 mmol) was dissolved in EtOH (10 mL), added into water (5 mL) and stirred, then iron powder (0.52 g, 9.3 mmol) and $NH_4Cl$ (0.20 g, 3.7 mmol) were sequentially added, air in a reaction vessel was replaced with nitrogen (balloon) according to standard operations, and refluxing was performed overnight. TLC monitoring showed that a reaction was completed. The reaction solution was cooled to room temperature and subjected to suction filtration to remove a solid. A filtrate was added into a saturated $NaHCO_3$ solution (50 mL) and extracted with EtOAc (40 mL×3). Organic phases were combined, washed with a saturated brine (100 mL), dried ($MgSO_4$), and subjected to suction filtration to remove the drying agent. A filtrate was concentrated under reduced pressure on a rotary evaporator to obtain a light brown oily substance. At room temperature, EtOAc (5 mL) was added to dilute the oily substance, then *p*-TsOH·$H_2O$ (0.38 g, 2.0 mmol) was added and stirred for dissolution, and the system was transferred into an ice water bath and stirred until a white flocculent solid was gradually precipitated out. EtOAc (7.5 mL) was added into the system, fully stirred, and continuously stirred overnight at room temperature. The solid was collected by suction filtration and dried with a vacuum oil pump to obtain the target compound (±)-**55** p-toluenesulfonate, a white solid, 0.60 g (70%) was obtained. A melting point was 171.6°C-175.8°C.

$^1H$ NMR (DMSO-$d_6$, 500 MHz) δ: 7.69 (brs, 3H), 7.47 (d, 2H, $J$ = 8.0 Hz), 7.11 (d, 2H, $J$ = 8.0 Hz), 6.43-6.45 (m, 1H), 6.36-6.38 (m, 1H), 3.08-3.13 (m, 1H), 2.97-3.02 (m, 1H), 2.84-2.90 (m, 1H), 2.55 (dd, 1H, $J$ = 4.5 Hz and 9.0 Hz), 2.37 (d, 1H, $J$ = 17.5 Hz), 2.31 (d, 1H, $J$ = 17.0 Hz), 2.29 (s, 3H), 2.17-2.21 (m, 2H), 1.79 (ddd, 1H, $J$ = 1.8 Hz, 8.5 Hz and 12.8 Hz), 1.41 (s, 9H), 1.19-1.23 (m, 1H), 0.36-0.42 (m, 2H), 0.27-0.31 (m, 1H), 0.21-0.25 (m, 1H);

$^{13}C$ NMR (DMSO-$d_6$, 126 MHz) δ: 170.55, 145.60, 137.71, 137.00, 136.45, 128.09, 125.51, 80.05, 50.56, 49.42, 48.59, 47.70, 46.97, 37.08, 36.34, 33.71, 31.86, 27.75, 20.80, 7.48, 4.45;

ESI-HRMS: (*m*/*z*) calcd. for $C_{18}H_{28}NO_2$ ($[M(\text{free base})+H]^+$) 290.2115, found: 290.2112.

Step 11: Synthesis of a p-toluenesulfonate of compound (±)-**I-9**

At room temperature, compound (±)-**55** p-toluenesulfonate (0.56 g, 1.2 mmol) was added into a saturated $NaHCO_3$ solution (100 mL) and stirred for 20 min (suspension state), and EtOAc (60 mL×3) was added for extraction. Organic phases were combined, dried ($MgSO_4$), and subjected to suction filtration to remove the drying agent. A filtrate was concentrated under reduced pressure on a rotary evaporator to obtain a yellow oily substance, and $CH_2Cl_2$ (20 mL×3) was added to perform concentration for several times to dissolve the oily substance in $CH_2Cl_2$ (5 mL). Under an ice water bath, TFA (3 mL) was slowly added dropwise. After the dropping was completed, a reaction was carried out at room temperature for 6 h. TLC monitoring showed that the reaction was completed. After the reaction solution was concentrated under reduced pressure on a rotary evaporator to obtain a brown oily substance, $CH_2Cl_2$ (20 mL×3) was added to perform concentration for several times, and then drying was performed with a vacuum oil pump (a small amount of solid was precipitated out during the drying). Then, EtOAc (3 mL) was added for dissolution, a solution prepared by dissolving *p*-TsOH·$H_2O$ (0.25 g, 1.3 mmol) in EtOAc (3 mL) was added dropwise into the reaction solution until an off-white solid was gradually precipitated out of the system, and after the dropping was completed, stirring was performed overnight at room temperature. The solid was collected by suction filtration, and a filter cake was washed with EtOAc (1 mL) and dried with a vacuum oil pump to obtain a p-toluenesulfonate of compound (±)-**I-9**, a white solid, 0.38 g (77%), with a melting point of 187.5°C-188.5°C.

$^1H$ NMR ($CD_3OD$, 500 MHz) δ: 7.71 (d, 2H, $J$ = 8.0 Hz), 7.23 (d, 2H, $J$ = 8.0 Hz), 6.50-6.52 (m, 1H), 6.38-6.40 (m, 1H), 3.27 (d, 1H, $J$ = 13.0 Hz), 3.13 (d, 1H, $J$ = 13.0 Hz), 2.95-3.01 (m, 1H), 2.66-2.69 (m, 1H), 2.63 (d, 1H, $J$ = 17.5 Hz), 2.40 (d, 1H, $J$ = 17.5 Hz), 2.37 (s, 3H), 2.26-2.28 (m, 1H), 2.20-2.22 (m, 1H), 1.78 (ddd, 1H, $J$ = 1.5 Hz, 8.5 Hz and 13.0 Hz), 1.44 (dd, 1H, $J$ = 6.5 Hz and 13.0 Hz), 0.40-0.46 (m, 2H), 0.30-0.34 (m, 1H), 0.25-0.29 (m, 1H);

$^{13}C$ NMR ($CD_3OD$, 126 MHz) δ: 175.76, 143.51, 141.71, 138.32, 137.84, 129.82, 126.96, 52.26, 51.30, 50.45, 49.93, 48.37, 38.43, 37.52, 35.51, 33.66, 21.31, 8.27, 5.20;

ESI-HRMS: (*m*/*z*) calcd. for $C_{14}H_{20}NO_2$ ($[M(\text{free base})+H]^+$) 234.1489, found: 234.1486.

The compound (±)-**I-9** is a specific form of the compound of general formula **I** in the present application.

**Example 12 Synthesis of compound (+)-I-10**

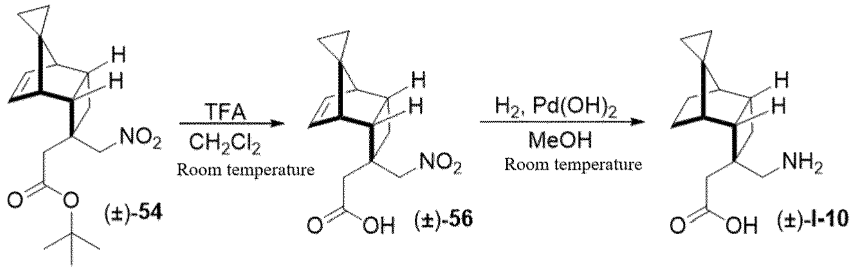


Step 1: Synthesis of compound (±)-**56**

The compound (±)-**54** (0.21 g, 0.66 mmol) was dissolved in $CH_2Cl_2$ (2 mL), and TFA (1.5 mL) was slowly added dropwise under an ice water bath. After the dropping was completed, a reaction was carried out at room temperature for 2-3 h. TLC monitoring showed that the reaction was completed. The reaction solution was concentrated under reduced pressure on a rotary evaporator. Then, $CH_2Cl_2$ (20 mL×4) was added to perform concentration for several times to obtain a brownish black oily substance, and then purified by a column chromatography [V(EtOAc)/V(n-hexane)=0/1-3/7] to obtain a yellow solid. n-hexane (1 mL) was added to the solid, stirred and slurried overnight at room temperature. The solid was collected by suction filtration and dried with a vacuum oil pump to obtain compound (±)-**56**, a white solid, 0.11 g (64%), with a melting point of 141.3°C-144.5°C.

$^1$H NMR (DMSO-$d_6$, 500 MHz) δ: 12.23 (s, 1H), 6.47-6.49 (m, 1H), 6.36-6.38 (m, 1H), 4.79 (s, 2H), 2.91-2.97 (m, 1H), 2.63-2.66 (m, 1H), 2.45 (d, 1H, *J* = 17.5 Hz), 2.27 (d, 1H, *J* = 17.5 Hz), 2.24-2.25 (m, 1H), 2.20-2.21 (m, 1H), 1.96 (ddd, 1H, *J* = 1.8 Hz, 8.5 Hz and 13.0 Hz), 1.36 (dd, 1H, *J* = 6.5 Hz and 13.0 Hz), 0.36-0.41 (m, 2H), 0.22-0.30 (m, 2H);
$^{13}$C NMR (DMSO-$d_6$, 126 MHz) δ: 172.26, 136.94, 136.57, 82.95, 50.35, 49.40, 48.77, 47.08, 38.50, 35.06, 33.94, 31.69, 7.44, 4.43;
ESI-HRMS: *(m/z)* calcd. for $C_{14}H_{18}NO_4$ ([M+H]$^+$) 264.1230, found: 264.1225.

Step 2: Synthesis of compound (±)-**I-10**

The compound (±)-**56** (0.11 g, 0.42 mmol) was dissolved in $CH_3OH$ (3 mL), 10% $Pd(OH)_2$/C (0.04 g) was added, air in a reaction vessel was replaced with hydrogen (balloon) according to standard operations, and stirring was performed overnight at room temperature. TLC monitoring showed that a reaction was completed (the reaction was generally completed within 12 h). Suction filtration was performed to remove a solid. A filtrate was concentrated under reduced pressure on a rotary evaporator to obtain a white solid, and EtOAc (2 mL) was added, stirred and slurried at room temperature for 1 h. The solid was collected by suction filtration and dried under vacuum to obtain target product (±)-**I-10**, a white solid, 0.03 g (31%), with a melting point of 197.3°C-201.1°C.

$^1$H NMR ($CD_3OD$, 500 MHz) δ: 3.14 (d, 1H, *J* = 13.0 Hz), 3.01 (d, 1H, *J* = 12.5 Hz), 2.75 (dd, 1H, *J* = 1.0 Hz and 16.5 Hz), 2.63-2.78 (m, 1H), 2.66 (d, 1H, *J* = 16.0 Hz), 2.53-2.57 (m, 1H), 2.10-2.17 (m, 1H), 2.01 (dd, 1H, *J* = 7.5 Hz and 13.0 Hz), 1.79-1.90 (m, 4H), 1.60-1.62 (m, 1H), 1.42-1.44 (m, 1H), 0.42-0.51 (m, 4H);
$^{13}$C NMR ($CD_3OD$, 126 MHz) δ: 180.04, 52.77, 49.79, 47.72, 46.30, 45.63, 39.39, 38.20, 35.05, 33.87, 26.24, 25.76, 6.33, 5.15;
ESI-HRMS: (*m*/*z*) calcd. for $C_{14}H_{22}NO_2$ ([M+H]$^+$) 236.1645, found: 236.1642.

The compound (±)-**I-10** is a specific form of the compound of general formula **I** in the present application.

**Example 13 Synthesis of compound (±)-I-11 and a p-toluenesulfonate thereof**

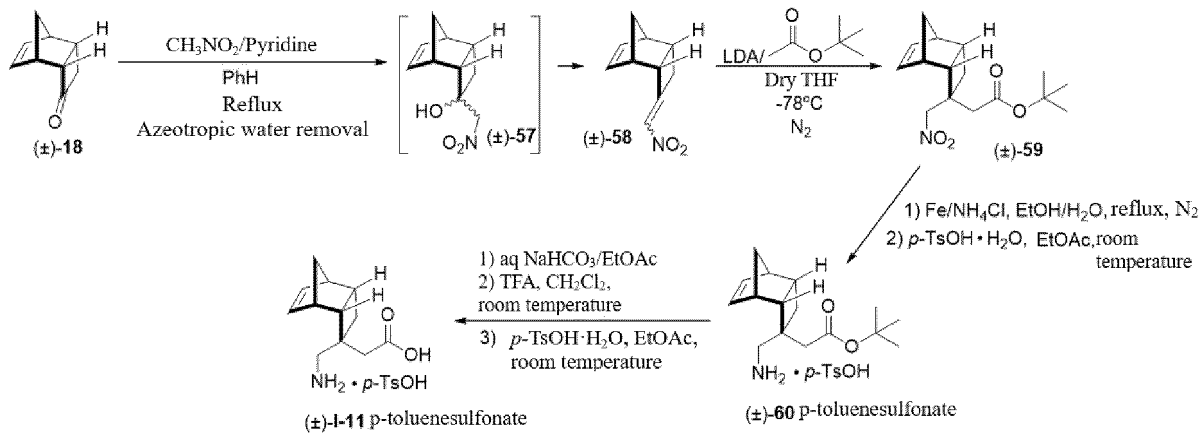


Step 1: Synthesis of compound (±)-**58**

The compound (±)-**18** (3.00 g, 22 mmol) was dissolved in benzene (40 mL), $CH_3NO_2$ (20 mL) and piperidine (0.95 g, 11 mmol) were sequentially added in a stirring state and heated to reflux, and water separation was performed by using a Dean-Stark water separator for 2 h. TLC monitoring showed that a reaction was completed. The reaction was stopped. The reaction solution was cooled to room temperature, poured into ice water (100 mL), and extracted with $CH_2Cl_2$ (50 mL×2). Organic phases were combined, sequentially washed with 1 M HCl (20 mL), a saturated $NaHCO_3$ solution (100 mL) and a saturated brine (100 mL), dried ($MgSO_4$), and filtered to remove the drying agent. A filtrate was concentrated under reduced pressure on a rotary evaporator to obtain a dark brown oily substance, and then purified by a column chromatography [V(EtOAc)/V(n-hexane)=0/1→3/97] to obtain target compound (±)-**58**, a yellow oily substance, 0.78 g. The product was composed of a Z/E mixture and directly used in a next reaction.

Step 2: Synthesis of compound (±)-**59**

Dry THF (5 mL) was added into a reaction vessel, and air in the reaction vessel was replaced with nitrogen (balloon) according to standard operations. Diisopropylamine (0.86 g, 8.5 mmol) was added under stirring and cooled to -78°C (liquid nitrogen-alcohol system). n-butyllithium (1.6 M hexane solution, 5.3 mL, 8.5 mmol) was added dropwise, and after the dropping was completed, a reaction was carried out at -78°C for 0.5 h. Then, a dry THF (1 mL) solution of tert-butyl acetate (0.99 g, 8.5 mmol) was added dropwise, and after the dropping was completed, a reaction was carried out continuously at -78°C for 0.5 h. A dry THF (1 mL) solution of the compound (±)-**58** (0.50 g, 2.8 mmol) was added dropwise, and after the dropping was completed, a reaction was carried out again at -78°C for 1-2 h. TLC monitoring showed that the reaction was completed. After the temperature thereof was restored to room temperature, the reaction solution was poured into ice water (15 mL) and extracted with $CH_2Cl_2$ (20 mL). An organic phase was sequentially washed with 1 M HCl (40 mL), a saturated $NaHCO_3$ solution (40 mL) and a saturated brine (40 mL), dried ($MgSO_4$), and filtered to remove the drying agent. A filtrate was concentrated under reduced pressure on a rotary evaporator to obtain a dark brown oily substance, and then purified by a column chromatography [V(EtOAc)/V(n-hexane)=0/1→3/97] to obtain target compound (±)-**59**, a light yellow oily substance, 0.51 g (a combined yield of (±)-**18**→(±)-**59** was 12%).

$^1H$ NMR ($CDCl_3$, 500 MHz) δ: 6.41-6.43 (m, 1H), 6.32-6.34 (m, 1H), 4.59 (d, 1H, *J* = 13.0 Hz), 4.54 (d, 1H, *J* = 13.0 Hz), 3.02-3.05 (m, 1H), 2.84-2.90 (m, 2H), 2.69 (dd, 1H, *J* = 1.0 Hz and 16.5 Hz), 2.59 (dd, 1H, *J* = 0.5 Hz and 16.5 Hz), 2.50-2.53 (m, 1H), 1.82-1.88 (m, 1H), 1.60-1.62 (m, 1H), 1.45-1.49 (m, 10H), 1.11-1.14 (m, 1H);
$^{13}C$ NMR ($CDCl_3$, 126 MHz) δ: 170.65, 138.31, 136.61, 81.21, 76.29, 53.54, 49.17, 45.75, 44.86, 44.48, 37.98, 34.36, 32.85, 28.27;
ESI-HRMS: (*m*/*z*) calcd. for $C_{16}H_{24}NO_4$ ($[M+H]^+$) 294.1700, found: 294.1698.

Step 3: Synthesis of compound (±)-**60** p-toluenesulfonate

At room temperature, the compound (±)-**59** (0.51 g, 1.7 mmol) was dissolved in EtOH (10 mL), added into water (5 mL) and stirred, then iron powder (0.47 g, 8.4 mmol) and $NH_4Cl$ (0.18 g, 3.4 mmol) were sequentially added thereto, air in a reaction vessel was replaced with nitrogen (balloon) according to standard operations, and refluxing was performed overnight under stirring. TLC monitoring showed that a reaction was completed. The reaction solution was cooled to room temperature and subjected to suction filtration to remove a solid. A filtrate was added into a saturated $NaHCO_3$ solution (50 mL) and extracted with EtOAc (40 mL×3). Organic phases were combined, sequentially washed with a saturated $NaHCO_3$

solution (80 mL) and a saturated brine (100 mL), dried ($MgSO_4$), subjected to suction filtration to remove the drying agent, and concentrated under reduced pressure on a rotary evaporator to obtain a brown oily substance. The brown oily substance was dissolved with EtOAc (5 mL), and then *p*-TsOH·$H_2O$ (0.35 g, 1.8 mmol) was added and stirred for dissolution until a white solid was gradually precipitated out. EtOAc (3 mL) was added into the system again, fully stirred, and continuously stirred and slurried at room temperature for 1-2 h. The solid was collected by suction filtration and dried with a vacuum oil pump to obtain target compound (±)-**60** p-toluenesulfonate, a white solid, 0.39 g (52%), with a melting point of 194.9°C-196.7°C.

$^1$H NMR (DMSO-$d_6$, 500 MHz) δ: 7.52 (brs, 3H), 7.48 (d, 2H, *J* = 8.5 Hz), 7.12 (d, 2H, *J* = 8.0 Hz), 6.40-6.42 (m, 1H), 6.26-6.28 (m, 1H), 2.99-3.00 (m, 1H), 2.83-2.89 (m, 1H), 2.76-2.82 (m, 3H), 2.58 (d, 1H, *J* = 16.0 Hz), 2.53 (d, 1H, *J* = 16.0 Hz), 2.33-2.35 (m, 1H), 2.29 (s, 3H), 1.63-1.68 (m, 1H), 1.47-1.49 (m, 1H), 1.42 (s, 9H), 1.27-1.31 (m, 1H), 1.05-1.07 (m, 1H);
$^{13}$C NMR (DMSO-$d_6$, 126 MHz) δ: 170.22, 145.63, 137.66, 137.30, 136.31, 128.06, 125.48, 80.39, 53.06, 47.38, 44.67, 43.98, 43.20, 40.12, 36.87, 32.62, 31.58, 27.82, 20.78;
ESI-HRMS: (*m*/*z*) calcd. for $C_{16}H_{26}NO_2$ ([M(free base)+H]$^+$) 264.1958, found: 264.1956.

Step 4: Synthesis of compound (±)-**I-11** and a p-toluenesulfonate thereof

At room temperature, the compound (±)-**60** p-toluenesulfonate (0.39 g, 0.90 mmol) was added into a saturated $NaHCO_3$ solution (50 mL) and stirred for 20 min (suspension state), and EtOAc (40 mL×3) was added again for extraction. Organic phases were combined, dried ($MgSO_4$), and subjected to suction filtration to remove the drying agent. A filtrate was concentrated under reduced pressure on a rotary evaporator to obtain a yellow oily substance, and $CH_2Cl_2$ (20 mL×3) was added to perform concentration for several times to dissolve the oily substance in $CH_2Cl_2$ (4 mL). Under an ice water bath, TFA (3 mL) was slowly added dropwise. After the dropping was completed, a reaction was carried out at room temperature for 5-6 h. TLC monitoring showed that the reaction was completed. After the reaction solution was concentrated under reduced pressure on a rotary evaporator to obtain a brown oily substance, $CH_2Cl_2$ (20 mL×3) and EtOAc (20 mL×3) were added to perform concentration for several times. The oily substance was dried with a vacuum oil pump to obtain (±)-**I-11**. The above (±)-**I-11** sample was dissolved with EtOAc (2 mL), and a*p*-TsOH·$H_2O$ (0.19 g in 2.5mL of EtOAc, 1.0 mmol) solution was added dropwise (an off-white solid was gradually precipitated out). After the dropping was completed, EtOAc (2.5 mL) was added to enable smooth stirring, and stirring was performed continuously at room temperature for 1 h. The solid was collected by suction filtration and dried with a vacuum oil pump to obtain a p-toluenesulfonate of compound (±)-**I-11**, a white solid 0.26 g (77%), with a melting point of 193.0°C-188.5°C.

$^1$H NMR ($CD_3OD$, 500 MHz) δ: 7.70 (d, 2H, *J* = 8.5 Hz), 7.23 (d, 2H, *J* = 8.0 Hz), 6.43-6.44 (m, 1H), 6.31-6.33 (m, 1H), 3.07 (d, 1H, *J* = 13.0 Hz), 3.02-3.03 (m, 1H), 2.99 (d, 1H, *J* = 13.0 Hz), 2.89-2.95 (m, 1H), 2.84-2.86 (m, 1H), 2.74 (d, 1H, *J* = 17.0 Hz), 2.70 (d, 1H, *J* = 17.0 Hz), 2.46 (ddd, 1H, *J* = 1.5 Hz, 4.5 Hz and 9.0 Hz), 2.37 (s, 3H), 1.75 (ddd, 1H, *J* = 1.8 Hz, 8.5 Hz and 12.8 Hz), 1.61-1.63 (m, 1H), 1.44 (dd, 1H, *J* = 6.5 Hz and 12.5 Hz), 1.16-1.18 (m, 1H);
$^{13}$C NMR ($CD_3OD$, 126 MHz) δ: 175.29, 143.52, 141.69, 139.13, 137.18, 129.81, 126.96, 54.43, 49.46, 46.36, 45.74, 44.77, 42.50, 37.95, 34.42, 33.32, 21.30;
ESI-HRMS: (*m*/*z*) calcd. for $C_{12}H_{18}NO_2$ ([M(free base)+H]$^+$) 208.1332, found: 208.1331.

The compound (±)-**I-11** is a specific form of the compound of general formula **I** in the present application.

**Example 14 Synthesis of compound (±)-I-12**

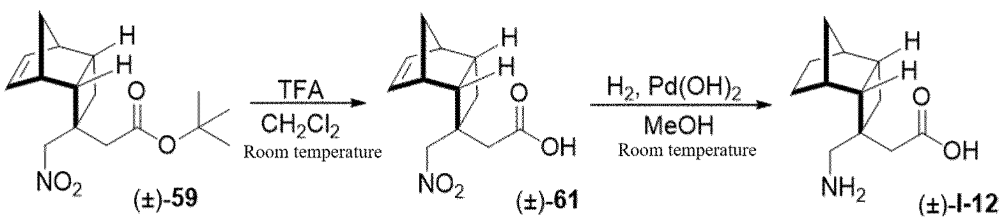


Step 1: Synthesis of compound (±)-**61**

The compound (±)-**59** (0.50 g, 1.7 mmol) was dissolved in $CH_2Cl_2$ (3 mL), and TFA (3 mL) was slowly added

dropwise under an ice water bath. After the dropping was completed, a reaction was carried out at room temperature for 3-4 h. TLC monitoring showed that the reaction was completed. After the reaction solution was concentrated under reduced pressure on a rotary evaporator to obtain a brown oily substance, $CH_2Cl_2$ (20 mL×5) was added to perform concentration for several times, and the resulting oily substance was purified by a column chromatography [V(EtOAc)/V(n-hexane) =0/1→3/7] to obtain an off-white solid. n-hexane (3 mL) was added to the solid and slurried at room temperature for 1 h. The solid was collected by suction filtration and dried with a vacuum oil pump to obtain compound (±)-**61**, a white solid, 0.32 g (79%), with a melting point of 91.4°C-93.8°C.

$^1$H NMR (DMSO-$d_6$, 500 MHz) δ: 12.33 (brs, 1H), 6.47-6.49 (m, 1H), 6.32-6.34 (m, 1H), 4.58 (d, 1H, *J* = 13.0 Hz), 4.52 (d, 1H, *J* = 13.0 Hz), 2.91-2.93 (m, 1H), 2.84-2.88 (m, 1H), 2.80-2.82 (m, 1H), 2.59 (d, 1H, *J* = 16.5 Hz), 2.55 (d, 1H, *J* = 17.0 Hz), 2.44-2.47 (m, 1H), 1.76 (ddd, 1H, *J* = 1.8 Hz, 8.3 Hz and 12.8 Hz), 1.47-1.49 (m, 1H), 1.40 (dd, 1H, *J* = 6.5 Hz and 13.0 Hz), 1.06-1.09 (m, 1H);
$^{13}$C NMR (DMSO-$d_6$, 126 MHz) δ: 172.14, 137.79, 136.27, 76.46, 52.74, 48.32, 45.10, 44.08, 42.66, 37.14, 33.58, 32.16;
ESI-HRMS: (*m*/*z*) calcd. for $C_{12}H_{16}NO_4$ ([M+H]$^+$) 238.1074, found: 238.1072.

Step 2: Synthesis of compound (±)-**I-12**

The compound (±)-**61** (0.30 g, 1.3 mmol) was dissolved in $CH_3OH$ (5 mL), 10% $Pd(OH)_2/C$ (0.08 g) was added thereto, air in a reaction vessel was replaced with hydrogen (balloon) according to standard operations, and stirring was performed overnight at room temperature. TLC monitoring showed that a reaction was completed (the reaction was generally completed within 12 h). Suction filtration was performed to remove a solid. A filtrate was concentrated under reduced pressure on a rotary evaporator to obtain a white solid, and a mixed solvent (3 mL) of $CH_3OH$/EtOAc = 1/3 (v/v) was added thereto and stirred at room temperature for 1 h. The solid was collected by suction filtration and dried to obtain target product (±)-**I-12**, a white solid, 0.05 g (19%), with a melting point of 153.2°C-157.3°C.

$^1$H NMR ($CD_3OD$, 500 MHz) δ: 3.17 (d, 1H, *J* = 13.5 Hz), 3.14 (d, 1H, *J* = 13.5 Hz), 2.68 (s, 2H), 2.57-2.64 (m, 1H), 2.49-2.51 (m, 1H), 2.25-2.27 (m, 1H), 2.13-2.16 (m, 1H), 2.01 (dd, 1H, *J* = 7.5 Hz and 12.5 Hz), 1.73-1.82 (m, 3H), 1.61-1.67 (m, 1H), 1.50-1.55 (m, 1H), 1.43-1.45 (m, 1H), 1.27-1.30 (m, 1H);
$^{13}$C NMR ($CD_3OD$, 126 MHz) δ: 180.18, 53.17, 53.31, 47.14, 43.23, 40.81, 40.06, 36.72, 34.78, 32.43, 26.51, 25.22;
ESI-HRMS: (*m*/*z*) calcd. for $C_{12}H_{20}NO_2$ ([M+H]$^+$) 210.1489, found: 210.1485.

The compound (±)-**I-12** is a specific form of the compound of general formula **I** in the present application.

**Example 15 Synthesis of compound (±)-I-13**

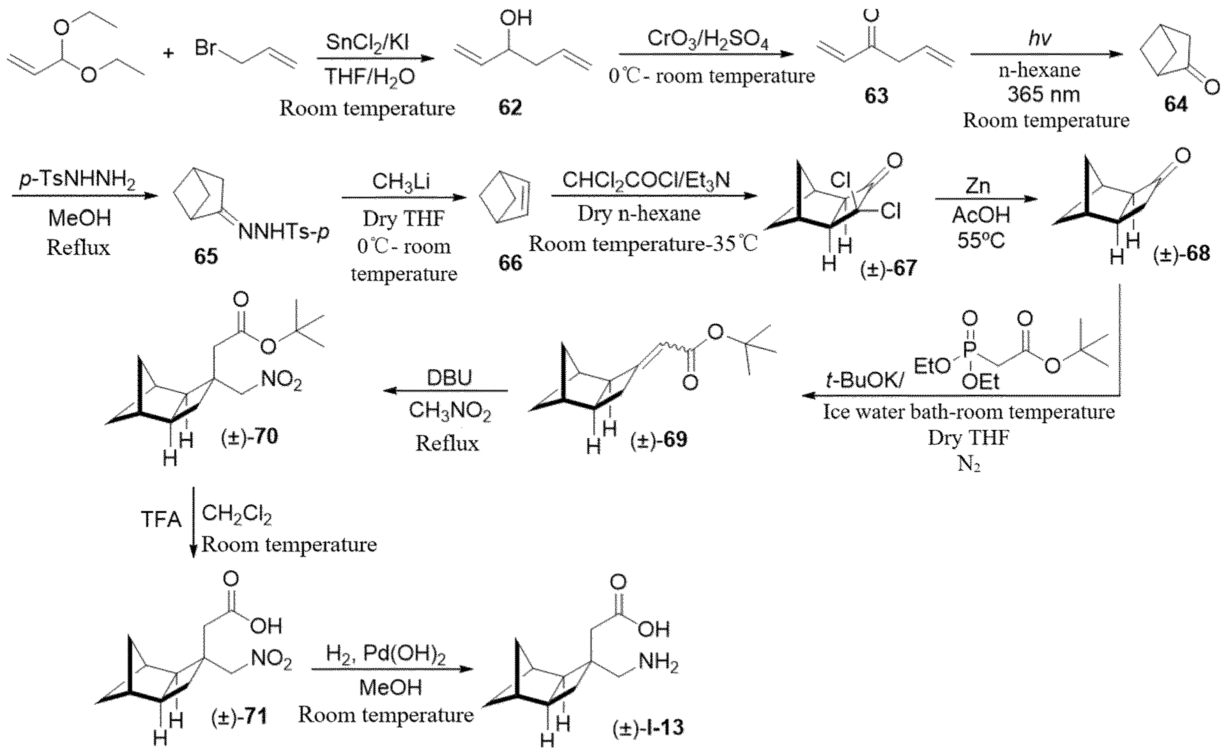


Step 1: Synthesis of compound **62**

Under cooling of an ice water bath and under stirring, $SnCl_2 \cdot 2H_2O$ (1299.93 g, 5.76 mol), KI (956.29 g, 5.76 mol) and allyl bromide (696.94 g, 5.76 mol) were sequentially added into deionized water (8.25 L) and stirred (the solution was orange-red). After the internal temperature of the system was stabilized at 10°C, a THF (768 mL) solution of acrolein diethyl acetal (500.00 g, 3.84 mol) was slowly added dropwise (the internal temperature was monitored to be not higher than 20°C, and the color of the system was changed from orange-red to light yellow). After the dropping was completed, stirring was performed overnight at room temperature. TLC monitoring showed that a reaction was completed. The reaction was stopped. The reaction solution was poured into $CH_2Cl_2$ (8 L) and stirred, an organic phase was separated, and an aqueous phase was extracted once again with $CH_2Cl_2$ (8 L). Organic phases were combined, washed with a saturated brine (5 L), dried ($MgSO_4$), and subjected to suction filtration to remove the drying agent. A filtrate was concentrated under reduced pressure on a rotary evaporator (at a low temperature of 30°C) to obtain a light yellow oily substance. The oily substance was purified by reduced pressure distillation. A fraction with a top temperature of about 60°C/30 mmHg was collected to obtain target product **62,** a colorless transparent oily substance, 183.50 g (49%). $^1H$ NMR ($CDCl_3$, 500 MHz) $\delta$: 5.78-5.93 (m, 2H), 5.24-5.28 (m, 1H), 5.13-5.18 (m, 3H), 4.17-4.21 (m, 1H), 2.33-2.39 (m, 1H), 2.26-2.32 (m, 1H). The product was directly used in a next step without further purification.

Step 2: Synthesis of compound **63**

Preparation of a Jones reagent: Water (267 mL) was added into a reaction vessel and placed in an ice water bath for cooling. $CrO_3$ (122.83 g, 1.23 mol) was added in portions under a stirring state to form an orange-red suspension, then concentrated $H_2SO_4$ (131 mL) was slowly added dropwise, and after the dropping was completed, the solution was stored for later use.

The compound **62** (120.56 g, 1.23 mol) was dissolved in acetone (430 mL), then placed in an ice water bath and stirred, and the Jones reagent was slowly added dropwise (a green solid was generated during the dropping, and the color of the solution was changed from blue-green to blackish green). TLC monitoring was performed during the dropping. After TLC monitoring showed that a reaction was completed, the dropping was stopped immediately. n-pentane (400 mL) was added into the reaction system for dilution, washed with water (400 mL), and an aqueous phase was extracted reversely with n-pentane (250 mL×2). Organic phases were combined, sequentially washed with a 10% sodium sulfite solution (200 mL) and a saturated brine (200 mL×3), dried ($MgSO_4$), and subjected to suction filtration to remove the drying agent. A filtrate was first subjected to atmospheric fractionation to remove n-pentane (with a top temperature of 36°C-38°C), and then distilled under reduced pressure. A fraction at 58°C-60°C/30 mmHg was collected (a receiving bottle needed to be placed in liquid nitrogen for protection at a low temperature) to obtain target compound **63,** a light yellow oily substance,

26.42 g (22%). $^1$H NMR ($CDCl_3$, 500 MHz) δ: 6.39 (dd, 1H, *J* = 10.5 Hz and 18.0 Hz), 6.26 (dd, 1H, *J* = 1.3 Hz and 17.8 Hz), 5.92-6.00 (m, 1H), 5.87 (dd, 1H, *J* = 1.0 Hz and 10.5 Hz), 5.15-5.23 (m, 2H), 3.37-3.39 (m, 2H). The product contained a certain amount of n-pentane and was directly used in a next step without further purification.

Step 3: Synthesis of compound **64**

The compound **63** (26.42 g, 0.27 mol) was dissolved in n-pentane (1.5 L) in a quartz vessel, and air in the reaction vessel was replaced with nitrogen (balloon) according to standard operations. The reaction device was placed in a dark environment, stirred, and irradiated with an ultraviolet lamp (365 nm; 15 W×6) at room temperature for 7-14 days (the color of the solution was changed from colorless transparent to purple-red and then faded into light purple). During a reaction, a small amount of a viscous polymer byproduct was generated and adhered to an inner wall of the reaction vessel, activated carbon (10 g) and diatomite (10 g) were added and stirred for 10 min every 2-3 days, suction filtration was performed to remove the polymer, and a filtrate was put into the reaction again according to the above operations. The reaction was stopped until TLC monitoring showed that a small amount of the raw materials were remained. After the reaction solution was concentrated under reduced pressure (< 30°C) on a rotary evaporator to 1/2 of the original volume, $Br_2$ was slowly added dropwise into the system until $Br_2$ was not faded (the solution was light orange). Then, a 10% sodium thiosulfate solution (200 mL) was added and stirred to destroy the excess $Br_2$, and at this time, the solution was faded to colorless. An organic phase was separated, dried ($MgSO_4$), and subjected to suction filtration to remove the drying agent. A filtrate was concentrated under reduced pressure (< 30°C for concentration) on a rotary evaporator to obtain an oily substance, and then $CH_3OH$ (20 mL) was added to perform concentration to obtain a crude product of target compound **64,** a light yellow oily substance, 6.61 g. The product was directly used in a next reaction without characterization.

Step 4: Synthesis of compound **65**

The compound **64** (16.00 g, 0.17 mol) was dissolved in $CH_3OH$ (320 mL), 4-methylbenzenesulfonhydrazide (31.00 g, 0.17 mol) was added thereto, air in a reaction vessel was replaced with nitrogen (balloon) according to standard operations, and then refluxing was performed overnight. TLC monitoring showed that a reaction was completed. The reaction solution was cooled to room temperature and concentrated under reduced pressure on a rotary evaporator to obtain a yellow oily substance. The oily substance was dissolved with $CH_2Cl_2$ (500 mL), then sequentially washed with 1 M HCl (400 mL×6) and a saturated brine (200 mL), dried ($MgSO_4$), and subjected to suction filtration to remove the drying agent. A filtrate was concentrated under reduced pressure on a rotary evaporator to obtain a yellow oily substance, and n-hexane (50 mL×2) was added to perform concentration for several times to obtain a yellow solid. EtOAc (3 mL)/n-hexane (30 mL) was added to the solid and slurried at room temperature for 1 h. The solid was collected by suction filtration and dried with a vacuum oil pump to obtain compound **65,** a light yellow solid, 17.09 g (a combined yield of **63**→**65** was 10%), with a melting point of 174.7°C-177.6°C.

$^1$H NMR ($CDCl_3$, 500 MHz) δ: 7.86 (d, 2H, *J* = 8.0 Hz), 7.32 (d, 2H, *J* = 8.0 Hz), 7.21 (brs, 1H), 2.98-3.00 (m, 1H), 2.65-2.68 (m, 1H), 2.43 (s, 3H), 2.16-2.17 (m, 2H), 1.98-2.04 (m, 2H), 1.31-1.32 (m, 2H);
$^{13}$C NMR ($CDCl_3$, 126 MHz) δ: 168.36, 144.10, 135.67, 129.74, 128.18, 49.53, 42.05, 36.29, 32.91, 21.76.

Step 5: Synthesis of compound **66**

The compound **65** (4.23 g, 19 mmol) was dissolved in dry THF (50 mL), and air in a reaction vessel was replaced with nitrogen (balloon) according to standard operations. Under an ice water bath and stirring, $CH_3Li$ (1.6 M diethoxymethane solution, 42 mL, 67.2 mmol) was slowly added dropwise into the system. After the dropping was completed, a reaction was carried out overnight at room temperature. TLC monitoring showed that the reaction was completed. Under the ice water bath, water (10 mL) was added into the system to quench the reaction. The reaction solution was poured into ice water (200 mL) and extracted with n-pentane (100 mL×3). Organic phases were combined, sequentially washed with 1 M HCl (100 mL×2) and a saturated brine (100 mL), dried ($MgSO_4$), and subjected to suction filtration to remove the drying agent. A filtrate was subjected to atmospheric fractionation to remove the solvent, so as to obtain a crude product of target compound **66** (1.52 g). The product was directly used in a next reaction without further purification.

Step 6: Synthesis of compound (±)-**67**

At room temperature, the compound **66** (1.52 g, calculated as 19 mmol) and dichloroacetyl chloride (3.72 g, 25 mmol) were sequentially dissolved in dry n-hexane (20 mL), and air in a reaction vessel was replaced with nitrogen (balloon) according to standard operations. At room temperature, a dry n-hexane (10 mL) solution of triethylamine (2.80g, 28 mmol) was slowly added dropwise in a stirring state. The system released heat during the dropping, and the internal

temperature was maintained at 30°C-35°C by controlling the dropping speed. After the dropping was completed, the reaction vessel was placed in an oil bath and stirred at 35°C for 2 h, and the dry n-hexane (50 mL) was supplemented and fully stirred to carry out a reaction at room temperature overnight. TLC monitoring showed that the reaction was completed. The reaction solution was cooled to room temperature and poured into ice water (200 mL). An organic phase was separated, sequentially washed with 1 M HCl (50 mL), water (200 mL), a saturated $NaHCO_3$ solution (100 mL×2) and a saturated brine (100 mL), dried ($MgSO_4$), and subjected to suction filtration to remove the drying agent. A filtrate was concentrated under reduced pressure on a rotary evaporator to obtain a crude product of compound (±)-**67**, a dark red oily substance, 1.89 g. The product was directly used in a next step without further purification.

Step 7: Synthesis of compound (±)-**68**

Zinc powder (3.23 g, 49 mmol) and glacial acetic acid (20 mL) were stirred and mixed, and a newly formulated glacial acetic acid (5 mL) solution of the compound (±)-**67** (1.89 g, 9.9 mmol) was added dropwise at room temperature. After the dropping was completed, under $N_2$ environment, the reaction mixture was stirred overnight in an oil bath at 55°C. TLC monitoring showed that a reaction was completed. The reaction mixture was cooled to room temperature and filtered with the assistance of diatomite. A filtrate was diluted with $CH_2Cl_2$ (200 mL), then sequentially washed with water (200 mL×3), a saturated $NaHCO_3$ solution (200 mL) and a saturated brine (200 mL), dried ($MgSO_4$), and subjected to suction filtration to remove the drying agent. The filtrate was concentrated under reduced pressure on a rotary evaporator to obtain a dark brown oily substance, and then purified by a column chromatography [V(EtOAc)/V(n-hexane)=0/1→1/20] to obtain a crude product of compound (±)-**68**, a dark brown oily substance, 0.31 g. The product was directly used in a next reaction without further purification.

Step 8: Synthesis of compound (±)-**69**

Under $N_2$ environment, *t*-BuOK (1.42 g, 13 mmol) was added into dry THF (5 mL) and stirred into a suspension under an ice water bath, and then tert-butyl diethylphosphonoacetate (3.20 g, 13 mmol) was added dropwise. After the dropping was completed, a reaction was carried out under the ice water bath for 1 h, and a newly formulated dry THF (10 mL) solution of the compound (±)-**68** (0.31 g, 2.5 mmol) was added dropwise. After the dropping was completed, stirring was performed overnight at room temperature. TLC monitoring showed that the reaction was completed. The reaction mixture was poured into ice water (100 mL), stirred, and extracted with $CH_2Cl_2$ (30 mL×3). Organic phases were combined by extraction, washed with a saturated brine, dried ($MgSO_4$), and subjected to suction filtration to remove the drying agent. A filtrate was concentrated under reduced pressure on a rotary evaporator to obtain a dark brown oily substance, and then purified by a column chromatography [V(EtOAc)/V(n-hexane)=0/1→1/12] to obtain a crude product of compound (±)-**69**, a dark brown oily substance, 0.40 g. The product was directly used in a next reaction without further purification.

Step 9: Synthesis of compound (±)-**70**

At room temperature, the compound (±)-**69** (0.40 g) was dissolved in $CH_3NO_2$ (10 mL) and stirred, and DBU (3.0 g) was added dropwise. After the dropping was completed, under $N_2$ environment, the reaction mixture was refluxed for one week. TLC monitoring showed that a large amount of the raw materials were still not reacted. A reaction was stopped. The reaction solution was cooled to room temperature, poured into ice water (100 mL), and extracted with $CH_2Cl_2$ (50 mL×2). Organic phases were combined, sequentially washed with ice water (100 mL) and a saturated brine (150 mL), dried ($MgSO_4$), and filtered to remove the drying agent. A filtrate was concentrated under reduced pressure on a rotary evaporator to obtain a brownish black oily substance, and then purified by a column chromatography [V(EtOAc)/V(n-hexane)=0/1→1/9] to obtain target product (±)-**70**, a light yellow oily substance, 0.10 g.

$^1$H NMR (acetone-$d_6$, 500 MHz) δ: 4.77 (d, 1H, *J* = 12.0 Hz), 4.65 (d, 1H, *J* = 12.0 Hz), 2.65 (d, 1H, *J* = 18.5 Hz), 2.57 (d, 1H, *J* = 18.5 Hz), 2.33-2.36 (m, 1H), 2.00-2.04 (m, 1H), 1.98-1.99 (m, 2H), 1.81-1.83 (m, 1H), 1.41 (s, 9H), 1.32-1.36 (m, 1H), 1.23-1.28 (m, 2H), 1.01-1.04 (m, 2H);

ESI-HRMS: (*m*/*z*) calcd. for $C_{15}H_{24}NO_4$ ($[M+H]^+$) 282.1700, found: 282.1702.

Step 10: Synthesis of compound (±)-**71**

The compound (±)-**70** (78 mg, 0.28 mmol) was dissolved in $CH_2Cl_2$ (1 mL), and TFA (0.5 mL) was slowly added dropwise under an ice water bath. After the dropping was completed, a reaction was carried out at room temperature for 4 h. TLC monitoring showed that the reaction was completed. The reaction mixture was poured into ice water (5 mL) and extracted with $CH_2Cl_2$ (2 mL×3). Organic phases were combined, washed with a saturated brine (2 mL×2), dried ($MgSO_4$), and filtered to remove the drying agent. A filtrate was concentrated on a rotary evaporator to obtain a yellow oily

substance. The oily substance was purified by a column chromatography [V(EtOAc)/V(n-hexane)=1/5→1/0] to obtain target product (±)-**71**, a colorless oily substance, 32 mg (51%).

$^1$H NMR (acetone-$d_6$+$D_2O$ (1 drop), 500 MHz) δ: 4.70 (d, 1H, *J* = 11.5 Hz), 4.53 (d, 1H, *J* = 11.5 Hz), 2.61 (d, 1H, *J* = 19.0 Hz), 2.55 (d, 1H, *J* = 18.5 Hz), 2.42-2.46 (m, 1H), 2.12-2.16 (m, 1H), 1.99-2.03 (m, 2H), 1.84-1.86 (m, 1H), 1.51-1.56 (m, 1H), 1.20-1.22 (m, 2H), 1.13-1.15 (m, 2H);
ESI-HRMS: (*m*/*z*) calcd. for $C_{11}H_{16}NO_4$ ([M+H]$^+$) 226.1074, found: 226.1070.

Step 11: Synthesis of compound (±)-**I-13**

The compound (±)-**71** (25 mg, 0.11 mmol) was dissolved in $CH_3OH$ (0.5 mL), 10% $Pd(OH)_2$/C (5 mg) was added thereto, air in a reaction vessel was replaced with hydrogen (balloon) according to standard operations, and stirring was performed overnight at room temperature. TLC monitoring showed that a reaction was completed (the reaction was generally completed within 12 h). Filtration was performed to remove a drying agent. A filtrate was concentrated under reduced pressure on a rotary evaporator to obtain a white solid, and EtOAc (0.5 mL) was added, stirred and slurried at room temperature for 2 h. The solid was collected by suction filtration and dried to obtain target product (±)-**I-13**, a white solid 11 mg (51%).

$^1$H NMR ($CD_3OD$+$D_2O$ (1 drop), 500 MHz) δ: 3.06 (s, 2H), 2.67 (d, 1H, *J* = 18.0 Hz), 2.43 (d, 1H, *J* = 18.0 Hz), 2.31-2.32 (m, 1H), 2.20-2.22 (m, 1H), 1.97-2.02 (m, 1H), 1.88-1.89 (m, 2H), 1.40-1.51 (m, 2H), 1.16-1.21 (m, 1H), 1.00-1.06 (m, 2H);
ESI-HRMS: (*m*/*z*) calcd. for $C_{11}H_{18}NO_2$ ([M+H]$^+$) 196.1332, found: 196.1341.

The compound (±)-**I-13** is a specific form of the compound of general formula **I** in the present application.

## Examples 16-21

Compounds in the following table were synthesized with reference to methods in Example 1 to Example 15.

| Example | Structure | Method used | ESI-MS |
|---|---|---|---|
| Example 16 | | Method in Example 12 | 238 ([M+H]$^+$) |
| Example 17 | 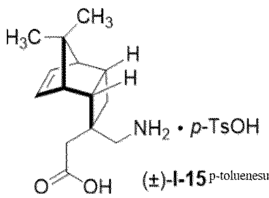 (±)-**I-15** p-toluenesulfonate | Method in Example 11 | 236 ([M M(free base)+H]$^+$) |
| Example 18 | | Method in Example 12 | 224 ([M+H]$^+$) |
| Example 19 | 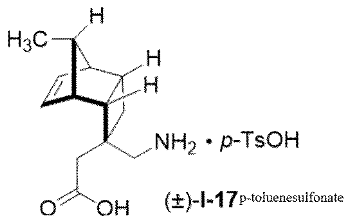 (±)-**I-17** p-toluenesulfonate | Method in Example 11 | 222 ([M(free base)+H]$^+$) |
| Example 20 | | Method in Example 10 | 264 ([M+H]$^+$) |
| Example 21 | | Method in Example 10 | 292 ([M+H]$^+$) |

## Example 22

| Capsule component | Amount/capsule |
|---|---|
| (±)-**I-3** sample | 75 mg |
| Lactose | 6.2 mg |
| Disodium hydrogen phosphate | 2.0 mg |
| Pregelatinized starch | 4.2 mg |
| Polyvinylpyrrolidone | 4.2 mg |
| Talc powder | 8.4 mg |

Preparation process: A (±)-**I-3** sample was pulverized and sieved for later use. The (±)-**I-3** sample, lactose and pre-gelatinized starch were added according to the above formulation amounts and premixed for 15 min. Disodium hydrogen phosphate and polyvinylpyrrolidone were added according to formulation amounts and mixed for 10 min. Talc powder was added according to a formulation amount and mixed for 30 min. The mixed material was filled into a capsule according to a specification to prepare a (±)-**I-3** capsule.

## Example 23

| Tablet component | Amount/tablet |
|---|---|
| (±)-**I-4** sample | 63 mg |
| Microcrystalline cellulose | 3 mg |
| Pregelatinized starch | 21 mg |
| Polyvinylpyrrolidone | 10 mg |
| Carboxymethyl starch sodium salt | 1.5 mg |
| Magnesium stearate | 1.5 mg |

Preparation process: A (±)-**I-4** sample and pregelatinized starch were separately sieved and fully mixed evenly. A polyvinylpyrrolidone solution was added and mixed to prepare a soft material, the soft material was sieved to prepare a wet particle that was dried at 80°C. A carboxymethyl starch sodium salt, microcrystalline cellulose and magnesium stearate were separately sieved in advance, then added into the above particle, mixed evenly, and tableted.

## Example 24

| Injection component | Amount/50 mL |
|---|---|
| (+)-**I-3** sample | 1000 mg |
| NaCl | 350 mg |
| NaOH | Appropriate amount (adjust the pH to 5.0-7.0) |
| Hydrochloric acid | Appropriate amount (adjust the pH to 5.0-7.0) |
| Water for injection | 50 mL |

Preparation process: Water for injection and a (+)-**I-3** sample were added first and stirred for dissolution, the pH value was adjusted to 5.0-7.0 with NaOH and hydrochloric acid, 0.3 g of activated carbon was added and stirred at room temperature for 30 min, filtration was performed with a microporous filter membrane to obtain a filtrate, the concentration of the solution was determined by a central control, and the solution was packed at 5 mL per ampoule and sterilized at 100°C for 30 min to obtain an injection.

**Example 25**

| Granule component | per 100 bag |
|---|---|
| (-)-**I-3** sample | 5.0 g |
| Sucrose laurate | 2.0 g |
| Sucrose | 40.0 g |
| Crosslinked povidone | 1.0 g |
| Carboxymethyl cellulose | 1.25 g |
| Silica | 0.7 g |
| Aspartame | 0.025 g |
| Apple essence | 0.025 g |

Preparation process: A (-)-**I-3** sample, sucrose, crosslinked povidone and carboxymethyl cellulose were separately sieved with a 100-mesh sieve, and sucrose laurate was prepared into a 25% concentrated solution for later use with ethanol at 60°C. The above components were weighed according to formulation amounts and fully fluidized and mixed. Then, 25% sucrose laurate was added to prepare a soft material, which was dried at 55°C and pelletized at 20 mesh. Granulation was performed with a 12-mesh sieve, silica, aspartame and apple essence were added, the bag weight was measured, and packaging was performed.

**Example 26**

| Freeze-dried formulation component | Amount |
|---|---|
| (+)-**I-4** sample | 5.0 g |
| Sodium hydroxide | Appropriate amount |
| Citric acid | Appropriate amount |
| Mannitol | 23.0 g |
| Lactose | 21.0 g |
| Water for injection | 100 mL |

Preparation process: 80 mL of water for injection was weighed, a (+)-**I-4** sample, mannitol and lactose were added thereto and stirred for dissolution, 1 mol/L citric acid and 1 mol/L sodium hydroxide were then added to adjust the pH to 5.0-7.0, and the water was supplemented to 100 mL. 0.5 g of activated carbon was added and stirred at 30°C for 20 min, and decarbonized. A microporous filter membrane was adopted for filtration and sterilization. A filtrate was separated into 1 mL per tube, pre-frozen at -40°C for 5 h, and freeze-dried under reduced pressure for 12 h (pressure<20 Pa). After the freeze-drying was completed, the sample temperature reached room temperature and then dried for 5 h to prepare a white loose block, which was obtained after being sealed.

**Example 27**

| Oral liquid component | Amount/100 mL |
|---|---|
| (+)-**I-3** sample | 2.0 g |
| Chitosan | 0.05 g |

(continued)

| Oral liquid component | Amount/100 mL |
|---|---|
| Acesulfame | 0.25 g |
| Strawberry essence | 0.03 g |
| Malic acid | 0.05 g |
| $NaHCO_3$ | Appropriate amount |
| Purified water | 100 mL |

Preparation process: The above formulation amount of malic acid was dissolved in 25 mL of purified water, a formulation amount of chitosan was added thereto and fully stirred for complete dissolution, and an appropriate amount of a 1 mol/L sodium bicarbonate solution was weighed and added into the above solution for rapid neutralization to adjust the pH value of the chitosan solution to 5.0-7.0 for later use. Then, the formulation amounts of acesulfame and strawberry essence were added into 40 mL of purified water and stirred for dissolution, then a formulation amount of a (+)-**I-3** sample was added thereto and stirred for dissolution, then the above standby chitosan solution was added thereto and stirred evenly, and the remaining formulation amount of purified water was supplemented into the mixed drug solution and stirred for even mixing to obtain a (+)-**I-3** oral liquid with the pH of 5.0-7.0.

**Example 28 *In vitro* binding of compounds to human recombinant calcium ion channel Cav2.2/β3/α2δ-1**

There are four subtypes of the voltage-gated calcium ion channel α2δ subunit,α2δ-1, α2δ-2, α2δ-3 and α2δ-4, in whichα2δ-1 is the subtype that mediates chronic neuropathic pain (Field, M.J. et al.; Proc. Natl. Acad. Sci. U.S.A. 2006, 103, 17537-17542). Therefore, the binding strength of a compound toα2δ-1 is a direct indicator for measuring an analgesic effect thereof on the chronic neuropathic pain (Calandre, E.P. et al.; Expert Rev. Neurother. 2016, 16, 1263-1277).

The *in vitro* binding strength of the compound of the present application to human recombinant calcium ion channel Cav2.2/β3/α2δ-1 is basically operated according to a reported method (Gee, N.S. et al.; J. Biol. Chem. 1996, 271, 5768-5776; Marais, E. et al.; Mol. Pharmacol. 2001, 59, 1243-1248.). CHO cells expressing the human recombinant calcium ion channel Cav2.2/β3/α2δ-1 were used in the test. After routine separation of cell membranes, 3 μg of the cell membranes were added into each well with a modified HEPES/KOH buffer solution (pH 7.4) as a solution of the test system. Then, 5 nM [$^3$H] gabapentin and the compound to be tested at 6 concentrations (3, 9, 27, 81, 243, and 729 nM) were added, and the test system was incubated at 25°C for 120 min. Non-specific binding was obtained by replacing the compound to be tested in the above test system with 10 μM gabapentin. After the incubation was completed, the cell membranes were collected by filtration and washed with 50 mM Tris-HCl (pH 7.4), and then the radioactivity of [$^3$H] gabapentin bound to the cell membranes was tested by a liquid scintillation technology. The compound of the present application and [$^3$H] gabapentin competitively bound to the human recombinant calcium ion channel Cav2.2/β3/α2δ-1. An inhibition rate of the compound of the present application on the binding of the [$^3$H] gabapentin to the human recombinant calcium ion channel Cav2.2/β3/α2δ-1 was calculated according to the following formula:

$$\text{inhibition rate}=[(I-I_U)/(I_0-I_U)]\times 100\%$$

wherein

I is the radioactivity corresponding to that the compound to be tested and [$^3$H] gabapentin are simultaneously co-incubated with the human recombinant calcium ion channel Cav2.2/β3/α2δ-1,
$I_0$ is the radioactivity corresponding to co-incubation of [$^3$H] gabapentin and the human recombinant calcium ion channel Cav2.2/β3/α2δ-1 without adding the compound to be tested into the incubation system, and
$I_U$ is the radioactivity corresponding to that 10 μM gabapentin and [$^3$H] gabapentin are simultaneously co-incubated with the human recombinant calcium ion channel Cav2.2/β3/α2δ-1.

The concentration of the compound to be tested that inhibits 50% of the binding of [$^3$H] gabapentin to the human recombinant calcium ion channel Cav2.2/β3/α2δ-1 is defined as $IC_{50}$, and the $IC_{50}$ is calculated by nonlinear least squares regression analysis using the above inhibition rate (MathIQ™, ID Business Solutions Ltd., UK). Test results are shown in the following table.

| Compound | $IC_{50}$ (nM) | Compound | $IC_{50}$ (nM) |
|---|---|---|---|
| (±)-**I-1** | > 729 | (±)-**I-9** p-toluenesulfonate | > 729 |
| (±)-**I-2** | 470 | (±)-**I-10** | > 729 |
| (±)-**I-3** | 15.2 | (±)-**I-11** p-toluenesulfonate | > 729 |
| (±)-**I-4** p-toluenesulfonate | 16.9 | **(±)-I-12** | > 729 |
| (±)-**I-5** | 450 | (±)-**I-13** | 6,234 |
| (±)-**I-6** p-toluenesulfonate | 300 | (±)-**I-14** | 600 |
| (-)-**I-3** p-toluenesulfonate | > 243 | (±)-**I-15** p-toluenesulfonate | 580 |
| (+)**-1-3** p-toluenesulfonate | 10.6 | (±)-**I-16** | 477 |
| (-)-**I-4** p-toluenesulfonate | > 243 | (±)-**I-17** p-toluenesulfonate | 453 |
| (+)-**I-4** p-toluenesulfonate | 7.64 | (±)-**I-18** | > 729 |
| (±)-**I-7** | 70 | (±)-**I-19** | > 729 |
| (±)-**I-8** | > 729 | Pregabalin | 38 |

As can be seen from activity data in this example, the compound of general formula **I** in the present application has good activity to bind to the human recombinant calcium ion channel Cav2.2/β3/α2δ-1, which can be used for preparing a drug for treatment of chronic neuropathic pain, epilepsy and anxiety. Gamma-aminobutyric acid drugs acting on the voltage-gated calcium ion channel α2δ-1 ligand, such as gabapentin, pregabalin, etc., in addition to having the analgesic effect on the chronic neuropathic pain, also have effects such as an antiepileptic effect (pregabalin, for indications approved by FDA of the United States) and an anti-anxiety effect (pregabalin, for indications approved by EMA of the Europe). These effects are related to the binding of the drugs to the voltage-gated calcium ion channel α2δ-1 ligand. Therefore, the compound of general formula **I** in the present application can also be used for preparing a drug for treatment of epilepsy and anxiety.

## Example 29 Analgesic effect of compounds in a chronic neuropathic pain rat model

The pharmacodynamics of the representative compounds (±)-**I-7**, (±)-**I-3** and (±)-**I-4** p-toluenesulfonate of the present application in a Sprague Dawley (SD) rat spared nerve injury (SNI) model after oral gavage administration at 30 mg/kg was evaluated.

Establishment of the SNI model: Surgery was performed according to an aseptic operation method, and various surgical instruments, including scalpels, tweezers, suture needles/threads, surgical cotton, etc., were disinfected before the surgery. Animals were anesthetized by intraperitoneal injection of a Zoletil™ 50 solution at 50 mg/kg and then placed in lateral decubitus positions. After hair in surgical areas of lower bodies of animals was shaved, the animals were disinfected alternately with iodophor and 75% alcohol. The skin was cut at upper edges of left hind limbs, muscles were bluntly separated to expose a sciatic nerve trunk and 3 branches thereof, i.e., a tibial nerve, a common peroneal nerve and a sural nerve. The tibial nerve and the common peroneal nerve were ligated and cut, and the sural nerve was preserved and maintained intact. After the surgery, wounds were sutured layer by layer, the animals were administered with 25% ampicillin (1 mL/kg) through intraperitoneal injection to prevent infection, and routine nursing was performed. In sham group, only the sciatic nerve and the branches thereof were exposed, the nerves were not ligated and cut, and other operations were the same as those in the model group.

Detection of a mechanical pain threshold: A Von-frey test fiber was used to detect the mechanical pain threshold of the sole of an operative side of each animal on day 1, day 3 and day 7 after the surgery, respectively. An "up-and-down" method was used as a method of detecting the mechanical pain threshold at the stimulation intensity of 0.4, 0.6, 1, 2, 4, 6, 8, and 15 g. During the test, the test fiber was used to vertically stimulate the site, close to an outer side, in the sole center of the left hind paw of a rat for 6-8 seconds each time at an interval of 5 seconds. A pain response was shown as an obvious paw withdrawal, paw licking, or paw lifting behavior of the animals during each test. From day 11 after the surgery, the animals were placed in an experimental environment for adaptation, wherein the adaptation was performed continuously for 15 min per day for 3 days. On day 13, a basic value of mechanical pain hypersensitivity was detected after the adaptation was completed. The mechanical pain hypersensitivity was expressed by a 50% paw-withdrawal threshold (PWT) according to a calculation formula of 50% PWT (g)=$10^{xf+k\delta}$, wherein xf is a log value of a fiber test force, k is a tabular value, and δ is an average spacing of the log value of the fiber test force. Pharmacodynamic evaluation: According to basic threshold detection results on day 13 after the surgery, the animals in the model group without the mechanical pain

hypersensitivity, that is, the PWT was greater than 4 g, were excluded, and the other successfully modeled animals were used for a formal test. On day 14 after the surgery, various compound to be tested were newly formulated. Pregabalin was directly dissolved with 0.9% sodium chloride at a dose of 30 mg/kg to obtain a solution at a desired concentration. (±)-**I-7**, (±)-**I-3** and (±)-**I-4** p-toluenesulfonate were also completely dissolved with a 0.9% sodium chloride solution at a dose of 30 mg/kg (calculated by free base). The successfully modeled animals were randomly divided into 5 groups with 8 animals in each group. In addition, 7 animals were selected in the sham group. After being labeled and weighed, the animals were administered the various drugs to be tested and a vehicle (0.9% sodium chloride) through oral gavage administration at a volume of 10 mL/kg, respectively. The mechanical pain hypersensitivity response of the left hind paw was detected at 1 h, 2 h, 4 h, 6 h, 8 h, 10 h, and 24 h after the administration. Evaluation was performed by using a blind method, and a detection method was the same as above. An area under a mechanical pain threshold-time curve (AUC) was calculated using GraphPad Prism Version 8.0.1 software. The analgesic intensity of rats in the SNI model was expressed as a maximum possible effect (MPE), wherein %MPE=[(AUC of administration group-AUC of vehicle group)/(AUC of sham group-AUC of vehicle group)]×100.

Results and discussions: Data were represented by mean ± standard error of the mean (SEM), and the GraphPad Prism Version 8.0.1 software was used for plotting. The data between the groups were compared by one-way ANOVA analysis, and a post hoc test was carried out using Dunnett for analysis. * represents a significant difference (*p*<0.05), ** represents *p*<0.01, and *** represents *p*<0.001. Statistical results are shown in details in a mechanical pain threshold time chart (FIG. 3A) and a chart of the area under the mechanical pain threshold-time curve (FIG. 3B). As can be seen from the figures below, similar to pregabalin as a positive drug, (±)-**I-7**, (±)-**I-3** and (±)-**I-4** p-toluenesulfonate have different degrees of inhibition effects on the mechanical pain hypersensitivity of the SNI rats after the administration at the dose of 30 mg/kg (calculated by free base), in which the areas under the mechanical pain threshold-time curves of the (±)-**I-7** and (±)-**I-4** p-toluenesulfonate groups is significantly improved compared with the vehicle control group. The maximum possible effects (%MPEs) of 4 compounds, i.e., the positive drug pregabalin, (±)-**I-7**, (±)-**I-3** and (±)-**I-4** p-toluenesulfonate, are 110.90%, 73.63%, 44.80% and 95.95%, respectively. The results show that the representative compounds (±)-**I-7**, (±)-**I-3** and (±)-**I-4** p-toluenesulfonate of the present application all have a certain analgesic effect in the SNI model at the dose of 30 mg/kg (calculated by free base), which can be used for preparing a drug for treatment of chronic neuropathic pain.

**Example 30 Analgesic effect of compounds in a chronic neuropathic pain rat model**

The pharmacodynamics of (-)-**I-3** p-toluenesulfonate, (+)-**I-3** p-toluenesulfonate, (-)-**I-4** p-toluenesulfonate and (+)-**I-4** p-toluenesulfonate in an SD rat spared nerve injury (SNI) model after oral gavage administration at 10 mg/kg (calculated by free base) was evaluated.

A model establishment method and a mechanical pain threshold detection method were the same as those in Example 29.

Pharmacodynamic evaluation: A specific detection method was the same as that in Example 29. On day 14 after surgery of animals, various compound to be tested were newly formulated. Pregabalin was directly dissolved with 0.9% sodium chloride at a dose of 10 mg/kg to obtain a solution at a desired concentration. The representative compounds (-)-**I-3** p-toluenesulfonate, (+)-**I-3** p-toluenesulfonate, (-)-**I-4** p-toluenesulfonate and (+)-**I-4** p-toluenesulfonate of the present application were used at a dose of 10 mg/kg (calculated by free base) with a vehicle involving 5% PEG 400 (polyethylene glycol 400) and 95% of 0.9% sodium chloride, that is, it was firstly dissolved with PEG 400 with a final volume of 5%, then added into 0.9% sodium chloride solution with a final volume of 95%, and subjected to vortex for full dissolution. The successfully modeled animals were randomly divided into 5 groups with 6 animals in each group. In addition, 5 animals were selected in a sham group. After being labeled and weighed, the animals were administered the various drugs to be tested and the vehicle (5% PEG 400+95% of 0.9% sodium chloride) through oral gavage administration at a volume of 10 mL/kg, respectively. The mechanical pain hypersensitivity response of the left hind paw was detected at 1 h, 2 h, 4 h, 6 h, 8 h, 10 h, and 24 h after the administration. Evaluation was performed by using a blind method, and a detection method was the same as above. Calculation methods of an area under a mechanical pain threshold-time curve (AUC) and a maximum possible effect (MPE) were the same as those in Example 29.

Results and discussions: Data were represented by mean ± standard error of the mean, and GraphPad Prism Version 8.0.1 software was used for plotting. The data between the groups were compared by one-way ANOVA analysis, and a post hoc test was carried out using Dunnett for analysis. * represents a significant difference(*p*<0.05), ** represents *p*<0.01, and *** represents *p*<0.001. Statistical results are shown in details in a mechanical pain threshold time chart (FIG. 4A) and a chart of the area under the mechanical pain threshold-time curve (FIG. 4B). As can be seen from FIG. 4A and FIG. 4B, the representative compounds (-)-**I-3** p-toluenesulfonate, (+)-**I-3** p-toluenesulfonate, (-)-**I-4** p-toluenesulfonate and (+)-**I-4** p-toluenesulfonate of the present application also show different degrees of inhibition effects on the mechanical pain hypersensitivity of the spared nerve injury model rats after the oral gavage administration at the dose of 10 mg/kg (calculated by free base), among which the area under the mechanical pain threshold-time curve of the (+)-**I-4** p-toluenesulfonate group is significantly improved compared with that of the vehicle control group, and the effect is significantly greater than that of the positive drug pregabalin at 10 mg/kg. The maximum possible effects (MPEs) of pregabalin, (-)-**I-3** p-toluenesulfonate, (+)-**I-3** p-toluenesulfonate, (-)-**I-4** p-toluenesulfonate and (+)-**I-4** p-toluenesulfonate were calculated as 23.04%, 30.22%, 48.50%, 18.53% and 69.09%, respectively. The above results show that (1) the representative compounds (-)-**I-3** p-toluenesulfonate, (+)-**I-3** p-toluenesulfonate, (-)-**I-4** p-toluenesulfonate and (+)-**I-4** p-toluenesulfonate of the present application show a better analgesic effect in the rat SNI model, which can be used for preparing a drug for treatment of chronic neuropathic pain; and (2) the analgesic activity of the compound (+)-**I-3** p-toluenesulfonate is higher than that of (-)-**I-3** p-toluenesulfonate, and the analgesic activity of the compound (+)-**I-4** p-toluenesulfonate is higher than that of (-)-**I-4** p-toluenesulfonate, indicating that the analgesic activity of the compound of the present application has stereochemical dependence. The *in vitro* binding test results of (-)-**I-3** p-toluenesulfonate, (+)-**I-3** p-toluenesulfonate, (-)-**I-4** p-toluenesulfonate and (+)-**I-4** p-toluenesulfonate in Example 28 are also consistent with the conclusions.

**Example 31 Antiepileptic effect of compounds in a mouse epilepsy model (maximum electroshock model (MES))**

A negative vehicle control group, a positive drug control group (pregabalin and gabapentin) and a test group of the compounds of the present application were set. Male ICR mice with a body weight of 22±2 g were selected. Test drugs and control drugs were both dissolved in DMSO (5% v/v) plus a 10% normal saline solution of 1,2-propanediol (95% v/v) and administered by gavage administration at a volume of 10 mL/kg. After fasted for 12 h, the mice were administered the compounds of the present application or the control drugs according to the body weight, and then subjected to electrical stimulation to induce generalized tonic-clonic seizure behaviors after a certain time interval (2 h for pregabalin, 1 h for gabapentin, and 3 h for the compounds of the present application). A YLS-9A electrophysiological stimulator (Shanghai Xinruan Information Technology Co., Ltd.) was used in an induction model, and parameters were set as follows: configuration 8, stimulation voltage 160 V, wave number 90. When the electrical stimulation was performed, ears on both sides of the mice were firstly wiped with normal saline and then given the electrical stimulation for 1 time with ear clip electrodes. Tonic straightening of hind limbs was used as a standard for epilepsy seizures. The animals with the tonic straightening of hind limbs were regarded as not being protected by the drugs, and the animals without the tonic straightening of hind limbs were regarded as being protected by the drugs. Response situations of the animals were observed and recorded, and obtained data were counted to calculate protection rates of the compounds.

Test results are shown in the following table.

| Compound | Administration dose (mg/kg) | Number of animals with tonic straightening of hind limbs/total number of detected animals | Protection rate (%) |
|---|---|---|---|
| Blank vehicle | - | 21/22 | 5 |
| Gabapentin | 300 | 2/8 | 75.0 |
| Pregabalin | 3 | 9/12 | 25 |
| Pregabalin | 6 | 10/12 | 16.67 |
| Pregabalin | 10 | 6/12 | 50 |
| Pregabalin | 18 | 5/12 | 58.33 |
| Pregabalin | 30 | 4/20 | 80 |
| Pregabalin | 60 | 1/12 | 91.66 |
| (+)-**I-3** p-toluenesulfonate | 3* | 7/8 | 12.5 |
| (+)-**I-3** p-toluenesulfonate | 6* | 3/8 | 62.5 |
| (+)-**I-3** p-toluenesulfonate | 10* | 3/8 | 62.5 |
| (+)-**I-3** p-toluenesulfonate | 18* | 3/12 | 75 |
| (+)-**I-3** p-toluenesulfonate | 30* | 1/8 | 87.5 |
| (+)-**I-3** p-toluenesulfonate | 60* | 0/8 | 100 |
| (+)-**I-4** p-toluenesulfonate | 3* | 7/8 | 12.5 |
| (+)-**I-4** p-toluenesulfonate | 6* | 6/8 | 25 |

(continued)

| Compound | Administration dose (mg/kg) | Number of animals with tonic straightening of hind limbs/total number of detected animals | Protection rate (%) |
|---|---|---|---|
| (+)-**I-4** p-toluenesulfonate | 10* | 7/8 | 12.5 |
| (+)-**I-4** p-toluenesulfonate | 18* | 7/12 | 41.7 |
| (+)-**I-4** p-toluenesulfonate | 30* | 3/8 | 62.5 |
| (+)-**I-4** p-toluenesulfonate | 60* | 1/6 | 83.3 |
| *Note: calculated by a drug prototype (free base). | | | |

Median effective doses ($ED_{50}$) of the compounds to be tested for animal protection were calculated by a least square method (Graphpad Prism 5) with an administration dose-protection rate curve. Fitting curves are shown in FIG. 5.

After calculation, the $ED_{50}$ of (+)-**I-3** is 6.52 mg/kg, the $ED_{50}$ of (+)-**I-4** is 21.51 mg/kg, and the $ED_{50}$ of pregabalin is 11.99 mg/kg. The above results show that the representative compounds (+)-**I-3** and (+)-**I-4** of the present application exhibit a strong antiepileptic effect in a maximum electroshock model of the mice, which can be used for preparing an antiepileptic drug.

**Example 32 Antiepileptic effect of compounds in a mouse epilepsy model (6-Hz psychomotor seizure model)**

A negative vehicle control group, a positive drug control group (pregabalin and gabapentin) and a test group of the compounds of the present application were set. Male C57BL/6 mice with a body weight of 20±2 g were selected. Test drugs and control drugs were both dissolved in DMSO (5% v/v) plus a 10% normal saline solution of 1,2-propanediol (95% v/v) and administered by gavage administration at a volume of 10 mL/kg. After fasted for 12 h, the mice were administered the compounds of the present application or the control drugs, and then subjected to 6-Hz electrical stimulation to induce epilepsy seizure behaviors after a certain time interval (2 h for pregabalin, 1 h for gabapentin, 1 h for levetiracetam, and 3 h for the compounds of the present application). In an induction model, when the stimulation was performed with a Model 4100 stimulator (A-M Systems, the United States), rear necks of the mice were immobilized by hands on a feeding cage, a corneal electrode was moisten with normal saline and allowed to gently contact bilateral corneas of the mice, the electrical stimulator was trod by a foot to give the mice the electrical stimulation with a stimulation parameter of 6-Hz, a one-way square wave of 32 mA, a wave width of 0.2 ms and a stimulation duration of 3 sec, and timing was performed with a stopwatch during the stimulation. After the electrical stimulation was completed, the mice with the seizure behaviors lasting for less than 7 sec were regarded as being protected by the drugs, and the mice with the seizure behaviors lasting for more than 7 sec were regarded as being not protected from the epilepsy by the drugs. Response situations of the animals were observed and recorded, and obtained data were counted to calculate protection rates of the compounds. Statistical method: The protection rates of different administration groups were separately compared with that of the vehicle control group to carry out a Fisher's exact test.

Test results are shown in the following table.

| Compound | Administration dose (mg/kg) | Number of animals with clonic seizures/total number | Protection rate (%) |
|---|---|---|---|
| Blank vehicle (vehicle) | - | 8/8 | 0 |
| Levetiracetam | 200 | 3/11** | 72.7 *** |
| Pregabalin | 30 | 1/6** | 83.3 *** |
| Gabapentin | 300 | 2/4 | 50.0 * |
| (+)-**I-3** p-toluenesulfonate | 100# | 4/8 | 50.0 * |
| (+)-**I-3** p-toluenesulfonate | 60# | 6/8 | 25.0 * |
| (+)-**I-4** p-toluenesulfonate | 60# | 2/4 | 50.0 * |
| #Note: calculated by a drug prototype (free base). | | | |

The above results show that the representative compounds (+)-**I-3** p-toluenesulfonate and (+)-**I-4** p-toluenesulfonate of the present application exhibit a very strong antiepileptic effect in the mouse epilepsy seizure model induced by the 6-Hz electrical stimulation, which can be used for preparing an antiepileptic drug.

**Example 33 Antiepileptic effect of compounds in a mouse epilepsy model (model with subcutaneous injection of pentylenetetrazole (sc-PTZ))**

A negative vehicle control group, a positive drug control group (pregabalin and gabapentin) and a test group of the compounds of the present application were set. Male ICR mice with a body weight of 22±2 g were selected. Test drugs and control drugs were both dissolved in DMSO (5% v/v) plus a 10% normal saline solution of 1,2-propanediol (95% v/v) and administered by intraperitoneal injection at an administration volume of 10 mL/kg. After fasted for 12 h, the mice were administered the compounds of the present application or the control drugs, and then subcutaneously injected with pentylenetetrazole (PTZ) to induce epilepsy seizure behaviors after a certain time interval (2 h for pregabalin, 1 h for gabapentin, and 3 h for the compounds of the present application). In an induction model, pentylenetetrazole (PTZ) was dissolved in normal saline and administered to the mice through the way of subcutaneous injection (10 mL/kg, administration dose 100 mg/kg). After the injection of PTZ, behavioral observations on the mice were conducted for 1 h, and the latent periods and numbers of the mice with convulsive seizures, clonic seizures, tonic seizures and death after the injection of PTZ were recorded.

Statistical method: Protection rates and mortality of different administration groups were separately compared with those of the vehicle control group to carry out a Fisher's exact test. The seizure time and the latent periods were analyzed by one-way ANOVA.

Test results are as follows.

| Compound | Dose (mg/kg) | Convulsive seizure incidence | Clonic seizure incidence | Generalized clonic seizure incidence | Tonic seizure incidence | Mortality |
|---|---|---|---|---|---|---|
| Blank vehicle (vehicle) | - | 8/8 | 8/8 | 8/8 | 6/8 | 6/8 |
| Gabapentin (GBP) | 30 | 10/10 | 10/10 | 10/10 | 6/10 | 7/10 |
| Pregabalin (PGB) | 30 | 8/8 | 8/8 | 5/8 | 1/8** | 1/8* |
| (+)-**I-3** p-toluenesulfonate | 30# | 10/10 | 10/10 | 6/10 | 0/10** | 0/10** |
| (+)-**I-4** p-toluenesulfonate | 30# | 10/10 | 10/10 | 6/10 | 2/10 | 3/10 |
| Note: In the Fisher's exact test, compared with the vehicle control group, *p<0.05, **p<0.01 represents a significant difference, and # represents that the dose is calculated by a drug prototype (free base). | | | | | | |

The subcutaneous injection of pentylenetetrazole (PTZ) induces intense convulsive behaviors in the mice: in the vehicle control group, generalized clonic seizures occurred in 8/8 (100%), generalized tonic-clonic seizures occurred in 6/8 (75%), and death occurred in 6/8 (75%). The positive control pregabalin (PGB) has a tendency to reduce generalized clonic seizures (100%→62.5%), which significantly decreases the tonic seizure incidence (75%→12.5%) and the mortality (75%→12.5%). (+)-**I-3** p-toluenesulfonate has a tendency to reduce generalized clonic seizures (100%→ 60%), which significantly decreases the tonic seizure incidence (75%→0%) and the mortality (75%→0%). (+)-**I-4** p-toluenesulfonate has a tendency to reduce generalized clonic seizures (100%→60%), and also has a tendency to decrease the tonic seizure incidence (75%→20%) and the mortality (75%→30%). Gabapentin (GBP) does not significantly improve the convulsive seizure incidence and the mortality at the dose.

For the representative compounds (+)-**I-3** p-toluenesulfonate and (+)-**I-4** p-toluenesulfonate of the present application and the control drugs, the time from the subcutaneous injection of pentylenetetrazole to the occurrence of different epilepsy related phenomena in the above test is defined as the latent period at a certain stage. As can be seen from FIG. 6, pregabalin (PGB), (+)-**I-3** p-toluenesulfonate and (+)-**I-4** p-toluenesulfonate significantly prolong the latent periods of generalized clonic seizures, tonic seizures and death at 30 mg/kg (calculated by free base), but gabapentin (GBP) is not effective at the dose.

The above results show that the representative compounds (+)-**I-3** p-toluenesulfonate and (+)-**I-4** p-toluenesulfonate of the present application have a strong inhibition effect on the mouse epilepsy seizures and death caused by the epilepsy in the mouse model with subcutaneous injection of pentylenetetrazole, which can be used for preparing an antiepileptic drug.

**Example 34 Pharmacokinetic test of compounds on rats**

Male SD rats were fasted for 12 h before the test and then allowed to drink water freely. The compound (+)-**I-3** p-toluenesulfonate or (+)-**I-4** p-toluenesulfonate of the present application was dissolved with a 10% distilled water solution of 1,2-propanediol and then administered orally or intravenously to the male SD rats, with 3 rats in each group. At 15 min, 30 min, 1 hour, 2 hours, 3 hours, 4 hours, 6 hours, 8 hours and 24 hours after the oral administration or at 5 min, 15 min, 30 min, 1 hour, 2 hours, 3 hours, 4 hours, 6 hours, 8 hours and 24 hours after the intravenous administration, 0.2 mL of blood was taken from retroocular venous plexuses, placed in an EDTA-$K_2$ test tube, centrifuged at 11,000 rpm for 5 min to separate plasma, and frozen in a refrigerator at -20°C. The concentration of a drug prototype was determined by HPLC-ESI-MS according to a verified method, and pharmacokinetic parameters were calculated by WinNonLin. Results are shown in the following table.

| Compound* | (+)-**I-3** p-toluenesulfonate | | (+)-**I-4** p-toluenesulfonate | |
|---|---|---|---|---|
| Administration route | Gavage | Intravenous injection | Gavage | Intravenous injection |
| Administration dose (mg/kg) | 2.74 | 1.10 | 2.73 | 1.09 |
| $t_{½}$ (h) | 1.14 | 1.25 | 1.08 | 0.94 |
| $T_{max}$ (h) | 1.33 | / | 1 | / |
| $C_{max}$ (ng/mL) | 1940 | / | 2350 | / |
| $AUC_{0-t}$ (h·ng/mL) | 7750 | 3250 | 7290 | 2640 |
| $AUC_{0-\infty}$ (h·ng/mL) | 7890 | 3280 | 7380 | 2670 |
| $MRT_{0-\infty}$ (h) | 2.74 | 1.99 | 2.38 | 1.63 |
| *F* (%) | 95.73% (calculated based on $AUC_{0-t}$)<br>96.57% (calculated based on $AUC_{0-\infty}$) | | 110.25% (calculated based on $AUC_{0-t}$)<br>110.36% (calculated based on $AUC_{0-\infty}$) | |
| * The two compoundsare administered in the form of p-toluenesulfonate, and test data are based on the drug prototype (free base). | | | | |

As can be seen from the above data, the representative compounds (+)-**I-3** p-toluenesulfonate and (+)-**I-4** p-toluenesulfonate of the present application have rapid absorption and extremely high bioavailability after the oral administration, and are suitable for an oral administration route.

**Example 35 Test of the impact of compounds on motor functions of rats**

Test drugs were all dissolved in DMSO (5% v/v) plus a 10% normal saline solution of 1,2-propanediol (95% v/v) and administered by gavage administration at a volume of 10 mL/kg. A negative vehicle control, a positive drug control group (pregabalin, (PGB), 10 mg/kg, 30 mg/kg) were set, and a test group (including (+)-**I-3** p-toluenesulfonate at 10 mg/kg (calculated by free base), (+)-**I-4** p-toluenesulfonate at 10 mg/kg (calculated by free base), (+)-**I-3** p-toluenesulfonate at 30 mg/kg (calculated by free base) and (+)-**I-4** p-toluenesulfonate at 30 mg/kg (calculated by free base)) were set. Male SD rats with the body weight of 200-250 g were selected, with 10 animals in each administration group. A YLS-31A fatigue rotarod (Shanghai Xinruan Information Technology Co., Ltd.) was used as rotarod test equipment, and a rotation speed was set at a constant speed of 6 rpm. A screening test was carried out on the first day. Tail tips of the rats were pinched, placed on the rotarod, and released the same after the rats maintained balance on the rotarod and moved along with the rotarod. The falling number of the rats within the next 1 min was recorded and counted. The rats that fell for 3 times within 1 min were excluded, which were not used for subsequent drug evaluation. A drug evaluation test was carried out on the second day. The rats were fasted for 8 h before the test, then a rotarod test was carried out once to serve as a time point (BL) before administration, and then administration was performed by gavage. The rotarod test was carried out once at 1 hour, 2 hours, 4 hours, 6 hours, 8 hours, 10 hours and 24 hours after the administration, respectively. The falling number of the rats within 1 min was recorded and counted. The rats falling for 3 times within one min were regarded as having "motor function impairments" caused by drug toxicity. The "motor function impairment" degree (rotarod fall) of each administration group at a specific time point was calculated as a proportion of a number of the animals with "motor function impairments" in the group to a total number of the animals in the group.

Test results are shown in FIG. 7.

The rotarod test is a classic test for measuring the damage of compounds to motor functions of animals. The rat rotarod test was used to evaluate impacts of the representative compounds (+)-**I-3** p-toluenesulfonate and

(+)-**I-4** p-toluenesulfonate of the present application on the motor functions of the rats. As can be seen from FIG. 7, at the dose of 10 mg/kg (calculated by free base), (+)-**I-3** p-toluenesulfonate, (+)-**I-4** p-toluenesulfonate and the control pregabalin (PGB) all have no significant impact on rotarod behaviors of the rats; and at the dose of 30 mg/kg (calculated by free base), the PGB group has obvious toxic responses and has a maximum impact on the rotarod behaviors of the rats at 4 h after the administration, and the impacted proportion of the rotarod behaviors of the rats in the (+)-**I-3** p-toluenesulfonate group and the (+)-**I-4** p-toluenesulfonate group are smaller than that in the PGB group.

## Example 36 Acute toxicity test of compounds on rats

The preliminary acute toxicity of (±)-**I-7**, (+)-**I-3** p-toluenesulfonate and (+)-**I-4** p-toluenesulfonate in male SD rats was assessed.

After adapting to the environment for one week, the SD rats were used in a preliminary acute toxicity test. The compounds were newly formulated on the day of the test. Pregabalin and (±)-**I-7** were directly dissolved with 0.9% sodium chloride to obtain solutions at desired concentrations. (+)-**I-3** p-toluenesulfonate and (+)-**I-4** p-toluenesulfonate were used with a vehicle of 5% PEG 400 plus 95% of 0.9% sodium chloride, that is, first dissolved in PEG 400 with a final volume of 5%, then added into the 0.9% sodium chloride solution with a final volume of 95%, and subjected to vortex for full dissolution. After being labeled and weighed, the animals were randomly grouped with 2-4 animals in each group. The animals were administered each compound to be tested and the vehicle (5% PEG 400+95% of 0.9% sodium chloride) through oral gavage administration at a volume of 10 mL/kg, respectively. Clinical symptoms of each animal were observed and recorded at 0.5 hours, 1 hour, 2 hours, 4 hours, 6 hours and 24 hours after the administration, respectively. Clinical symptom codes were as follows: 0 no abnormality, 1 mild tremor, 2 unsteady gait, 3 prone position, 4 startle reflex, 5 difficult breathing, 6 righting reflex disappeared, 7 eyelid closure, 8 shortness of breath, and × animal death.

Results and discussions: Results are shown in the following table. When the compounds (+)-**I-3** p-toluenesulfonate at a dose of 164.5 mg/kg (calculated by free base) and (+)-**I-4** p-toluenesulfonate at 163.8 mg/kg (calculated by free base) were administered through the oral gavage administration, at 0-24 h after the administration, all the animals in the two groups were consistent with the animals in the vehicle control group and had no obvious abnormal behaviors. When (+)-**I-3** p-toluenesulfonate and (+)-**I-4** p-toluenesulfonate were given at a dose of 300 mg/kg (calculated by free base) through the oral gavage administration, in the (+)-**I-3** p-toluenesulfonate group, two animals exhibited prone position only at 1 h after the administration, wherein one animal also had symptoms of mild tremor and eyelid closure; while in the (+)-**I-4** group, one animal exhibited prone position only at 1 h after the administration, and another animal exhibited prone position phenomenon at both 1 h and 3 h after the administration. When the other compound (±)-**I-7** was administered at a dose of 300 mg/kg (calculated by free base) through the oral gavage administration, it was found that each animal exhibited prone position at 1 h and 2 h after the administration, in which two animals also had a sign of mild tremor. At 24 h after the administration, the animals administered (±)-**I-7**, (+)-**I-3** p-toluenesulfonate and (+)-**I-4** p-toluenesulfonate all had no obvious abnormalities, which were consistent with the animals in the vehicle control group. The test results show that when the representative compounds (+)-**I-3** p-toluenesulfonate at the dose of 164.5 mg/kg (calculated by free base) and (+)-**I-4** p-toluenesulfonate at the dose of 163.8 mg/kg (calculated by free base) of the present application are administered orally to the male rats, they do not exhibit neurotoxicity. Combined with the analgesic and antiepileptic efficacy doses disclosed in Examples 29-33 of the present application, it can be concluded that the representative compounds (+)-**I-3** p-toluenesulfonate and (+)-**I-4** p-toluenesulfonate of the present application have a wide safety window. The representative compounds (±)-**I-7**, (+)-**I-3** p-toluenesulfonate and (+)-**I-4** p-toluenesulfonate of the present application can cause relatively obvious acute toxic responses in the animals at the high dose of 300 mg/kg (calculated by free base), but no animals are dead.

| Group | Animal number | Body weight (g) | Observation of clinical symptoms (administration time point) | | | | | |
|---|---|---|---|---|---|---|---|---|
| | | | 0.5 h | 1 h | 2h | 4h | 6h | 24 h |
| Vehicle control group 1 | #1 | 261 | 0 | 0 | 0 | 0 | 0 | 0 |
| | #2 | 256 | 0 | 0 | 0 | 0 | 0 | 0 |
| | #3 | 243 | 0 | 0 | 0 | 0 | 0 | 0 |
| | #4 | 255 | 0 | 0 | 0 | 0 | 0 | 0 |

(continued)

| Group | Animal number | Body weight (g) | Observation of clinical symptoms (administration time point) | | | | | |
|---|---|---|---|---|---|---|---|---|
| | | | 0.5 h | 1 h | 2h | 4h | 6h | 24 h |
| (+)-**I-3** p-toluenesulfonate (164.5 mg/kg, calculated by free base) | #1 | 252 | 0 | 0 | 0 | 0 | 0 | 0 |
| | #2 | 247 | 0 | 0 | 0 | 0 | 0 | 0 |
| | #3 | 243 | 0 | 0 | 0 | 0 | 0 | 0 |
| | #4 | 243 | 0 | 0 | 0 | 0 | 0 | 0 |
| (+)-**I-4** p-toluenesulfonate (163.8 mg/kg, calculated by free base) | #1 | 247 | 0 | 0 | 0 | 0 | 0 | 0 |
| | #2 | 248 | 0 | 0 | 0 | 0 | 0 | 0 |
| | #3 | 240 | 0 | 0 | 0 | 0 | 0 | 0 |
| | #4 | 240 | 0 | 0 | 0 | 0 | 0 | 0 |
| Vehicle control group 2 | #1 | 291 | 0 | 0 | 0 | 0 | 0 | 0 |
| | #2 | 286 | 0 | 0 | 0 | 0 | 0 | 0 |
| | #3 | 283 | 0 | 0 | 0 | 0 | 0 | 0 |
| (+)-**I-3** p-toluenesulfonate (300 mg/kg, calculated by free base) | #1 | 287 | 0 | 3 | 0 | 0 | 0 | 0 |
| | #2 | 281 | 0 | 1,3,7 | 0 | 0 | 0 | 0 |
| **(+)-I-4** p-toluenesulfonate (300 mg/kg, calculated by free base) | #1 | 292 | 0 | 3 | 0 | 3 | 0 | 0 |
| | #2 | 271 | 0 | 3 | 0 | 0 | 0 | 0 |
| (±)-**I-7** (300 mg/kg) | #1 | 269 | 0 | 1,3 | 3 | 0 | 0 | 0 |
| | #2 | 266 | 0 | 3 | 3 | 0 | 0 | 0 |
| | #3 | 257 | 0 | 3 | 3 | 0 | 0 | 0 |
| | #4 | 280 | 0 | 1,3 | 3 | 0 | 0 | 0 |

## Claims

**1.** A compound of general formula **I,** a chiral isomer thereof or a pharmaceutically acceptable salt thereof,

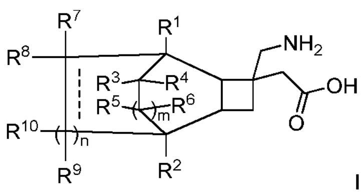

I

wherein

$R^1$ and $R^2$ are independently selected from H, halogen, and $C_1$-$C_6$ alkyl;
each of $R^3$, $R^4$, $R^5$ and $R^6$ is independently selected from H, halogen, $C_1$-$C_6$ alkyl, and $C_1$-$C_6$ alkoxy; or $R^3$ and $R^4$ together with a C atom to which they are connected form $C_3$-$C_6$ cycloalkyl, or $R^5$ and $R^6$ together with a C atom to which they are connected form $C_3$-$C_6$ cycloalkyl;
each of $R^7$, $R^8$, $R^9$ and $R^{10}$ is independently selected from H, halogen, and $C_1$-$C_6$ alkyl;
m and n are independently selected from 0, 1, 2, and 3;
alternatively, when n≥1, a C atom connected to $R^8$ and an adjacent C atom connected to $R^{10}$ are capable of forming $C_3$-$C_6$ cycloalkyl together with $R^8$ and $R^{10}$;
when n≥1, a solid line and a dashed line between the C atom connected to $R^8$ and the adjacent C atom connected to $R^{10}$ represent that a chemical bond between the C atom connected to $R^8$ and the C atom connected to $R^{10}$ can

be a single bond or a double bond; and when the chemical bond represents the double bond, corresponding $R^7$ and $R^9$ are absent.

2. The compound of general formula **I,** the chiral isomer thereof or the pharmaceutically acceptable salt thereof according to claim 1, wherein

$R^1$ and $R^2$ are independently selected from H and $C_1$-$C_3$ alkyl;
$R^3$ and $R^4$ are independently selected from H, halogen, and $C_1$-$C_3$ alkyl; or $R^3$ and $R^4$ together with the C atom to which they are connected form cyclopropyl;
$R^7$, $R^8$, $R^9$ and $R^{10}$ are independently selected from H and $C_1$-$C_6$ alkyl; or the C atom connected to $R^8$ and the C atom connected to $R^{10}$ form cyclopropyl together with $R^8$ and $R^{10}$;
the solid line and the dashed line between the C atom connected to $R^8$ and the C atom connected to $R^{10}$ represent that the chemical bond between the C atom connected to $R^8$ and the C atom connected to $R^{10}$ can be the single bond or the double bond; when the chemical bond represents the double bond, $R^7$ and $R^9$ are absent;
m=0; and n=1.

3. The compound of general formula **I,** the chiral isomer thereof or the pharmaceutically acceptable salt thereof according to claim 1 or 2, wherein

$R^1$ and $R^2$ are independently selected from H or methyl;
$R^3$ and $R^4$ are independently selected from H and methyl; or $R^3$ and $R^4$ together with the C atom to which they are connected form cyclopropyl;
$R^7$, $R^8$, $R^9$ and $R^{10}$ are independently selected from H or methyl; or the C atom connected to $R^8$ and the C atom connected to $R^{10}$ form cyclopropyl together with $R^8$ and $R^{10}$;
the solid line and the dashed line between the C atom connected to $R^8$ and the C atom connected to $R^{10}$ represent that the chemical bond between the C atom connected to $R^8$ and the C atom connected to $R^{10}$ can be the single bond or the double bond; when the chemical bond represents the double bond, $R^7$ and $R^9$ are absent;
m=0; and n=1.

4. The compound of general formula **I,** the chiral isomer thereof or the pharmaceutically acceptable salt thereof according to claim 1, wherein

$R^1$ and $R^2$ are independently selected from H or methyl;
$R^3$ and $R^4$ are independently selected from H and methyl; or $R^3$ and $R^4$ together with the C atom to which they are connected form cyclopropyl;
$R^7$, $R^8$, $R^9$ and $R^{10}$ are independently selected from H or methyl;
m=0; and n=1.

5. The compound of general formula **I,** the chiral isomer thereof or the pharmaceutically acceptable salt thereof according to claim 1 or 2, selected from the following compounds:

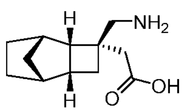, 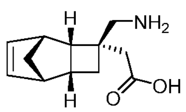, 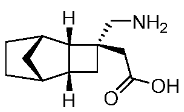, 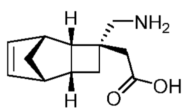, 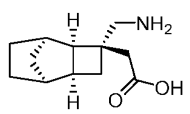, 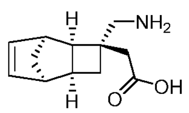,

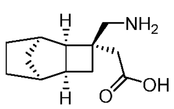, 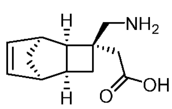, 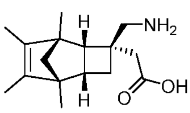, 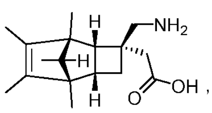, 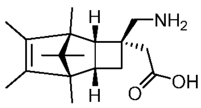, 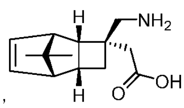,

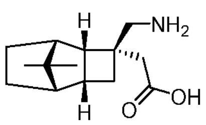, 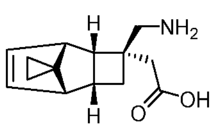, 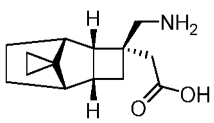, and 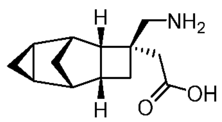.

6. A method of preparing the compound of general formula I according to any one of claims 1-5, comprising:

1) conducting a Wittig condensation reaction with ketone K and phosphonoacetate **W1** in the presence of a base to obtain $\alpha,\beta$-unsaturated acetate **L-1,**

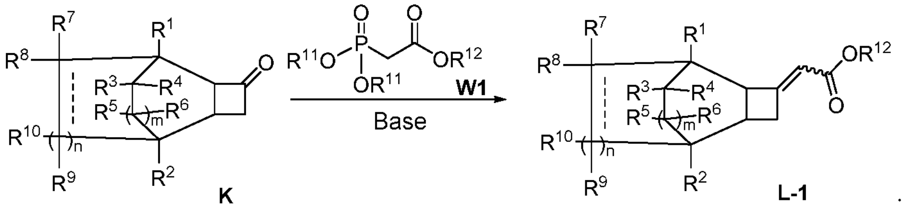


;

and conducting a Michael addition reaction with **L-1** and nitromethane in the presence of a base to obtain **M-1**,

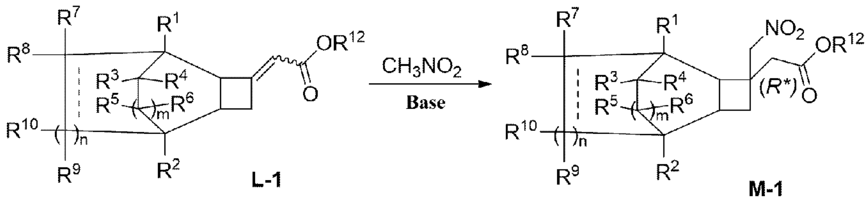


;

alternatively,

2) conducting a Knoevenagel condensation reaction with ketone K and nitromethane in the presence of a catalyst to obtain $\alpha,\beta$-unsaturated nitro compound **L-2,**

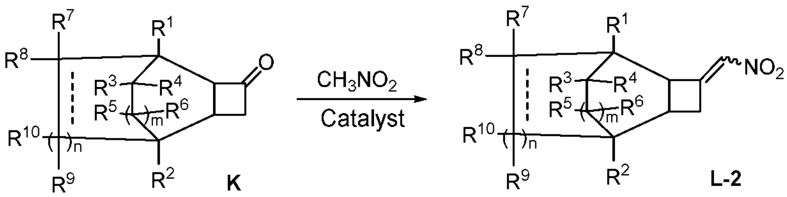


;

and conducting a Michael-like addition reaction with **L-2** and acetate **W2** in the presence of a strong base to obtain **M-2,**

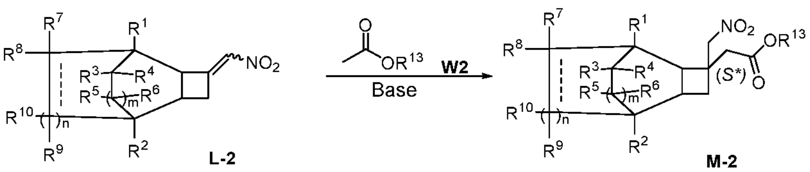


;

wherein $R^{11}$, $R^{12}$ and $R^{13}$ are selected from $C_1$-$C_6$ alkyl; and $R^1$-$R^{10}$, m and n have definitions according to any one of claims 1-5.

**7.** Use of the compound of general formula I, the chiral isomer thereof and the pharmaceutically acceptable salt thereof according to any one of claims 1-5 in preparation of a medicament for treatment of chronic neuropathic pain, epilepsy and anxiety.

**8.** A pharmaceutical composition, comprising the compound of general formula **I,** the chiral isomer thereof and the pharmaceutically acceptable salt thereof according to any one of claims 1-5, and a pharmaceutically acceptable auxiliary material, wherein the auxiliary material is selected from one or more of a carrier, a diluent, and an excipient.

**9.** The pharmaceutical composition according to claim 8, wherein the composition is a solid oral preparation, a liquid oral preparation, or an injection.

**10.** The pharmaceutical composition according to claim 9, wherein the solid oral preparation and the liquid oral

preparation comprise a dispersible tablet, an enteric-coated tablet, a chewable tablet, an orally disintegrating tablet, a capsule, a granule, and an oral solution, and a preparation of the injection comprises water for injection, lyophilized powder for injection, an infusion solution, and a small volume parenteral solution.

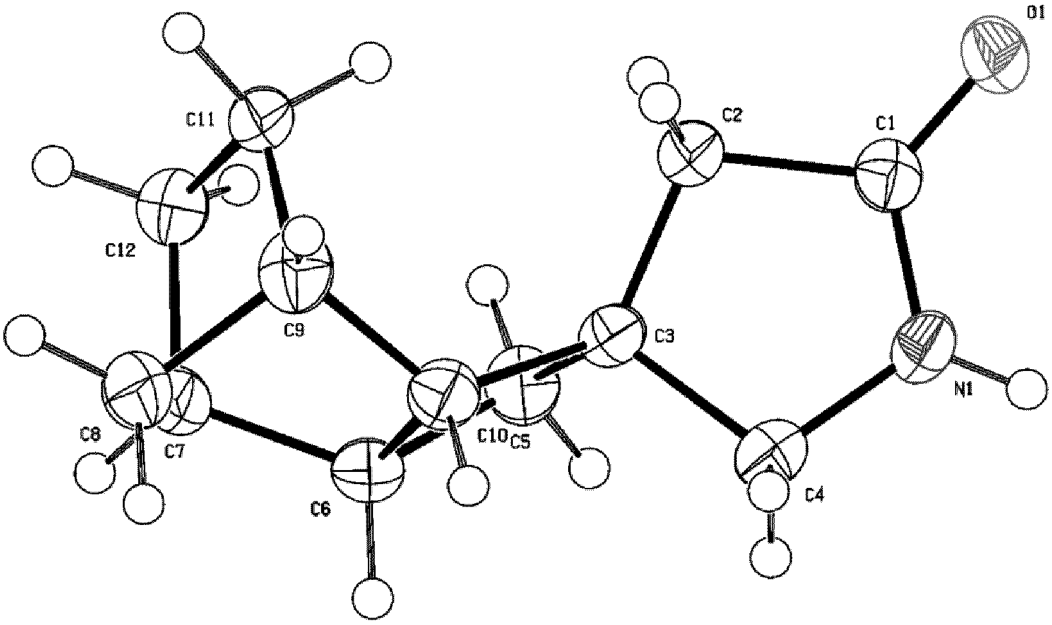

FIG. 1

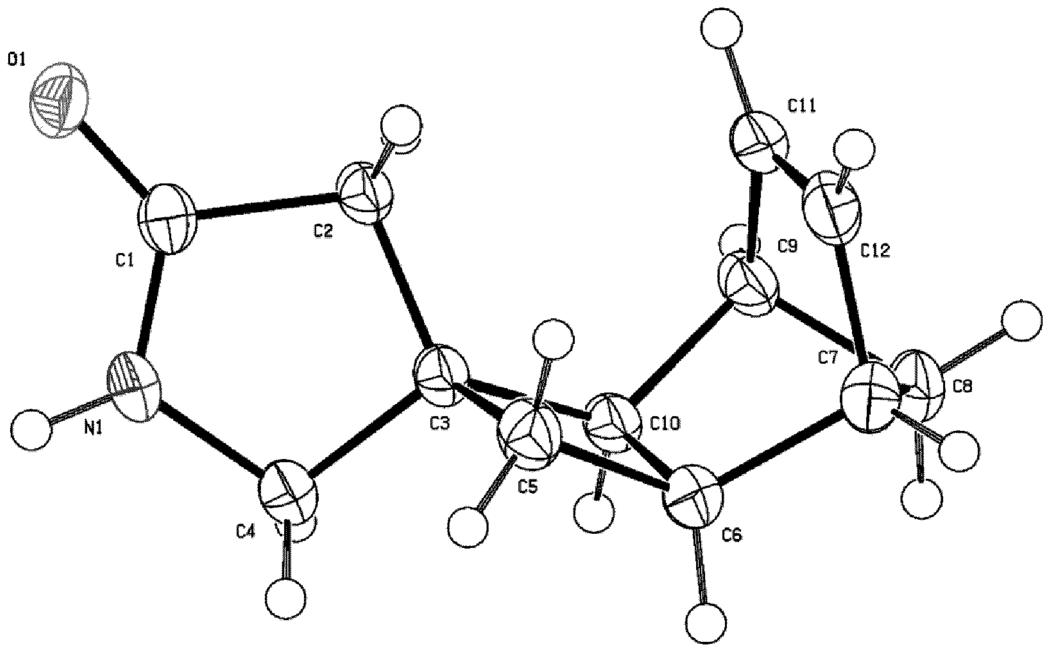

FIG. 2

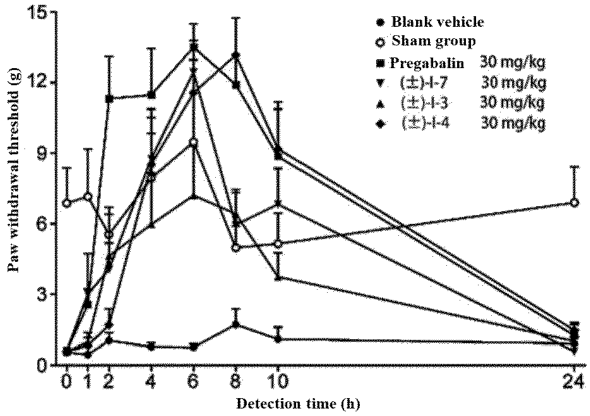


FIG. 3A

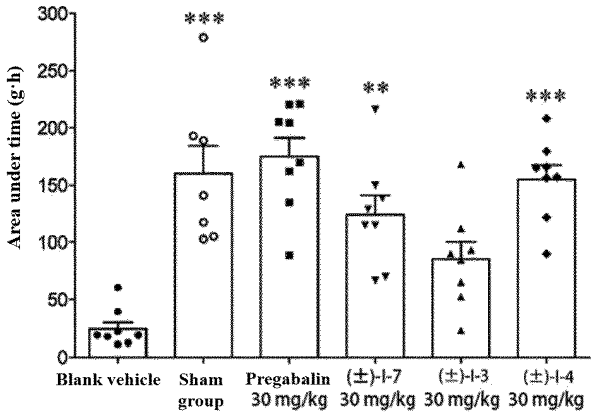


FIG. 3B

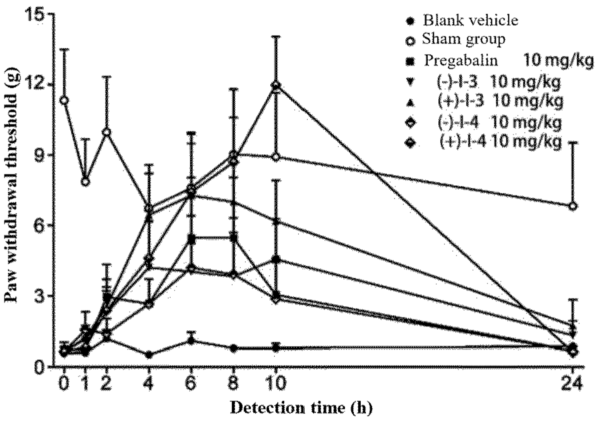


FIG. 4A

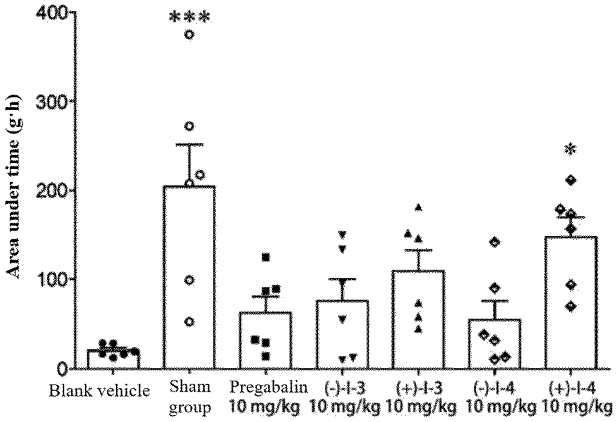


FIG. 4B

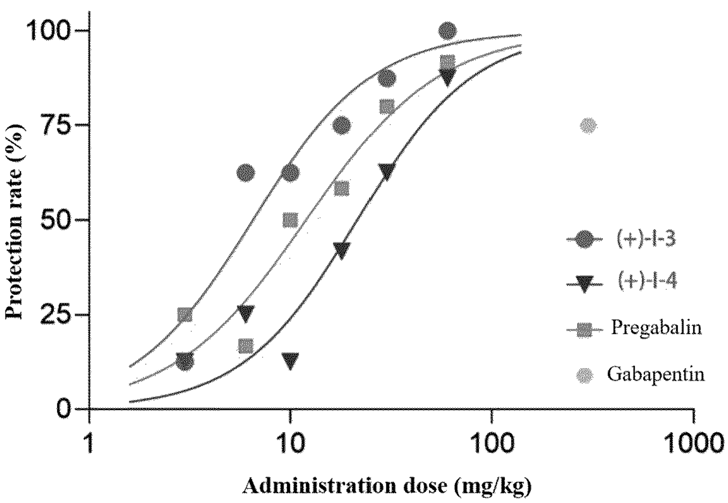


FIG. 5

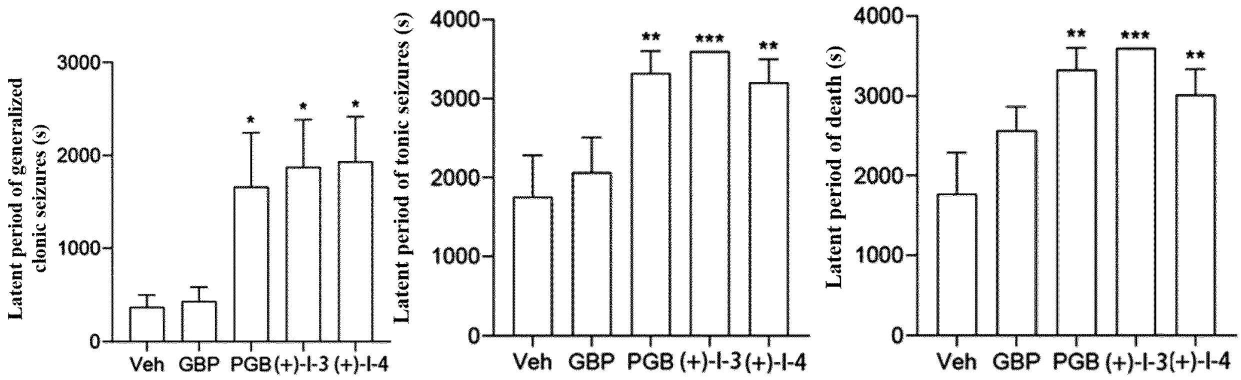
Latent period of generalized clonic seizures (s)
Latent period of tonic seizures (s)
Latent period of death (s)
Veh
GBP
PGB
(+)-I-3
(+)-I-4

FIG. 6

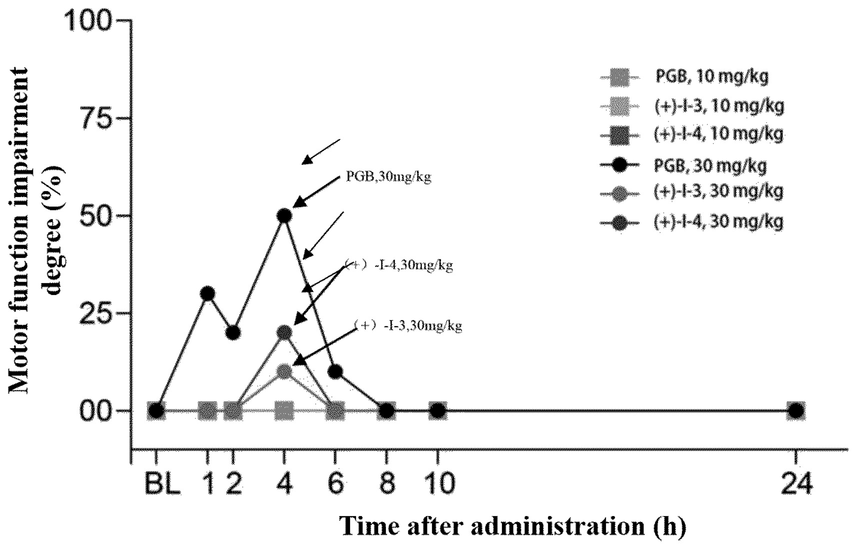
Motor function impairment degree (%)
Time after administration (h)
PGB, 10 mg/kg
(+)-I-3, 10 mg/kg
(+)-I-4, 10 mg/kg
PGB, 30 mg/kg
(+)-I-3, 30 mg/kg
(+)-I-4, 30 mg/kg
PGB,30mg/kg
(+) -I-4,30mg/kg
(+) -I-3,30mg/kg

FIG. 7

**INTERNATIONAL SEARCH REPORT**

International application No.
**PCT/CN2023/125229**

**A. CLASSIFICATION OF SUBJECT MATTER**

C07C 229/32(2006.01)i; C07C 227/04(2006.01)i; A61K 31/195(2006.01)i; A61P25/04(2006.01)i; A61P25/08(2006.01)i; A61P25/22(2006.01)i

According to International Patent Classification (IPC) or to both national classification and IPC

**B. FIELDS SEARCHED**

Minimum documentation searched (classification system followed by classification symbols)

IPC:C07C A61K A61P

Documentation searched other than minimum documentation to the extent that such documents are included in the fields searched

Electronic data base consulted during the international search (name of data base and, where practicable, search terms used)

CNABS, CNTXT, VEN, USTXT, EPTXT, WOTXT, CNKI, 万方, WANFANG, Web of Science, 百度学术, BAIDU SCHOLAR, Registry, Caplus, Marpat: 氨基丁酸, 氨基酸, 环, 疼痛, 焦虑, 癫痫, amino acid, acetic acid, pain, anxiety, epilepsy.

**C. DOCUMENTS CONSIDERED TO BE RELEVANT**

| Category* | Citation of document, with indication, where appropriate, of the relevant passages | Relevant to claim No. |
|---|---|---|
| X | CN 106928080 A (SICHUAN HAISCO PHARMACEUTICAL CO., LTD.) 07 July 2017 (2017-07-07)<br>claims 1-9, and embodiment 8 | 1-10 |
| A | CN 101878193 A (DAIICHI SANKYO CO., LTD.) 03 November 2010 (2010-11-03)<br>entire document | 1-10 |
| A | WO 2010084798 A1 (DAIICHI SANKYO CO., LTD.) 29 July 2010 (2010-07-29)<br>entire document | 1-10 |
| A | CN 107848952 A (SICHUAN HAISCO PHARMACEUTICAL CO., LTD.) 27 March 2018 (2018-03-27)<br>entire document | 1-10 |
| A | CN 1279673 A (WARNER-LAMBERT CO.) 10 January 2001 (2001-01-10)<br>entire document | 1-10 |

☐ Further documents are listed in the continuation of Box C.　☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **13 December 2023** | **04 January 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**
**Information on patent family members**

International application No.
**PCT/CN2023/125229**

| Patent document cited in search report | Publication date (day/month/year) | Patent family member(s) | Publication date (day/month/year) |
|---|---|---|---|
| CN 106928080 A | 07 July 2017 | CN 106928080 B | 25 December 2020 |
| CN 101878193 A | 03 November 2010 | KR 20130093181 A | 21 August 2013 |
| | | KR 101409079 B1 | 18 June 2014 |
| | | CO 6270314 A2 | 20 April 2011 |
| | | KR 20100065342 A | 16 June 2010 |
| | | KR 101335784 B1 | 05 December 2013 |
| | | BRPI 0818193 A2 | 12 December 2017 |
| | | BRPI 0818193 B1 | 10 November 2020 |
| | | BRPI 0818193 B8 | 25 May 2021 |
| | | NZ 584699 A | 29 April 2011 |
| | | MX 2010003394 A | 09 April 2010 |
| | | TW 201439053 A | 16 October 2014 |
| | | TWI 526425 B | 21 March 2016 |
| | | EP 2657219 A1 | 30 October 2013 |
| | | EP 2657219 B1 | 10 December 2014 |
| | | ES 2433890 T3 | 12 December 2013 |
| | | EP 2192109 A1 | 02 June 2010 |
| | | EP 2192109 A4 | 18 April 2012 |
| | | EP 2192109 B1 | 04 September 2013 |
| | | PT 2192109 E | 09 December 2013 |
| | | CY 1114836 T1 | 14 December 2016 |
| | | US 2010249229 A1 | 30 September 2010 |
| | | US 7947738 B2 | 24 May 2011 |
| | | DK 2192109 T3 | 16 December 2013 |
| | | SI 2192109 T1 | 31 March 2014 |
| | | HK 1139381 A1 | 17 September 2010 |
| | | BR 122013020635 A2 | 06 August 2019 |
| | | WO 2009041453 A1 | 02 April 2009 |
| | | AU 2008304933 A1 | 02 April 2009 |
| | | AU 2008304933 B2 | 24 February 2011 |
| | | TW 200924743 A | 16 June 2009 |
| | | TWI 436762 B | 11 May 2014 |
| | | PH 12013501663 B1 | 09 February 2015 |
| | | HRP 20130935 T1 | 22 November 2013 |
| | | CA 2701110 A1 | 02 April 2009 |
| | | CA 2701110 C | 08 January 2013 |
| | | PL 2192109 T3 | 31 January 2014 |
| | | IL 204621 A0 | 30 November 2010 |
| | | IL 204621 A | 30 April 2013 |
| | | MY 153910 A | 15 April 2015 |
| | | RU 2010116708 A | 10 November 2011 |
| | | RU 2446148 C2 | 27 March 2012 |
| | | HK 1190698 A1 | 11 July 2014 |
| | | ES 2531324 T3 | 13 March 2015 |
| | | JP 4479974 B2 | 09 June 2010 |
| | | JPWO 2009041453 A1 | 27 January 2011 |
| | | MY 172034 A | 12 November 2019 |
| | | ZA 201002013 B | 24 November 2010 |
| | | CN 101878193 B | 09 July 2014 |
| WO 2010084798 A1 | 29 July 2010 | None | |

Form PCT/ISA/210 (patent family annex) (July 2022)

**INTERNATIONAL SEARCH REPORT**
**Information on patent family members**

International application No.
**PCT/CN2023/125229**

| Patent document cited in search report | Publication date (day/month/year) | Patent family member(s) | Publication date (day/month/year) |
|---|---|---|---|
| CN 107848952 A | 27 March 2018 | WO 2017114225 A1 | 06 July 2017 |
| | | TW 201726592 A | 01 August 2017 |
| | | CN 107848952 B | 09 April 2021 |
| | | TW 662019 B1 | 11 June 2019 |
| CN 1279673 A | 10 January 2001 | DE 69822214 D1 | 08 April 2004 |
| | | DE 69822214 T2 | 10 March 2005 |
| | | DK 1045839 T3 | 05 July 2004 |
| | | IL 135314 A0 | 20 May 2001 |
| | | KR 20010033154 A | 25 April 2001 |
| | | HU 0100472 A2 | 28 September 2001 |
| | | HU 0100472 A3 | 28 March 2003 |
| | | CA 2304965 A1 | 24 June 1999 |
| | | CA 2304965 C | 21 August 2007 |
| | | JP 2002508361 A | 19 March 2002 |
| | | EP 1045839 A2 | 25 October 2000 |
| | | EP 1045839 B1 | 03 March 2004 |
| | | US 2003229054 A1 | 11 December 2003 |
| | | ES 2216338 T3 | 16 October 2004 |
| | | TR 200001794 T2 | 23 October 2000 |
| | | NO 20003037 D0 | 14 June 2000 |
| | | NO 20003037 L | 27 September 2004 |
| | | AU 1796299 A | 05 July 1999 |
| | | AU 759619 B2 | 17 April 2003 |
| | | PL 348305 A1 | 20 May 2002 |
| | | SI 1045839 T1 | 30 June 2004 |
| | | EP 1400526 A2 | 24 March 2004 |
| | | EP 1400526 A3 | 31 March 2004 |
| | | NZ 503981 A | 20 December 2002 |
| | | WO 9931074 A2 | 24 June 1999 |
| | | WO 9931074 A3 | 04 November 1999 |
| | | IS 5436 A | 07 April 2000 |
| | | PT 1045839 E | 30 July 2004 |
| | | NO 20040817 L | 24 February 2004 |
| | | ATE 260904 T1 | 15 March 2004 |
| | | CU 23028 A3 | 23 February 2005 |
| | | US 6521650 B1 | 18 February 2003 |
| | | BR 9813656 A | 10 October 2000 |
| | | CN 1210268 C | 13 July 2005 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **GOLDSTEIN, D. J. et al.** *Pain*, 2005, vol. 116 (1-2), 109-118
- **RICE, A. S. C. et al.** *Pain*, 2001, vol. 94 (2), 215-224
- **ROWBOTHAM, M. et al.** *JAMA*, 1998, vol. 280 (21), 1837-1842
- **DWORKIN, R. H. et al.** *Neurology*, 2003, vol. 60 (8), 1274-1283
- **SABATOWSKI, R. et al.** *Pain*, 2004, vol. 109 (1-2), 26-35
- **FIELD, M.J. et al.** *Proc. Natl. Acad. Sci. U.S.A.*, 2006, vol. 103, 17537-17542
- **CALANDRE, E.P. et al.** *Expert Rev. Neurother.*, 2016, vol. 16, 1263-1277
- **GEE, N.S. et al.** *J. Biol. Chem.*, 1996, vol. 271, 5768-5776
- **MARAIS, E. et al.** *Mol. Pharmacol.*, 2001, vol. 59, 1243-1248